# EUROPEAN PATENT APPLICATION

(11) **EP 4 353 253 A1**
(43) Date of publication of application: **17.04.2024**
(21) Application number: 22200627.2
(22) Date of filing: 10.10.2022
(51) Int. Cl.: A61K 39/00, C07K 14/725, C12N 5/00, C12N 15/113

(54) **PURIFICATION OF TCR-MODIFIED T CELLS USING TCR-SPECIFIC CAR-NK CELLS**

(71) Applicant: Charité - Universitätsmedizin Berlin, 10117 Berlin (DE)
(72) Inventor: WAGNER, Dimitrios, Laurin, 13347 Berlin (DE); DU, Weijie, 16321 Bernau (DE); MARTINI, Stefania, 10711 Berlin (DE); ELSALLAB, Magdi, Brighton MA, 02135 (US); ABOU-EL-ENEIN, Mohamed, South Pasadena, CA 91030 (US)
(74) Representative: Hertin und Partner Rechts- und Patentanwälte PartG mbB

(57) **Abstract**

The invention relates to a genetically modified natural killer (NK) cell, comprising a nucleic acid sequence encoding a chimeric antigen receptor (CAR), wherein said CAR comprises an extracellular antigen-binding domain that specifically binds a component of a T cell receptor complex, and wherein said nucleic acid sequence is integrated in the NK cell genome without a viral vector. The invention further relates to a method for destroying a T cell, comprising an endogenous TCR expressed and/or presented on the cell surface, the method comprising contacting said T cell to be destroyed with a genetically modified natural killer (NK) cell of the invention. The invention further relates to a method for manufacturing a therapeutic genetically modified T cell, preferably a CAR T cell, to the resulting in vitro cell population, and to the in vitro cell population for use in the treatment and/or prevention of a medical condition susceptible to T cell therapy.

## Description

The invention relates to the field of biology and medicine, in particular to the field of genetically modified therapeutic cell products and methods for generating such cells.

The invention relates to a genetically modified natural killer (NK) cell, comprising a nucleic acid sequence encoding a chimeric antigen receptor (CAR), wherein said CAR comprises an extracellular antigen-binding domain that specifically binds a component of a T cell receptor complex, and wherein said nucleic acid sequence is integrated in the NK cell genome without a viral vector.

The invention further relates to a method for destroying a T cell comprising an endogenous TCR expressed and/or presented on the cell surface, the method comprising contacting a genetically modified natural killer (NK) cell of the invention with the T cell to be destroyed.

The invention further relates to a method for manufacturing a therapeutic genetically modified T cell, preferably a CAR T cell, comprising one or more disruptive modifications to endogenous TCR components, the method comprising purifying and/or enriching said cells using the genetically modified natural killer (NK) cell of the invention.

Further aspects of the invention relate to an in vitro cell population obtained by the method of the invention, and to the cell population for use in the treatment and/or prevention of a medical condition susceptible to T cell therapy in a subject, wherein the cell population is preferably allogeneic to said subject.

### BACKGROUND OF THE INVENTION

CAR T cells have emerged as a potent therapy for hematological malignancies. In September 2022, six CAR T products have been approved in the United States. While all approved products are based on patient-derived derived T cells, there is a growing interest in developing allogeneic CAR T cells. Large-scale manufacturing of allogeneic off-the-shelf CAR T cell products is a way to reduce costs and simplify the clinical logistics associated with autologous cell manufacturing ¹. However, Graft-versus-Host-disease (GvHD) remains one important risk when administering allogeneic CAR T cell products to patients².

Gene editing tools can be utilized to reduce the risk for GvHD by the elimination of allo-reactive T cell receptor (TCR)/CD3 complex via a knockout of one of its components³, usually the TCRα constant (*TRAC*) gene⁴. Different gene editing systems such as CRISPR/Cas or TALENs can be used to disrupt *TRAC* expression⁵. Furthermore, targeted in-frame insertion of CAR transgenes into an early exon of *TRAC* can be used to functionally replace the endogenous TCR with tumor-specific CARs⁶⁻⁹. While gene-editing procedures have been improved to achieve very high knockout rates, conventional manufacturing strategies incorporate a purification step prior to the final formulation and/or cryopreservation of the allogeneic CAR T cell product¹⁰⁻¹².

Previous clinical trials with patients undergoing haploidentical stem cell transplantation have estimated that 5×10⁴ TCRαβ T cells / kg bodyweight represents the threshold for the occurrence of GvHD ^{13,14}. Clinical case reports using un-matched allogeneic CAR T cells demonstrated that as little as 1-2% contaminating TCRαβ+ T cells in gene-edited cell products have the capacity to induce GvHD in heavily pre-treated leukemia patients.¹⁰ While reported symptoms were manageable with steroid treatment, this indicates a need for enhanced enrichment strategies which achieve higher purity to prevent GvHD at high CAR T cell doses required for effective treatment in bulky disease¹⁵.

The predominant method for purification of allogeneic TCR-edited CAR T cells is magnetic cell separation. The limitations of this are the significant cell loss and purification results with remaining impurities ranging from 0.7% to 1.0% at clinical scale ^{10,11}. Jullierat *et al* demonstrated that transfection of a CD3-specific CAR mRNA enables enrichment of TCR-negative T cells during expansion with final contaminations of about 0.5-1.1% TCRαβ⁺ cells.¹⁶ Methods which allow in-process purification would alleviate reagent intensive cell handling and the cell loss associated with magnetic TCR depletion at the end of CAR T cell expansion.

Despite recent advances in the art, improvements in the purification of (e.g. allogeneic) TCR-disrupted CAR T cells are urgently required in order to reduce residual risk to patients caused by GvHD. Methods and reagents are required that enable large-scale cell manufacture and purification that have a low risk to patients potentially caused by residual levels of therapeutic T cells with an intact TCR.

### SUMMARY OF THE INVENTION

The present invention addresses these challenges by providing genetically modified NK cells suitable to purify TCR-disrupted (therapeutic) T cells, preferably CAR T cells, during *ex vivo* expansion. As described in the Examples below, by overexpressing a TCR/CD3-specific CAR, irradiated CAR NK-92 cells effectively deplete residual TCR/CD3⁺ T cells from a therapeutic CAR T cell population. The inventors show that product purity in terms of TCR/CD3-negative T cell rates could be increased to >99.9%, which significantly exceeded purity achieved by prior art magnetic bead-mediated separation. The present invention enables dynamic TCR/CD3-elimination throughout CAR T cell expansion resulting in a sustained removal of TCR/CD3⁺ cells without negatively impacting CAR T cell functionality of the remaining cells.

In light of the prior art, the technical problem underlying the present invention is to provide improved or alternative means to deplete residual TCR⁺ T cells from a therapeutic T cell population.

One problem underlying the invention can be seen as the provision of means for generating a high-purity therapeutic T cell population suitable for administration to a patient.

Another problem underlying the invention is the provision of a therapeutic T cell population with reduced risk of GvHD in a subject receiving said therapeutic T cells.

Another problem underlying the invention is the provision of means to avoid or reduce the considerable loss of therapeutic cell material due to the column-based MACS procedure typically employed to remove residual TCR+ T cells from a therapeutic T cell (preferably CAR T cell) preparation.

These problems are solved by the features of the independent claims. Preferred embodiments of the present invention are provided in the dependent claims.

The invention therefore relates to a genetically modified natural killer (NK) cell, comprising a nucleic acid sequence encoding a chimeric antigen receptor (CAR), wherein said CAR comprises an extracellular antigen-binding domain that specifically binds a component of a T cell receptor complex, and wherein said nucleic acid sequence is integrated in the NK cell genome without a viral vector.

A related aspect of the invention relates to a method for destroying a T cell comprising an endogenous TCR expressed and/or presented on the cell surface, the method comprising contacting said T cell (to be destroyed) with a genetically modified natural killer (NK) cell as described herein.

The genetically modified T cell comprising an endogenous TCR expressed and/or presented on the cell surface is, in embodiments, in a mixture with a genetically modified T cell comprising one or more disruptive modifications to endogenous TCR components, such as a therapeutic CAR T cell in which the TCR has been disrupted.

Using the cells and methods of the invention improvements are achieved with respect to depleting residual TCR⁺ T cells from a therapeutic T cell population, generating a high-purity therapeutic T cell population suitable for administration to a patient, and generating a therapeutic T cell population with reduced risk of GvHD in a subject receiving said therapeutic T cells.

Graft-versus-host disease (GvHD) is a major risk when applying healthy donor-derived CAR T cells to HLA-unmatched immunocompromised patients. To address this risk, gene editing can be used to disrupt potentially alloreactive T cell receptors (TCRs) in CAR T cells and reduce the risk of GvHD. Despite high knock-out rates with optimized methods, a subsequent purification step is necessary to obtain a safe allogeneic T cell product. To date, magnetic cell separation (MACS) is the gold standard to purify TCRαβ- CAR T cells, but product purity can still be insufficient to completely avoid GvHD in the recipient of the therapeutic CAR T cells.

To overcome this challenge, the present invention presents a new approach to eliminate residual TCRαβ/CD3+ T cells after TRAC gene editing by providing and using a genetically modified natural killer (NK) cell, comprising a nucleic acid sequence encoding a chimeric antigen receptor (CAR), wherein said CAR comprises an extracellular antigen-binding domain that specifically binds a component of a CD3-T cell receptor complex, wherein said nucleic acid sequence is integrated in the NK cell genome without a viral vector. This approach, in embodiments and as described in the examples below, is demonstrated by adding a genetically modified CD3-specific CAR NK-92 cell line to the therapeutic T cell population during the expansion process. As shown in the examples, two consecutive co-cultures with irradiated, short-lived CD3-specific CAR NK-92 cells produce CAR T cells with less than 0.1% TCR+ T cells, which marks over 10-fold reduction of TCR+ T cells compared to MACS-purification. The present invention also circumvents MACS-associated cell loss, which can be as high as 30-50% of the cell population, thereby improving the overall TCR/CD3- CAR T cell yield and allowing for a better cost-per-dose ratio. The final CAR T cell products show no significant changes in CAR T cell memory phenotype or cytotoxicity. The inventive cell-mediated purification method thus improves the production process of allogeneic CAR T cells for clinical applications and reduces the risk of unwanted side effects that may occur after allogeneic T cell transfer.

Further advantages to the present invention include the finding that co-cultures with irradiated TCR/CD3-specific CAR NK-92 cells appear to also support CAR T cell expansion and do not negatively impact *in vitro* CAR T cell function. The invention thus enables improved production methods and functionality for therapeutic T cell populations. The invention potentially enables positive effects to the CD4:CD8 ratio and anti-tumor performance of the treated therapeutic T cell population *in vivo.*

The present invention enables optimized manufacturing to achieve high yields and enable higher dosages of therapeutic T cells, which is important in pursuing cost-effectiveness of cell therapy products. For example, the use of TCR/CD3-specific CAR NK-92 cells may effectively replace bead-mediated depletion procedures with an uncomplicated co-culture approach. It simplifies cell handling and avoids cell loss associated with magnetic bead separation prior to CAR T cell product formulation.

The present invention achieves more efficient and cost-effective manufacture of CAR NK cells using gene editing strategies, compared to other CAR NK cell approaches in the prior art, as disclosed in the examples. There is also a reduced risk of malignant transformation when using the sequence-specific gene editing technique of the present invention compared to viral vector modified cells, due to a reduced risk of insertional mutagenesis and lower transgene copy numbers.

Additional advantages are evident over the prior art approaches. For example, Jullierat et al¹⁶ shows about 0.5%-1.0%residual CD3+/TCRαβ+ T cells in the final mixture after CAR NK cell treatment; therefore the prior art methods seem to have less reproducible reduction of unwanted T cells below 0.5% CD3+ T cells. A further improvement in the present method over Jullierat et al¹⁶ is that they observed skewing toward CD8+ T cells. This is suboptimal and in contrast to the present method, which actually increases the amount of CD4+ cells relative to the control (untreated) conditions. An additional risk to the approach of Jullierat et al¹⁶ is that the CAR could trigger additional differentiation of the CAR T cells, introducing a potentially disruptive feature to the cells of the prior art. Thus, the approach employed herein using gene editing techniques to generate anti-TCR CAR NK cells is associated with multiple unexpected advantages over the prior art.

The present invention thus achieves, without limitation, at least two significant advantages which allow improvements over the prior art, such as (1) a significant quality improvement of the therapeutic cell material due to the unprecedented high purity of the TCR- T cell population - leading to a highly decreased risk of GvHD, and (2) the high amount of cell loss due to the column-based MACS approach can be avoided completely.

### Embodiments relating to the modified CAR NK cells and CAR:

In embodiments of the invention, the component of the T cell receptor complex bound by the CAR (in the modified NK cell) is CD3, preferably CD3 epsilon, or a TCR component, preferably a TCR alpha, beta, gamma and/or delta, preferably a TCR alpha/beta dimer.

The invention is thus not limited to the particular TCR/CD3 target against which the CAR NK cell is targeted. Effective recognition of TCR+ T cells by the CAR NK cell leads to cytotoxicity against such TCR+ T cells. Preferred and non-limiting CD3-TCR complex targets are therefore CD3 or TCR alpha, beta, gamma and/or delta targets, preferably CD3 epsilon or a TCR alpha/beta dimer.

The TCR typically comprises a TCRalpha/beta dimer or a TCRgamma/delta dimer. CD3 typically comprises 6 individual protein chains (1x CD3 gamma, 1x CD3 delta, 2x CD3 epsilon, 2x CD3 zeta). TCR and CD3 together form the TCR complex (or CD3-TCR complex; also termed TCR/CD3 complex). As used herein, the modified CAR NK cells preferably target the TCR, CD3 and/or TCR complex (CD3-TCR complex). References to TCR+ cells may be used interchangeably with TCR+ T cells, references to TCR- cells may be used interchangeably with TCR- T cells. References to the TCR are considered to include reference to the TCR complex (or CD3-TCR complex), as may be understood by a skilled person and within the function of the present invention.

As used herein, the NK cells of the invention may be termed a modified CAR NK cell, or TCR/CD3-specific CAR NK cell, wherein references to CAR NK-92 cells are not considered limiting. Any disclosure relating to a CAR NK-92 cells may be considered reference to a modified CAR NK cell of the invention.

In preferred embodiments of the invention, the modified CAR NK cell is an NK-92 cell in which the nucleic acid sequence encoding a chimeric antigen receptor (CAR) is integrated. As shown in the examples, the NK-92 cell line exhibits the desired cytotoxicity and exhibits various positive properties in the context of the invention. NK-92 cells are an interleukin-2 (IL-2) dependent natural killer cell line that are cytotoxic to a wide range of malignant cells, but also to T cells in co-culture.

The present invention employs modified CAR NK cells comprising an exogenous nucleic acid sequence encoding a chimeric antigen receptor (CAR), wherein said nucleic acid sequence is integrated in the NK cell genome without a viral vector. In embodiments, the nucleic acid encoding the CAR is integrated in the host NK cell genome by a gene editing technique. For example, the gene editing technique comprises one or more of CRISPR-Cas, zinc finger nucleases (ZFNs), integrases, site specific recombinases, meganucleases, homing endonucleases, or TALENs. The term "without a viral vector" may be termed as "with a gene editing technique" or "using a gene editing technique". Such gene editing techniques are known to a skilled person and are easily differentiated from viral vector-based approaches towards integrating a CAR encoding sequence. The resulting genetic modification after using a gene editing technique is clearly distinguished from the resulting genetic modification after modification of the cells using a viral vector. Routine approaches involving sequencing of genetic modifications may be employed to differentiate the two approaches.

In preferred embodiments the CAR is introduced into the NK cell genome using a gene editing technique to create a double strand break, preferably at a target site for the intended integration, and the nucleic acid construct is incorporated into the target site by homology directed repair (HDR) at the site of the double-strand break. A non-viral vector is preferably used for transferring the nucleic acid construct encoding the antigen binding domain fragment into the cell. In one embodiment, a delivery method is non-viral, preferably electroporation. In one embodiment, the invention provides means to insert the CAR encoding transgene into an NK cell using homology-directed repair (HDR). In one embodiment, the CAR encoding nucleic acid construct together with a CRISPR-Cas ribonucleoprotein (Cas) complexed with a guide RNA (gRNA) is transferred into the T cell. In one preferred embodiment the CRISPR-Cas is CRISPR-Cas9.

### Embodiments related to a CAR employed in the modified CAR NK cells of the invention:

Aspects of the invention relate to the CAR encoding nucleic acid construct, the CAR polypeptide and CAR modified cells, as described herein.

In embodiments of the invention, the CAR that specifically binds a component of a T cell receptor complex comprises:
a. an extracellular antigen-binding domain that specifically binds a component of a T cell receptor complex, preferably specifically binds CD3 epsilon or a TCR alpha/beta dimer, preferably comprising an antibody or antigen binding fragment thereof,
b. an extracellular hinge and/or transmembrane domain, preferably a CD8α or a CD28 extracellular hinge and/or transmembrane domain, and
c. an intracellular domain, preferably comprising a 4-1BB and/or a CD28 co-stimulatory domain, optionally comprising additionally a CD3zeta (4-1BB or CD28) signaling domain.

In one embodiment, the CAR that specifically binds a component of a T cell receptor complex comprises:
- an extracellular antigen-binding domain, comprising an antibody or antibody fragment that binds a T cell receptor complex component, preferably CD3 epsilon or a TCR alpha/beta dimer, wherein said antibody or antibody fragment comprises VH and VL domains of a single chain antibody fragment, wherein preferably a linker polypeptide is positioned between the VH and VL domains, wherein said linker is preferably configured to not interfere with the antibody fragment-antigen (CD3 epsilon or a TCR alpha/beta dimer) interaction;
- optionally a spacer polypeptide (also referred to as a hinge) positioned between the extracellular antigen-binding domain and a transmembrane domain, wherein said spacer polypeptide is preferably configured to not interfere with the antibody fragment-antigen interaction and/or with NK cell activation when said CAR is expressed in an NK cell;
- a transmembrane domain, wherein said transmembrane domain is preferably configured to not interfere with the antibody fragment-antigen interaction and/or with NK cell activation when said CAR is expressed in a NK cell;
- and an intracellular domain, wherein said intracellular domain comprises a co-stimulatory domain and a signalling domain, wherein said intracellular domain is preferably configured to provide signals to stimulate NK cell activation upon binding to the TCR component target, for example by increasing cytokine production, thus leading to cytotoxic effects.

The CAR of the present invention may therefore employ various formats, comprising potentially different protein sequences for each of the functional domains described herein. A skilled person is capable of selecting and testing the desired function of the CARs, for example based on the experimental approaches demonstrated in the examples below. As such, the election of any given specific protein sequence to be used in the CAR of the invention, in any of the functional domains discussed herein, can be assessed by a skilled person using routine methods for functional efficacy. For example, various linker polypeptide sequences positioned between the VH and VL domains, various spacer polypeptide sequences (also referred to as a hinge) positioned between the extracellular antigen-binding domain and a transmembrane domain, various transmembrane domains and various intracellular domains, preferably comprising co-stimulatory and signalling domains, may be employed.

### Embodiments relating to CAR antigen binding domain sequences:

In one embodiment, the chimeric antigen receptor (CAR) binding to a TCR complex target as described herein comprises an antigen binding domain that binds TCR alpha/beta, wherein the antigen-binding domain comprises a variable heavy chain (VH), said VH comprising:
- a heavy chain complementary determining region 1 (H-CDR1) with at least 80% sequence identity to SEQ ID NO 33 (SYVMH),
- a heavy chain complementary determining region 2 (H-CDR2) with at least 80% sequence identity to SEQ ID NO 34 (YINPYNDVTKYNEKFKG), and
- a heavy chain complementary determining region 3 (H-CDR3) with at least 80% sequence identity to SEQ ID NO 35 (GSYYDYDGFVY),
and a variable light chain (VL), said VL comprising:
- a light chain complementary determining region 1 (L-CDR1) with at least 80% sequence identity to SEQ ID NO 36 (SATSSVSYMH),
- a light chain complementary determining region 2 (L-CDR2) with at least 80% sequence identity to SEQ ID NO 37 (DTSKLAS), and
- a light chain complementary determining region 3 (L-CDR3) with at least 80% sequence identity to SEQ ID NO 38 (QQWSSNPLT).

The above-mentioned CDR sequences are derived from the antigen binding domain, preferably a short chain variable fragment, from the muBMA13 VH and VL domains, as used in the examples below. This antigen binding domain specifically binds TCR alpha/beta as in CAR1 (TCRα/β.CD28.CD3ζ) and CAR3 (TCRα/β.CD8α.4-1BB.CD3ζ).

In one embodiment, the CAR binding to TCR alpha/beta as described herein comprises an antigen binding domain, wherein the antigen-binding domain comprises a variable heavy chain (VH), that comprises CDR sequences of SEQ ID NO. 33, SEQ ID No. 34 and SEQ ID NO. 35, and a VL domain that comprises CDR sequences of SEQ ID NO. 36; SEQ ID NO 37, and SEQ ID NO. 38.

In preferred embodiments, the sequence variants with 80% or more sequence identity to the specific CDR sequences of SEQ ID 33-38 maintain TCR alpha/beta binding with essentially the same or similar functional properties as VH and VL domains with the specific CDR sequences of SEQ ID NO 33-38, i.e. the TCR alpha/beta binding is essentially the same or similar with respect to affinity, specificity and/or epitope binding mode.

In embodiments of the invention, the extracellular antigen-binding domain comprises a single chain variable fragment (scFv) that binds TCR alpha/beta, preferably according to SEQ ID NO 21, or sequences with at least 80% identity thereto.

In embodiments of the invention, the extracellular antigen-binding domain comprises a VH and a VL domain, wherein the VH domain comprises or consists of a sequence according to SEQ ID NO 31, or sequences with at least 80% identity thereto, and wherein the VL domain comprises or consists of a sequence according to SEQ ID NO 32, or sequences with at least 80% identity thereto.

In one embodiment, the chimeric antigen receptor (CAR) binding to a TCR complex target as described herein comprises an antigen binding domain that binds CD3 epsilon, wherein the antigen-binding domain comprises a variable heavy chain (VH), said VH comprising:
- a heavy chain complementary determining region 1 (H-CDR1) with at least 80% sequence identity to SEQ ID NO 41 (RYTMH),
- a heavy chain complementary determining region 2 (H-CDR2) with at least 80% sequence identity to SEQ ID NO 42 (YINPSRGYTNYNQKFKD), and
- a heavy chain complementary determining region 3 (H-CDR3) with at least 80% sequence identity to SEQ ID NO 43 (YYDDHYSLDY),
and a variable light chain (VL), said VL comprising:
- a light chain complementary determining region 1 (L-CDR1) with at least 80% sequence identity to SEQ ID NO 44 (SASSSVSYMN),
- a light chain complementary determining region 2 (L-CDR2) with at least 80% sequence identity to SEQ ID NO 45 (DTSKLAS), and
- a light chain complementary determining region 3 (L-CDR3) with at least 80% sequence identity to SEQ ID NO 46 (QQWSSNPFT).

The above-mentioned CDR sequences are derived from the antigen binding domain, preferably a short chain variable fragment, from the OKT3 VH and VL domains, as used in the examples below. This antigen binding domain specifically binds CD3 epsilon as in CAR2 (CD3ε.CD28.CD3ζ) and CAR4 (CD3ε.CD8α.4-1BB.CD3ζ).

In one embodiment, the CAR binding to CD3 epsilon as described herein comprises an antigen binding domain, wherein the antigen-binding domain comprises a variable heavy chain (VH), that comprises CDR sequences of SEQ ID NO. 41, SEQ ID No. 42 and SEQ ID NO. 43, and a VL domain that comprises CDR sequences of SEQ ID NO. 44; SEQ ID NO 45, and SEQ ID NO. 46.

In preferred embodiments, the sequence variants with 80% or more sequence identity to the specific CDR sequences of SEQ ID 41-46 maintain CD3 epsilon binding with essentially the same or similar functional properties as VH and VL domains with the specific CDR sequences of SEQ ID NO 41-46, i.e. the CD3 epsilon binding is essentially the same or similar with respect to affinity, specificity and/or epitope binding mode.

In embodiments of the invention, the extracellular antigen-binding domain comprises a single chain variable fragment (scFv) that binds CD3 epsilon, preferably according to SEQ ID NO 22, or sequences with at least 80% identity thereto.

In embodiments of the invention, the single chain variable fragment (scFv) that binds CD3 epsilon comprises a VH and a VL domain, wherein the VH domain comprises or consists of a sequence according to SEQ ID NO 39, or sequences with at least 80% identity thereto, and wherein the VL domain comprises or consists of a sequence according to SEQ ID NO 40, or sequences with at least 80% identity thereto.

Furthermore, the order of the light and heavy chain fragments may be inverted upon the desired configuration of the antigen binding fragment. The specific VH-VL arrangements provided in the examples are non-limiting.

Additionally, in some embodiments a linker sequence between heavy and light chains can be employed, and may vary from the specific linker sequence disclosed in the examples, for example it may have been modified, for example by shortening, in order to enhance the CAR function.

Additionally, the nucleic acid sequence encoding the CAR may be codon-optimized in order to improve expression of the CAR.

These modifications enable sufficient surface expression on NK cells and still maintain proper antigen binding. High affinity and high avidity enable CAR NK cells to i) recognize, ii) be activated against, and iii) kill target cells with high, intermediate or low TCR expression and/or surface presentation.

### Embodiments relating to other CAR sequences:

In one embodiment, the antigen-binding domain of the CAR comprises a CD8a derived membrane export signal, for example according to SEQ ID NO 19, or a sequence with at least 80% sequence identity to SEQ ID NO 19.

In one embodiment, the antigen-binding domain of the CAR comprises a myc tag, for example according to SEQ ID NO 20, or a sequence with at least 80% sequence identity to SEQ ID NO 20.

In one embodiment, the antigen-binding domain of the CAR comprises a linker polypeptide positioned between the VH and VL domains, wherein said linker is preferably selected from
- Gly-Ser (SEQ ID NO 47) linker, or
- a Whitlow (SEQ ID NO 48), or
- linkers with at least 80% sequence identity to SEQ ID NO 47 or 48.

In one embodiment, the CAR comprises optionally additionally a spacer polypeptide positioned between the antigen-binding domain and the transmembrane domain, wherein said spacer is preferably selected from:
- IgG1 spacer (SEQ ID NO 49),
- IgG1Δ spacer (SEQ ID NO 50),
- IgG4 (Hi-CH2-CH3) spacer (SEQ ID NO 51),
- IgG4 (Hi-CH3) spacer (SEQ ID NO 52),
- IgG4 (Hi) spacer (SEQ ID NO 53), or
- a spacer with at least 80% sequence identity to any one of SEQ ID NO 49-53.

In one embodiment, the invention relates to a chimeric antigen receptor (CAR) polypeptide as described herein, wherein the transmembrane domain is selected from:
- a CD28 domain (SEQ ID NO 23), or
- a CD8α domain (SEQ ID NO 24), or
- transmembrane domains with at least 80% sequence identity to SEQ ID NO 23 or 24.

In one embodiment, the invention relates to a chimeric antigen receptor (CAR) polypeptide as described herein, wherein the intracellular domain comprises:
- a co-stimulatory domain selected from a 4-1BB co-stimulatory domain (SEQ ID NO 25), or
- a co-stimulatory domain with at least 80% sequence identity to SEQ ID NO 25.

In one embodiment, the invention relates to a chimeric antigen receptor (CAR) polypeptide as described herein, comprising additionally a signaling domain (otherwise known as an activation domain), wherein said signaling domain is
- a CD3zeta (4-1BB or CD28) signaling domain (SEQ ID NO 26), or
- a signaling domain with at least 80% sequence identity to SEQ ID NO 26.

In one embodiment, the CAR as described herein comprises or consists of a sequence according to any one of SEQ ID NO 27, 28, 29 or 30.

In some embodiments, the transmembrane domain comprises a protein selected from the group consisting of the alpha, beta or zeta chain of the T cell receptor, CD28, CD3 epsilon, CD45, CD4, CD5, CD8, CD9, CD16, CD22, CD33, CD37, CD64, CD80, CD86, CD134, CD137, and CD154.

In some embodiments, the intracellular signaling domain comprises a costimulatory signaling domain comprising a functional signaling domain obtained from a protein selected from the group consisting of a MHC class I molecule, a TNF receptor protein, an Immunoglobulin-like protein, a cytokine receptor, an integrin, a signaling lymphocytic activation molecule (SLAM protein), an activating NK cell receptor, gp130, BTLA, a Toll ligand receptor, OX40, CD2, CD7, CD27, CD28, CD30, CD40, CDS, ICAM-1, LFA-1 (CD11a/CD18), 4-1BB (CD137), B7-H3, CDS, ICAM-1, ICOS (CD278), GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, CD19a or derivates thereof with at least 80% amino acid sequence identity.

In a further aspect of the invention, the invention relates to an isolated nucleic acid molecule, selected from the group consisting of:
i. a nucleic acid molecule comprising a nucleotide sequence,
   which encodes a CAR polypeptide according to any embodiment described herein,
   which encodes an extracellular antigen-binding domain, a transmembrane domain, and an intracellular co-stimulatory domain, or
   wherein the extracellular antigen-binding domain is encoded by at least one sequence of SEQ ID NO 5 or 6,
   wherein the transmembrane domain is encoded by at least one sequence of SEQ ID NO 7 or 8,
   wherein the intracellular co-stimulatory domain is encoded by at least one sequence of SEQ ID NO 9 or 10,
   and/or
ii. a nucleic acid molecule comprising a nucleotide sequence,
   which encodes a targeting sequence comprising identical sequences to a recognition site of an endogenous DNA sequence into which integration is intended, also termed homology arms, according to any embodiment of the CAR fragment described herein, wherein the homology arm is according to at least one sequence of SEQ ID NO 1 or 13, and/or
iii. a nucleic acid molecule comprising a nucleotide sequence,
   which encodes a CAR polypeptide as described herein, for example according to SEQ ID NO 14, 15, 16 or 17, and/or
iv. a nucleic acid molecule which is complementary to a nucleotide sequence in accordance with i, ii and/or iii,
v. a nucleic acid molecule comprising a nucleotide sequence having sufficient sequence identity to be functionally analogous/equivalent to a nucleotide sequence according to i-iv, comprising preferably a sequence identity to a nucleotide sequence according to i-iv of at least 70%, 80%, 90% or 95%;
vi. a nucleic acid molecule which, as a consequence of the genetic code, is degenerate to a nucleotide sequence according to i-v; and/or
vii. a nucleic acid molecule according to a nucleotide sequence of i-vi which is modified by deletions, additions, substitutions, translocations, inversions and/or insertions and is functionally analogous/equivalent to a nucleotide sequence according to i-vi.

In some embodiments, the nucleic acid molecule includes a targeting sequence to integrate the CAR into a preferred site of the NK cell genome.

For example, a targeting sequence may comprise an identical sequence to a recognition site of the endogenous DNA sequence into which integration is intended, preferably selected from the group consisting of a homology arm, a guide RNA target site, a restriction enzyme recognition site, a ZFN recognition site, a ZFN cleaving site, a TALEN DNA-binding site, a recombinase recognition site, an integrase site and/or a homing nuclease recognition site.

In certain embodiments, the targeting sequence, also termed "homology arm", comprises at least one sequence complementary to an endogenous genomic sequence of the target cell. In embodiments, the homology arms are defined by SEQ ID 1 and/or 13.

In some embodiments, the nucleic acid construct comprises in 5' to 3' order: optionally a first vector DNA sequence, a left homology arm, a promoter, a membrane export signal, a tag, such as a myc tag, a CAR-encoding region, an adapter for cloning, a polyA sequence, and a right homology arm. In some embodiments, no vector sequence is required or used. Details are provided in the sequence table disclosed herein.

In embodiments, the nucleic acids are introduced into the cell by homology directed repair (HDR). In some embodiments, a homology directed repair (HDR) template is a nucleic acid molecule, preferably a double strand DNA, also used in the homology directed repair gene editing based technique. In certain embodiments of the method for generating a genetically modified NK cell, the double strand DNA repair template is a homology directed repair (HDR) template. In some embodiments, a homology directed repair (HDR) template is generated as described in Roth et al. (2018 Nature).

Genome editing through HDR is advantageous for the specific and efficient and viral vector free insertion of large transgenes into target cells (NK cells). It is advantageous that the viability and cell function of the transfected cells is maintained and allows a high yield of genetically modified cells for application in the method of the invention.

Additional sequences are presented in the sequence herein and may be employed by a skilled person, as in the examples, as necessary.

### Embodiments relating to cell mixtures and methods:

In one embodiment, the genetically modified NK cell of the invention is present in an in vitro mixture with:
   a. a first genetically modified T cell, preferably a CAR T cell, comprising one or more disruptive modifications to endogenous TCR components, and
   b. optionally a second T cell, comprising an endogenous TCR expressed and/or presented on the cell surface.

In embodiments, the first genetically modified T cell, preferably a CAR T cell, comprising one or more disruptive modifications to endogenous TCR components, is a therapeutic T cell, in which the TCR has been disrupted in order to reduce the risk of unwanted immune reactions upon (allogeneic) administration to a patient. This cell type may also be referred to as TCR- cell or TCR-T cell. For example, a component of the TCR or TCR complex may have been mutated, knocked-out or RNAi-inhibited, such as disruption of a TCR alpha constant (TRAC) gene and/or CD3 gene. In embodiments, "disruptive modifications to endogenous TCR components" may also include "disruptive modifications to endogenous TCR complex components".

In embodiments, the second T cell, comprising an endogenous TCR expressed and/or presented on the cell surface, is an "unwanted" cell that remains in a therapeutic cell population and may cause an unwanted immune reaction, e.g. GvHD, after allogeneic transplantation to a subject. This cell type may also be referred to as TCR+ cell.

As described above, even though gene-editing procedures have been improved to achieve very high knockout rates in removing unwanted TCRs in allogeneic cell products, conventional manufacturing strategies still typically require a purification step prior to the final formulation in order to remove residual TCR+ T cells. Previous clinical trials with patients undergoing haploidentical stem cell transplantation have estimated that residual TCRαβ⁺ T cells may lead to the occurrence of GvHD. Clinical case reports using un-matched allogeneic CAR T cells demonstrated that as little as 1-2% contaminating TCRαβ⁺ T cells in gene-edited cell products have the capacity to induce GvHD in heavily pre-treated leukemia patients.

Various cell populations and/or mixtures cells are therefore aspects of the invention. The modified CAR NK cells of the invention may in embodiments be present in a mixture of cells with either the first genetically modified T cell (desired therapeutic cell product) and/or the second T cell (unwanted TCR+ residual contaminant).

With respect to the first genetically modified T cell, preferably a CAR T cell, comprising one or more disruptive modifications to endogenous TCR components, the invention contemplates any given disruption to a TCR component in such cells. Various TCR disruptions may be employed and are known to a skilled person. For example, the T cells may have a genetic disruption in one or more TCR and/or TCR complex encoding genes. Other options relate to genes encoding TCR components, preferably a disruption in the coding sequence and/or expression of genes such as TCR alpha constant (TRAC) and/or TCR beta constant 1 and 2 (TRBC1 and TRBC2); and/or TCR complex components, preferably CD3 zeta (CD247) and/or CD3 epsilon (CD3E) and/or CD3G. Other options represent any given TCR component, such as, without limitation, TRAC, TRBC1, TRBC2, TRDC, TRGC1, TRGC2, CD247 (CD3 zeta), CD3D, CD3E, CD3G, or others.

In embodiments, other options for TCR disruption include, without limitation, cells in which surface TCRαβ has been removed. For example, to remove surface TCRαβ, an antibody-derived single-chain variable fragment specific for CD3e may retain TCRαβ intracellularly protein expression blockers (PEBLs). After transduction in T cells, several of these protein expression blockers (PEBLs) colocalize intracellularly with CD3ε, blocking surface CD3 and TCRαβ expression. A skilled person may also be able to design PEBLs for other CD3-TCR complex components to prevent its expression to the surface.

In embodiments, other options include in essence any modification fully or partially disruptive to surface expression of TCR and/or TCR complex components encoded by their respective genes.

By bringing the modified CAR NK cells of the invention into mixtures of therapeutic T cells comprising one or more disruptive modifications to endogenous TCR components, any residual T cells comprising an endogenous TCR expressed and/or presented on the cell surface can be eliminated, or reduced to very low relative cell numbers in the mixture, by the cytotoxic activity of the modified CAR NK cells.

A further aspect of the invention therefore relates to a method for destroying a T cell, the method comprising contacting:
a. a genetically modified natural killer (NK) cell, according to any one of claims 1 to 6, with
b. a T cell comprising an endogenous TCR expressed and/or presented on the cell surface.

In embodiments, the invention relates to a method for destroying a T cell, either genetically modified or unmodified, for example a CAR T cell, comprising an endogenous TCR expressed and/or presented on the cell surface, the method comprising contacting:
a. a genetically modified natural killer (NK) cell, as described herein, with
b. a T cell, either genetically modified or unmodified, for example a CAR T cell, comprising an endogenous TCR expressed and/or presented on the cell surface (second

### T cell mentioned above).

As portrayed in detail herein, during production of genetically modified T cells for therapeutic use, in which preferably a TCR component or TCR complex component is removed or disrupted, for example when said therapeutic cells are intended for allogeneic use, not all T cells in the in vitro cell population are effectively converted into TCR- T cells. It is possible that one target allele (target of the TCR disruption) within the T cells is modified, but not the other. It is also possible that some T cells are not modified at all. It is also possible, in the context of CAR T cells, that the T cell is successful modified to express a CAR, but the TCR locus is not effectively disrupted. The elimination of such residual TCR+ T cells is thus achieved using the NK cells of the invention. Thus, in embodiments, the T cell to be destroyed may be genetically modified or unmodified. The T cell to be destroyed may be a CAR T cell, expressing a CAR but with an (unwanted) intact TCR (TCR+ T cells).

According to the invention "contacting" refers to any condition or environment of a cellular mixture, in which the modified CAR NK cells of the invention may exhibit a cytotoxic activity against the second T cell. The term "destroying" in this context refers to a disabling, destructive, killing (cytotoxic) and/or otherwise incapacitating effect against the second T cell. A skilled person is capable of determining said destroying using routine methods in the art.

In one embodiment, the T cell comprising an endogenous TCR expressed and/or presented on the cell surface (second T cell mentioned above) is in a mixture with a genetically modified T cell comprising one or more disruptive modifications to endogenous TCR components (first genetically modified T cell mentioned above).

In such embodiments, although the primary goal of the method is to destroy TCR+ T cells, this may occur in a mixture of TCR+ and TCR- T cells, whereby TCR- T cells are the primary component (measured by relative cell number) of a mixture, from which the (residual) TCR+ T cells are to be removed.

The method of the invention thus represents a novel strategy to purify TCR-disrupted T cells via co-culture with modified CAR NK cells of the invention, preferably gamma-irradiated TCR/CD3-specific CAR NK-92 cells, during ex vivo expansion. As disclosed in the examples below, the invention provides multiple efficient TCR alpha/beta-specific and CD3-specific CAR constructs in NK-92 cells that enable a process with purification rates exceeding 99.9% TCR- T cells. The method thus enables a >10-fold improvement over magnetic cell separation and shows additional advantages over other approaches in the prior art. Co-cultures with modified CAR NK cells of the invention, preferably irradiated TCR/CD3-specific CAR NK-92 cells, appear to also support CAR T cell expansion and do not negatively impact in vitro CAR T cell function. Thus, the method of the invention enables a surprising double advantage, including both removal of unwanted TCR+ T cells, thereby reducing risk of an unwanted immune reaction post-administration, and also improves function and culturing of the remaining wanted TCR- T cell population.

In embodiments, the method may therefore be carried out for the purification and/or enrichment of a (first; TCR-) genetically modified T cell comprising one or more disruptive modifications to endogenous TCR components.

In embodiments, the modified CAR NK cell of the invention is irradiated prior to contacting with the genetically modified T cell(s). The irradiation is preferably a gamma irradiation.

Through this step, the NK cells are not long-lived in culture and are effectively predestined for "self-removal" from the final therapeutic T cell population. Gamma-irradiation of NK cells, preferably NK-92 derived cell lines, is an established standard safety measurement taken before their clinical application which prevents their outgrowth without affecting their short-term cytotoxicity. In the context of the present invention, the gamma irradiation of the NK cells surprisingly had no detrimental effect on cytotoxicity against TCR+ T cells. The NK cells of the invention were surprisingly capable of effective TCR+ T cell removal within the time frame of NK cell activity post irradiation.

In embodiments, gamma-irradiation may be carried out at 1 to 200 Gy, preferably 10 to 100 Gy, more preferably 20 to 60 Gy, or at about 30 Gy.

In embodiments, the CAR NK cell may be inactivated sing alternative methods, either in place of or in combination with irradiation. For example, to prevent NK cell proliferation, other forms of irradiation, such as low energy irradiation, may be applied. As disclosed in Walcher et al (Front Immunol. 2021 Jun 4;12:684052) low energy electron irradiation is a functional alternative to gamma irradiation for the inactivation of (CAR) NK cells in cell manufacturing.

In embodiments, the modified CAR NK cell of the invention, preferably a TCR/CD3-specific CAR NK-92 cell, is added in relative ration to the T cells (NK:T cell ratio) of 100:1 to 1:100, or 10:1 to 1:10, or 5:1 to 1:1, preferably at about a 2:1 cellular ratio to the expanding CAR T cells.

In embodiments, the modified CAR NK cells of the invention are administered to the T cells after 1 to 21 days, preferably 3 to 10, or 4 to 8 days, more preferably on day 7 after T cell isolation.

In embodiments, the mixture of genetically modified T cells is administered one or more, preferably two or more, treatments of the NK cells of the invention. In embodiments, the mixture of genetically modified T cells is administered 1, 2, 3, 4, 5, 6, or more NK cell treatments.

In embodiments, the modified CAR NK cells of the invention are administered again to the T cells after 1 to 21 days after the first administration, preferably 3 to 10, or 4 to 8 days, more preferably on day 6 or 7 after the first NK cell administration.

A single administration of NK cells to T cells proved effective. However, in a preferred embodiment, two consecutive administrations (co-culture cycles) were more effective than 1 administration, when using the conditions of the examples described below. For example, in embodiments, NK cells are administered about 7 days after T cell isolation (culturing). Following that, six days after the initial NK/T cell co-culture (about day 13 after T cell isolation), the purity of TCR/CD3-deleted CAR T cells was 63 % without NK cell treatment, 99.8% with a single NK cell treatment, and >99.98% with two rounds of NK cell treatment. This translates to a removal of 98.6% of residual wildtype T cells through a single administration and more than 99.95% through 2 co-culture cycles.

This represents a surprising improvement over methods of the prior art. For example, CD3-MACS-depletion, also performed on day 13 after T cell isolation for comparison in an independent experiment, only increased product purity from 88.1% to 99.67%, which corresponds to a removal of contaminating TCR/CD3+ T cells of only 97.3%. Thus, frequencies of potentially allo-reactive wildtype T cells (TCR+) achieved with the invention method are approx. 19-fold lower than with classic bead-mediated depletion.

As shown in the examples below, higher NK cell amounts (eg CAR4 NK-92 amounts) during co-culture and additional (for example a second, third or fourth) NK cell administration (clean-up) in co-culture tended to improve the purity of TCR- T cells. Similarly, increasing the time between first and second co-culture (to more than 6 days between first and second NK cell treatment time points) can, in embodiments, lead to improved purity and more sustained TCR-depletion in the desired therapeutic T cell population.

In embodiments, the mixture of genetically modified T cells after NK cell treatment comprises at least 99.5% genetically modified T cells comprising one or more disruptive genetic modifications to TCR component-encoding genes (TCR- T cells), preferably at least 99.9%.

The percentage of cells in this embodiment represents a relative cell count. For example, the TCR-T cells are in mixture with TCR+ T cells, and the TCR- T cells make up at least 99.5% of total cell numbers. In other embodiments, TCR- T cells make up at least 99.5% of total cell numbers, preferably 99.6%, 99.7%, 99.8%, 99.9%, or 99.91%, 99.92%, 99.93%, 99.94%, 99.95%, 99.96%, 99.97%, 99.98%, 99.99%, 99.991%, 99.992%, 99.993%, 99.994%, 99.995%, or higher.

The invention therefore further relates to a method for manufacturing a therapeutic cell composition, preferably a genetically modified T cell, more preferably a CAR T cell, comprising one or more disruptive modifications to endogenous TCR components, the method comprising purifying and/or enriching said cells using the method as described herein.

As described at length herein, CAR T cells now represent an established and potent therapy for various medical indications, in particular for treating hematological malignancies. Multiple CAR T products have regulatory approval and in essence any given CAR T cell product in which TCR complex components have been removed may benefit from the present invention. There is a growing interest in developing allogeneic CAR T cells, thus enabling large-scale manufacturing of allogeneic off-the-shelf CAR T cell products to reduce costs and simplify the clinical logistics associated with autologous cell manufacturing.

The presented strategy may be used to improve CAR T cell yields for any allogeneic CAR T cell product which have been previously described, including, without limitation, donor-derived CAR T cells for leukemia after allogeneic hematopoietic stem cell transplantation, universal off-the-shelf CAR T cells for T cell malignancies, multiple myeloma or even CAR T cells for solid tumors. Hypo-immunogenic cell products with B2M-KO overexpressing NK cell inhibitory receptors, such as HLA-E or CD47, may be combined with our NK-92 cell based approach in future studies.

In embodiments, the method is carried out using a bioreactor, preferably closed-system bioreactor, such as the G-Rex system from Wilson Wolf. Such closed-system bioreactors are designed for predictable, efficient, and scalable cell expansion and enable production of large numbers of cells needed for cell-based therapeutics, from preclinical through commercial scale manufacturing.

As shown in the examples below, the modified CAR NK cells of the invention supported the expansion of TCR-edited CAR T cells, thus demonstrating a "feeder cell effect". Surprisingly, the invention therefore achieved a cultivation advantage for the therapeutic T cells. For example, by enhancing both the product purity and optimizing the overall cell yield, the invention increases the safety of high dosages of allogeneic CAR T cells without exceeding the TCR⁺ T cell per kg bodyweight thresholds associated with GvHD.

The invention may also be carried out in other bioreactor systems, such as semi-closed G-rex culture devices. The approach described herein is considered in essence to be compatible with any given bioreactor systems or automated cell manufacturing devices, whereby closed-system bioreactors may be preferred.

### Embodiments relating to cell products:

A further aspect of the invention relates to an in vitro cell population obtained by the method of the invention.

In embodiments, the mixture comprises:
a. first genetically modified T cells, preferably CAR T cells, comprising one or more disruptive modifications to endogenous TCR components, and
b. second T cells, comprising an endogenous TCR expressed and/or presented on the cell surface,
wherein the cell population comprises at least 99.5% (by cell number) of said first genetically modified T cells, preferably at least 99.9%. The use of NK cells thus enables this high level of purity, which has to the knowledge of the inventors not been reliably demonstrated in the prior art.

As described for the method, the cell population may be defined by the cell "purity". In embodiments, the mixture of genetically modified T cells after NK cell treatment comprises at least 99.5% genetically modified T cells comprising one or more disruptive genetic modifications to TCR component-encoding genes (TCR- T cells), preferably at least 99.9%. The percentage of cells in this embodiment represents a relative cell count. For example, the TCR- T cells are in mixture with TCR+ T cells, and the TCR- T cells make up at least 99.5% of total cell numbers. In other embodiments, TCR- T cells make up at least 99.5% of total cell numbers, preferably 99.6%, 99.7%, 99.8%, 99.9%, or 99.91%, 99.92%, 99.93%, 99.94%, 99.95%, 99.96%, 99.97%, 99.98%, 99.99%, 99.991%, 99.992%, 99.993%, 99.994%, 99.995%, or higher.

As shown in the examples, the resulting cell population may be defined by relative amounts of TCR- and TCR+ T cells. For example, the purity of TCR/CD3-deleted CAR T cells was 63 % without NK cell treatment, 99.8% with a single NK cell treatment, and >99.98% with two rounds of NK cell treatment. This translates to a removal of 98.6% of residual wildtype T cells through a single administration and more than 99.95% through 2 co-culture cycles. This represents a surprising improvement over methods of the prior art. For example, CD3-MACS-depletion only increased product purity from 88.1% to 99.67%, which corresponds to a removal of contaminating TCR/CD3+ T cells of only 97.3%. Thus, frequencies of potentially allo-reactive wildtype T cells (TCR+) achieved with the invention method are approx. 19-fold lower than with classic bead-mediated depletion.

In embodiments, the in vitro cell population may comprise the modified CAR NK cells of the invention, or may not comprise the modified CAR NK cells of the invention. This can depend on the time point upon which the presence of NK cells is assessed. During production (co-culture), NK cells may still be evident. At later stages, for example in a final therapeutic T cell product, NK cells may no longer be present. Regardless, the resulting amounts of TCR- T cells, for example in relative proportion to TCR+ T cells, represents a novel in vitro cell population.

Alternative approaches in the prior art, such as in Juillerat et al, show about 0.5-1.0% residual CD3+/TCRαβ+ T cells in the final mix; therefore, the prior art method seems to have less reproducible reduction below 0.5% TCR+ T cells. Furthermore, the present cell population may differ from cell populations of the prior art due to the ration of CD4:CD8 cells. Methods of the prior art show skewing towards more CD8+ T cells. This contrasts with the present method, which actually increases the amount of CD4+ T cells relative to the untreated conditions.

In embodiments, the genetically modified T cells (TCR-) of the cell population is a CD4+ and/or CD8+ T cell, preferably a mixture of CD4+ and CD8+ T cells. These T cell populations, and preferably the composition comprising both CD4+ and CD8+ transformed cells, show particularly effective cytolytic activity against various solid or liquid tumors.

In one embodiment, the TCR- T cells comprise CD4+ and CD8+ T cells, preferably in a ratio of 1:10 to 10:1, more preferably in a ratio of 5:1 to 1:5, 2:1 to 1:2 or 1:1. Administration of anti-tumor modified CAR T cells at the ratios mentioned, preferably at about a 1:1 CD4+/CD8+ ratio, lead to beneficial characteristics during treatment of the diseases mentioned herein, for example these ratios lead to improved therapeutic response and reduced toxicity. The CD4:CD8 ratios may represent a novel feature over other cell populations produced in a similar manner.

Furthermore, cell populations of the prior art may exhibit the additional risk that the CAR could trigger differentiation of the CAR T cells, which appears to be largely avoided in the cell populations of the present invention.

A further aspect of the invention relates to the cell population as described herein for use in the treatment and/or prevention of a medical condition susceptible to T cell therapy in a subject.

As known to a skilled person, therapeutic T cells may be used in treating various medical conditions. The disease to be treated is not limiting to the present invention. In preferred embodiments, the cell population for medical use is allogeneic to said subject.

The antigen-specific CAR-T cells described herein are applicable in preferred embodiments for the treatment of patients with solid or liquid tumors that are not eligible for other therapies. More specifically, the embodiments of the invention relate to the treatment of the following patient populations:
i. patients with multidrug resistances,
ii. patients not eligible for allogeneic/hematopoietic stem cell transplantation,
iii. patients with co-morbidities that preclude further chemotherapies,
iv. patients suffering from solid and/or liquid cancers,
v. aged patients who do not tolerate chemotherapies,
vi. the CAR is applicable for salvage therapies even after progressive disease and multiple lines of other standard of care therapies have failed, and/or
vii. it is applicable even at low antigen density on target tumor cells, where antibodies can fail, and/or
viii. it is applicable as a monotherapy which is not the case for antibodies, and/or
ix. it is applicable in combination with one or more anti-cancer treatments, anti-cancer medicaments or transplantation.

In some embodiments, the cell population is for use in the treatment of a medical disorder associated with the presence of pathogenic cells expressing oncogenes, such as cancer cells, more preferably cancer stem cells of solid and hematologic malignancies.

In some embodiments, the cell population is for use in the treatment of an autoimmune disease or a graft versus host disease.

In one embodiment, the genetically modified cell is allogeneic with respect to a patient.

In one embodiment, the genetically modified cell is an allogeneic Treg with respect to a patient.

In one embodiment, the genetically modified cell is an allogeneic TCR gamma/delta T-cell with respect to a patient.

Thus, the production and use of allogeneic cells is one important aspect of the invention. Cells can be obtained from a subject different from the intended patient, therefore allogeneic cells. As used herein, a cell is "allogeneic" with respect to a subject if it or one of its progenitor cells is derived from another subject of the same species. As used herein, a cell is "autologous" with respect to a subject if it or its progenitor cells originate from the same subject. For example, allogeneic "off the shelf" CAR-T cell products, as described in the present invention, can be produced from cells of healthy donors in stock and are applicable to a large patient population, depending on the indication and/or tumor-specific antigen. The means provided by the invention enable to reduce the cost of individual doses of CAR-T cell products and failures in the production of CAR-T cells.

The most serious risk associated with the use of allogeneic T-cell products is an often-fatal acute graft-versus-host disease (GvHD), whereby the donor's (CAR-modified) T-cells recognize and attack the patient's tissue as "foreign" through their endogenous TCR. Due to the beneficial properties of the cells of the invention these risks are reduced compared to known approaches.

The particular CAR antigen of the therapeutic T cell is not limiting to the present invention.

In embodiments, the CAR antigen-binding domain (of the therapeutic TCR- CAR T cell) binds to a cancer antigen. In some embodiments, the antigen binding domain binds to a cancer associated antigen selected from the group consisting of: CD19, APRIL/TNFSF13, Mic A/B, T cell associated antigens: CD3, CD5, CD7, Folate receptor alpha (FRa), ERBB2 (Her2/neu), EphA2, IL-13Ra2, epidermal growth factor receptor (EGFR), Mesothelin, TSHR, CD123, CD22, CD30, CD171, CS-1, CLL-1, CD33, EGFRvlll, GD2, GD3, BCMA, Tn Ag, prostate specific membrane antigen (PSMA), ROR1, FLT3, FAP, TAG72, CD38, CD44v6, CEA, EPCAM, B7H3, KIT, interleukin-11 receptor a (IL-11Ra), PSCA, PRSS21, VEGFR2, LewisY, CD24, platelet-derived growth factor receptor-beta (PDGFR-beta), SSEA-4, CD20, MUC1, NCAM, Prostase, PAP, ELF2M, Ephrin B2, IGF-I receptor, CAIX, LMP2, gp100, bcr-abl, tyrosinase, Fucosyl GM1, sLe, GM3, TGS5, HMWMAA, o-acetyl-GD2, Folate receptor beta, TEM1/CD248, TEM7R, CLDN6, GPRC5D, CXORF61, CD97, CD179a, ALK, Polysialic acid, PLAC1, GloboH, NY-BR-1, UPK2, HAVCR1, ADRB3, PANX3, GPR20, LY6K, OR51E2, TARP, WT1, NY-ESO-1, LAGE-1a, MAGE-A1, legumain, HPV E6,E7, MAGE A1, ETV6-AML, sperm protein 17, XAGE1, Tie 2, MAD-CT-1, MAD-CT-2, Fos-related antigen 1, p53, p53 mutant, prostein, survivin, telomerase, PCTA-1/Galectin 8, MelanA/MART1, Ras mutant, hTERT, sarcoma translocation breakpoints, ML-IAP, ERG (TMPRSS2 ETS fusion gene), NA17, PAX3, Androgen receptor, Cyclin B1, MYCN, RhoC, TRP-2, CYP1B1, BORIS, SART3, PAX5, OY-TES1, LCK, AKAP-4, SSX2, RAGE-1, human telomerase reverse transcriptase, RU1, RU2, intestinal carboxyl esterase, mut hsp70-2, CD79a, CD79b, CD72, LAIR1, FCAR, LILRA2, CD300LF, CLEC12A, BST2, EMR2, LY75, GPC3, FCRL5, IGLL1, and derivates thereof comprising post-translational modifications of the polypeptide, for example, glycosylations, ubiquitination, acetylations, phosphorylations and the like, as well as other modifications known in the art, both naturally occurring and non-naturally occurring.

In one embodiment, the CAR antigen-binding domain binds to a self- or alloantigen, wherein the self- or alloantigen is preferably selected from a group of HLA haplotypes including HLA-A2, HLA-B7, HLA-DR as well as HLA associated antigens, such as Mic A, or Mic B.

In one embodiment, the CAR antigen-binding domain binds to a self-antigen associated with inflammation or an exogenously introduced antigen, wherein said exogenously introduced self-antigen is preferably selected from a group including MOG, Aquaporin 4, Gluten-tissue transglutaminase complex, adenovirus, or AAV.

The invention further relates to a pharmaceutical composition comprising a genetically modified cell as described herein and a pharmaceutically acceptable carrier. Treatment with the genetically modified cell according to the invention can in some embodiments be combined with one or more anti-cancer treatments or medicaments, preferably selected from the group of antibody therapy, vaccines, oncolytic viral therapy, chemotherapy, radiation therapy, cytokine therapy, dendritic cell therapy, gene therapy, hormone therapy, laser light therapy, immune suppression or transplantation.

The invention relates further to methods of treatment of the medical conditions described herein, comprising typically the administration of the cell population described herein.

All features described in the present specification may be employed to define any other embodiment or aspect of the invention, for example, features used to describe the cell may be used to describe the construct, and vice versa. Features used to describe the NK cells of the invention may be used to describe the mixtures of cells, or cell populations, or the methods of the invention. Similarly, features used to describe the methods of the invention may be used to describe the cells or constructs, and vice versa.

### DETAILED DESCRIPTION OF THE INVENTION

The present invention provides genetically modified CAR NK cells suitable to purify TCR-disrupted (therapeutic) T cells, preferably CAR T cells, during *ex vivo* expansion. The present invention enables dynamic TCR/CD3-elimination throughout CAR T cell expansion resulting in a sustained removal of TCR/CD3⁺ cells without negatively impacting CAR T cell functionality of the remaining cells. The following passages provide further embodiments and definitions of the invention, which may be combined with the embodiments presented above.

### Chimeric Antigen Receptors:

In various embodiments, genetically modified receptors, which redirect the cytotoxicity of immune effector cells are provided. These genetically engineered receptors are referred to herein as chimeric antigen receptors (CARs). CARs are molecules that combine antibody-based specificity for a desired antigen (e.g., CD19) with a T cell receptor-activating intracellular domain to generate a chimeric protein that exhibits a specific anti-gene cellular immune activity, e.g. against cells expressing a TCR. As used herein, the term, "chimeric," describes being composed of parts of different proteins or DNAs from different origins.

According to the present invention, a CAR comprises an extracellular antigen-binding domain, comprising an antibody or antibody fragment that binds a target antigen, a transmembrane domain, and an intracellular domain. CARs are typically described as comprising an extracellular ectodomain (antigen-binding domain) derived from an antibody and an endodomain comprising signaling modules originally derived from T cell signaling proteins.

In a preferred embodiment, the ectodomain preferably comprises variable regions from the heavy and light chains of an immunoglobulin configured as a single-chain variable fragment (scFv). The scFv is preferably attached to a hinge region that provides flexibility and transduces signals through an anchoring transmembrane moiety to an intracellular signaling domain. The transmembrane domains originate preferably from either CD8a or CD28. In the first generation of CARs the signaling domain consists of the zeta chain of the TCR complex. The term "generation" refers to the structure of the intracellular signaling domains. Second generation CARs are equipped with a single costimulatory domain originated from CD28 or 4-1 BB. Third generation CARs already include two costimulatory domains, e.g. CD28, 4-1 BB, ICOS or OX40, CD3 zeta. Fourth generation CARs additionally deliver immune stimulatory proteins and/or checkpoint regulatory proteins. The fifth CAR generation incorporates control elements for the regulation of CAR expression, which allows to induce and/or completely switch off the CAR expression or CAR expressing cells, e.g. by suicide genes. The present invention preferably relates to a third, fourth or fifth generation CAR.

The methods of the present invention comprise the administration of cells designed to express a CAR. In some embodiments, the ligand or extracellular ligand binding domain is selected so that the cell expressing the CAR is targeted to a cancer cell or tumor. Any cancer antigen can be a tumor antigen and any tumor antigen can be a caner antigen. The extracellular antigen-binding domain of a CAR is usually derived from a monoclonal antibody (mAb) or from receptors or their ligands.

CARs contemplated herein, comprise an extracellular domain (also referred to as a binding domain or antigen-binding domain) that binds to a preferred target protein, a transmembrane domain, and an intracellular domain, or intracellular signaling domain. "Target protein" and "target antigen" are used interchangeably. In one embodiment, the preferred target protein is a TCR complex component, as described herein.

"Specific binding" is to be understood as via one skilled in the art, whereby the skilled person is clearly aware of various experimental procedures that can be used to test binding and binding specificity. Methods for determining equilibrium association or equilibrium dissociation constants are known in the art. Some cross-reaction or background binding may be inevitable in many protein- protein interactions; this is not to detract from the "specificity" of the binding between CAR and epitope. By way of example, "specific binding" describes binding of an e.g. anti-CD3 epsilon antibody or antigen binding fragment thereof (or a CAR comprising the same) to CD3 epsilon at greater binding affinity than background binding. The term "directed against" is also applicable when considering the term "specificity" in understanding the interaction between antibody and epitope.

An "antigen (Ag)" refers to a compound, composition, or substance that can stimulate the production of antibodies or a T cell response in an animal. In particular embodiments, the target antigen is an epitope of a CD3 epsilon polypeptide. An "epitope" refers to the region of an antigen to which a binding agent binds. Epitopes can be formed both from contiguous amino acids or noncontiguous amino acids juxtaposed by tertiary folding of a protein. Any suitable antigen can be selected depending on the type of cancer.

### Antibodies and antibody fragments:

The CAR comprises an extracellular antigen-binding domain, comprising for example an antibody or antibody fragment that binds CD3 epsilon or a TCR alpha/beta dimer polypeptide. Antibodies or antibody fragments of the invention therefore include, but are not limited to polyclonal, monoclonal, bispecific, human, humanized or chimeric antibodies, single chain fragments (scFv), single variable fragments (ssFv), single domain antibodies (such as VHH fragments from nanobodies), Fab fragments, F(ab')2 fragments, fragments produced by a Fab expression library, anti-idiotypic antibodies and epitope-binding fragments or combinations thereof of any of the above, provided that they retain similar binding properties of the CAR described herein, preferably comprising the corresponding CDRs, or VH and VL regions as described herein. Also mini-antibodies and multivalent antibodies such as diabodies, triabodies, tetravalent antibodies and peptabodies can be used in a method of the invention. The immunoglobulin molecules of the invention can be of any class (i.e. IgG, IgE, IgM, IgD and IgA) or subclass of immunoglobulin molecules. Thus, the term antibody, as used herein, also includes antibodies and antibody fragments comprised by the CAR of the invention, either produced by the modification of whole antibodies or synthesized de novo using recombinant DNA methodologies.

As used herein, an "antibody" generally refers to a protein consisting of one or more polypeptides substantially encoded by immunoglobulin genes or fragments of immunoglobulin genes. Where the term "antibody" is used, the term "antibody fragment" may also be considered to be referred to. The recognized immunoglobulin genes include the kappa, lambda, alpha, gamma, delta, epsilon and mu constant region genes, as well as the myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD, and IgE, respectively. The basic immunoglobulin (antibody) structural unit is known to comprise a tetramer or dimer. Each tetramer is composed of two identical pairs of polypeptide chains, each pair having one "light" (L) (about 25 kD) and one "heavy" (H) chain (about 50-70 kD). The N-terminus of each chain defines a variable region of about 100 to 1 10 or more amino acids, primarily responsible for antigen recognition. The terms "variable light chain" and "variable heavy chain" refer to these variable regions of the light and heavy chains respectively. Optionally, the antibody or the immunological portion of the antibody, can be chemically conjugated to, or expressed as, a fusion protein with other proteins.

"Single-chain Fv" or "scFv" antibody fragments comprise the VH and VL domains of an antibody, wherein these domains are present in a single polypeptide chain and in either orientation {e.g., VL-VH or VH-VL). Generally, the scFv polypeptide further comprises a polypeptide linker between the VH and VL domains which enables the scFv to form the desired structure for antigen binding. In preferred embodiments, a CAR contemplated herein comprises antigen-specific binding domain that is a scFv and may be a murine, human or humanized scFv. Single chain antibodies may be cloned from the V region genes of a hybridoma specific for a desired target.

The CARs of the invention are intended to bind against mammalian, in particular human, protein targets. The use of protein names may correspond to either mouse or human versions of a protein.

Affinities of binding domain polypeptides and CAR proteins according to the present disclosure can be readily determined using conventional techniques, e.g., by competitive ELISA (enzyme-linked immunosorbent assay), or by binding association, or displacement assays using labeled ligands, or using a surface-plasmon resonance device such as the Biacore.

A variable region of an antibody refers to the variable region of the antibody light chain or the variable region of the antibody heavy chain, either alone or in combination. The variable regions of the heavy and light chain each consist of four framework regions (FR) connected by three complementarity determining regions (CDRs) also known as hypervariable regions. The CDRs in each chain are held together in close proximity by the FRs and, with the CDRs from the other chain, contribute to the formation of the antigen-binding site of antibodies.

### Additional components of the CAR

In certain embodiments, the CARs contemplated herein may comprise linker residues between the various domains, added for appropriate spacing and conformation of the molecule, for example a linker comprising an amino acid sequence that connects the VH and VL domains and provides a spacer function compatible with interaction of the two sub-binding domains so that the resulting polypeptide retains a specific binding affinity to the same target molecule as an antibody that comprises the same light and heavy chain variable regions. CARs contemplated herein, may comprise one, two, three, four, or five or more linkers. In particular embodiments, the length of a linker is about 1 to about 25 amino acids, about 5 to about 20 amino acids, or about 10 to about 20 amino acids, or any intervening length of amino acids. Illustrative examples of linkers include glycine polymers; glycine-serine polymers; glycine-alanine polymers; alanine-serine polymers; and other flexible linkers known in the art, such as the Whitlow linker. Glycine and glycine-serine polymers are relatively unstructured, and therefore may be able to serve as a neutral tether between domains of fusion proteins such as the CARs described herein. In particular embodiments, the binding domain of the CAR is followed by one or more "spacers" or "spacer polypeptides," which refers to the region that moves the antigen binding domain away from the effector cell surface to enable proper cell/cell contact, antigen binding and activation. In certain embodiments, a spacer domain is a portion of an immunoglobulin, including, but not limited to, one or more heavy chain constant regions, e.g., CH2 and CH3. The spacer domain can include the amino acid sequence of a naturally occurring immunoglobulin hinge region or an altered immunoglobulin hinge region. In one embodiment, the spacer domain comprises the CH2 and CH3 domains of IgG1 or IgG4. In one embodiment the Fc-binding domain of such a spacer/hinge region is mutated in a manner that prevents binding of the CAR to Fc-receptors expressed on macrophages and other innate immune cells.

The binding domain of the CAR may in some embodiments be followed by one or more "hinge domains," which play a role in positioning the antigen binding domain away from the effector cell surface to enable proper cell/cell contact, antigen binding and activation. A CAR may comprise one or more hinge domains between the binding domain and the transmembrane domain (TM). The hinge domain may be derived either from a natural, synthetic, semi-synthetic, or recombinant source. The hinge domain can include the amino acid sequence of a naturally occurring immunoglobulin hinge region or an altered immunoglobulin hinge region. Illustrative hinge domains suitable for use in the CARs described herein include the hinge region derived from the extracellular regions of type 1 membrane proteins such as CD8 alpha, CD4, CD28, PD1, CD 152, and CD7, which may be wild-type hinge regions from these molecules or may be altered. In another embodiment, the hinge domain comprises a PD1, CD 152, or CD8 alpha hinge region.

The "transmembrane domain" is the portion of the CAR that fuses the extracellular binding portion and intracellular signaling domain and anchors the CAR to the plasma membrane of the immune effector cell. The TM domain may be derived either from a natural, synthetic, semi-synthetic, or recombinant source. The TM domain may be derived from the alpha, beta or zeta chain of the T cell receptor, CD3s, O'Ω3ζ, CD4, CD5, CD8 alpha, CD9, CD16, CD22, CD27, CD28, CD33, CD37, CD45, CD64, CD80, CD86, CD134, CD137, CD152, CD154, and PD1 . In one embodiment, the CARs contemplated herein comprise a TM domain derived from CD8 alpha or CD28.

In particular embodiments, CARs contemplated herein comprise an intracellular signaling domain. An "intracellular signaling domain," refers to the part of a CAR that participates in transducing the message of effective anti-CD19 CAR binding to a human CD19 polypeptide into the interior of the immune effector cell to elicit effector cell function, e.g., activation, cytokine production, proliferation and cytotoxic activity, including the release of cytotoxic factors to the CAR-bound target cell, or other cellular responses elicited with antigen binding to the extracellular CAR domain. The term "effector function" refers to a specialized function of an immune effector cell. Effector function of the T cell, for example, may be cytolytic activity or help or activity including the secretion of a cytokine. Thus, the term "intracellular signaling domain" refers to the portion of a protein which transduces the effector function signal and that directs the cell to perform a specialized function. CARs contemplated herein comprise one or more co-stimulatory signaling domains to enhance the efficacy, expansion and/or memory formation of T cells expressing CAR receptors. As used herein, the term, "co-stimulatory signaling domain" refers to an intracellular signaling domain of a co-stimulatory molecule. Co-stimulatory molecules are cell surface molecules other than antigen receptors or Fc receptors that provide a second signal required for efficient activation and function of T lymphocytes upon binding to an antigen. The costimulatory molecule is a cell surface molecule other than an antigen receptor or its ligand that contributes to an efficient immune response. Costimulatory molecules include MHC class I molecules, BTLA and Toll ligand receptors, OX40, CD27, CD28, CDS, ICAM-1, LFA-1 (CD11a / CD18), ICOS (CD278) and 4-1BB (CD137). Including, but not limited to, further examples of such costimulatory molecules are CDS, ICAM-1, GITR, BAFFR, HVEM (LIGHTTR), SLAMF7, NKp80 (KLRF1), NKp44, NKp30, NKp46, CD160, CD19, CD4, CD8 alpha, CD8 beta , IL2Rbeta, IL2Rgamma, IL7Ralpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, TNFR2, TRANCE / RANKL, DNAM1 (CD 26), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG / Cbp, CD19a and ligands that specifically bind to CD83,.

A costimulatory intracellular signaling domain can be the intracellular portion of a costimulatory molecule. In one embodiment, the CAR comprises an intracellular domain, which comprises a co-stimulatory domain and a signaling (activation) domain. The CAR construct may therefore include an intracellular signaling domain (CD3 zeta) of the native T cell receptor complex and one or more co- stimulatory domains that provide a second signal to stimulate full T cell activation. Co-stimulatory domains are thought to increase CAR-T cell cytokine production and facilitate T cell replication and T cell persistence. Co-stimulatory domains have also been shown to potentially prevent CAR-T cell exhaustion, increase T cell anti-tumor activity, and enhance survival of CAR-T cells in patients. As a non-limiting example, CAR constructs with the 4-1 BB co-stimulatory domain have been associated with gradual, sustained expansion and effector function, increased persistence, and enriched central memory cells (TCM) in the T cell subset composition in preclinical studies. 4-1 BB is a member of the tumor necrosis factor (TNF) superfamily, and it is an inducible glycoprotein receptor in vivo that is primarily expressed on antigen-activated CD4 and CD8 T cells. As a non-limiting example, CD28 is member of the immunoglobulin (Ig) superfamily. It is constitutively expressed on resting and activated CD4 and CD8 T cells and plays a critical role in T cell activation by stimulating the PI3K- AKT signal transduction pathway. In one embodiment, the intracellular domain comprises both 4- 1 BB and CD28 co-stimulatory domains. Other co-stimulatory domains comprise ICOS and OX40 that can be combined with the CD3 zeta signaling (activation) domain.

In some non-limiting embodiments, an intracellular domain of a CAR may additionally comprise at least one co-stimulating signal domain. Such a co-stimulatory signal domain can result in increased activation of a T cell.

The polypeptide domains described herein may be useful in an anti-cancer CAR to mediate a desired function such as that of a signal peptide, antigen, binding domain, transmembrane domain, intracellular signal domain and/or co-stimulating domain. Potential desirable functions may comprise, but are not limited to, providing a signal peptide and/or transmembrane domain.

The cytokines described herein may relate to any mammalian cytokine corresponding to the cytokine named herein. Preferably, the cytokines relate to the human cytokines, or mouse cytokines. Cancer immunotherapy attempts to stimulate the immune system to reject and destroy tumors. Initially, immunotherapy treatments involved administration of cytokines such as "Interleukin", as described herein.

Checkpoint inhibitors, also known as immune checkpoint modulators, are designed to lessen the effectiveness of checkpoint proteins. They may have a variety of mechanisms of action, but if effective, they enable the immune system to recognize other molecules on the surface of the cancer cells. The checkpoint inhibitor of the immune response is selected from the group consisting of: PD1, PD-L1, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGFR beta.

CAR-regulating proteins can also be proteins that induce or completely switch off a CAR expression or a cell expressing a CAR. In another embodiment, the CAR-T cells or CAR-NK cells additionally comprise one or more immune suppression defeating proteins and/or an inducible suicide gene that provokes CAR-T cell or CAR-NK cell death allowing their selective destruction. In some cases, it may be desirable to provide a safety mechanism that allows selective deletion of the administered genetically modified cells, as the manipulated cells can spread and persist for years after administration. Therefore, the method of the invention in some embodiments may include the transformation of preferably the T cells, NK cells or Tregs with a recombinant suicide gene. This recombinant suicide gene can be used to preferably reduce the risk of direct toxicity and/or uncontrolled proliferation of these T cells, NK cells or Tregs after administration to a subject. Suicide genes also enable the selective deletion of transformed cells in vivo. In particular, the suicide gene has the ability to convert a non-toxic prodrug into a cytotoxic drug or to express the toxic gene expression product. In other words, "suicide gene" is preferably a nucleic acid that codes for a product, whereby the product itself or in the presence of other compounds causes cell death.

### TCR, CD3

A "T cell receptor" (TCR) is a heterodimer of the TCR alpha and TCR beta chains. The TCR complex is formed by TCR and CD3 gamma, CD3 delta, CD3 epsilon and CD3 zeta. The interruption or decreased expression of one or more of the chains T cell receptor alpha, T cell receptor beta, CD3 gamma, CD3 delta, CD3 epsilon or CD3 zeta can be used to reduce or prevent the formation of a functional T cell receptor (TCR) complex. By reducing or preventing the formation of a functional TCR complex, the T cell no longer mediates an immune response via its TCR complex. In an embodiment, a nucleic acid encoding a CAR is integrated at a site inside the cell's genome that interrupts or reduces the expression of a T cell receptor alpha chain, T cell receptor beta chain, CD3 gamma chain, CD3 delta chain, CD3 epsilon chain or CD3 zeta chain. While the reduction of one of the proteins of the TCR complex may be sufficient, it is understood that more than one component of the TCR complex can be reduced if desired. Antigen-presenting cells activate the T cell receptor (TCR) and TCR-mediated signals are transmitted by the CD3 chains zeta, delta, epsilon, and gamma across the cell membrane. All CD3 chains contain immune receptor tyrosine-based activation motifs (ITAMs) in their cytoplasmic domain.

### Polypeptides

"Peptide" "polypeptide", "polypeptide fragment" and "protein" are used interchangeably, unless specified to the contrary, and according to conventional meaning, i.e., as a sequence of amino acids. Polypeptides are not limited to a specific length, e.g., they may comprise a full length protein sequence or a fragment of a full length protein, and may include post-translational modifications of the polypeptide, for example, glycosylations, acetylations, phosphorylations and the like, as well as other modifications known in the art, both naturally occurring and non-naturally occurring.

In various embodiments, the CAR polypeptides contemplated herein comprise a signal (or leader) sequence at the N-terminal end of the protein, which co-translationally or post-translationally directs transfer of the protein. Polypeptides can be prepared using any of a variety of well-known recombinant and/or synthetic techniques. Polypeptides contemplated herein specifically encompass the CARs of the present disclosure, or sequences that have deletions from, additions to, and/or substitutions of one or more amino acid of a CAR as disclosed herein.

An "isolated peptide" or an "isolated polypeptide" and the like, as used herein, refer to in vitro isolation and/or purification of a peptide or polypeptide molecule from a cellular environment, and from association with other components of the cell, i.e., it is not significantly associated with in vivo substances. Similarly, an "isolated cell" refers to a cell that has been obtained from an in vivo tissue or organ and is substantially free of extracellular matrix.

### Fusion protein, fusion gene

"Fusion proteins" or "chimeric proteins" as used herein, comprise proteins formed by joining two or more nucleic acid segments that originally encoded separate proteins, polypeptides or protein fragments. "Fusion gene", "gene fusion" or "fused gene" are according to the invention preferably joined nucleic acid sequence segments, wherein at least one nucleic acid sequence is also an exogenous nucleic acid sequence and at least one other nucleic acid sequence is also an endogenous nucleic acid sequence. In preferred embodiments, the exogenous nucleic acid is integrated into and fused to an endogenous nucleic acid sequence, e.g. an endogenous gene. In some embodiments, the integrated exogenous nucleic acid sequence to be fused may have several combined gene structures and/or polypeptide-encoding sequences separated by one or more protein separation sites. The translation of a fusion gene results in a single or multiple polypeptides with functional properties derived from each of the original proteins or protein fragments. In a further embodiment, a fused gene may contain one or more nucleic acid sequences encoding a polypeptide, wherein the polypeptides may be present as individual polypeptides or proteins after translation, with separation being mediated by a protein separation site.

Chimera or chimeras refer to hybrid proteins made up of polypeptides with different functions or attributes. In some embodiments, fusion proteins combine whole peptides and therefore contain all functional domains of the original proteins. In certain embodiments, fusion proteins combine only parts of coding sequences and therefore only retain parts of the original functions of the genes from which they were formed. A gene fusion can also cause regulatory changes that alter when, to what extent, and where these genes are expressed. In some embodiments, the rearrangement of different active sites and/or binding domains can additionally lead to the formation of proteins with new functions and/or specificities.

### Nucleic acids

As used herein, the terms "polynucleotide" or "nucleic acid molecule" refers to messenger RNA (mRNA), RNA, genomic RNA (gRNA), plus strand RNA (RNA(+)), minus strand RNA (RNA(-)), genomic DNA (gDNA), complementary DNA (cDNA) or recombinant DNA. Polynucleotides include single and double stranded polynucleotides. Preferably, polynucleotides of the invention include polynucleotides or variants having at least about 50%, 55%, 60%, 65%, 70%, 75%, 80%, 85%, 90%, 95%, 96%, 97%, 98%, 99% or 100%) sequence identity to any of the reference sequences described herein, typically where the variant maintains at least one biological activity of the reference sequence.

In various illustrative embodiments, the present invention contemplates, in part, polynucleotides comprising expression vectors, viral vectors, and transfer plasmids, and compositions, and cells comprising the same. Polynucleotides can be prepared, manipulated and/or expressed using any of a variety of well- established techniques known and available in the art. In order to express a desired polypeptide, a nucleotide sequence encoding the polypeptide, can be inserted into appropriate vector. Examples of vectors are plasmid, autonomously replicating sequences, and transposable elements. Additional exemplary vectors include, without limitation, plasmids, phagemids, cosmids, artificial chromosomes such as yeast artificial chromosome (YAC), bacterial artificial chromosome (BAC), or PI-derived artificial chromosome (PAC), bacteriophages such as lambda phage or MI 3 phage, and animal viruses. Examples of categories of animal viruses useful as vectors include, without limitation, retrovirus (including lentivirus), adenovirus, adeno-associated virus, herpesvirus {e.g., herpes simplex virus), poxvirus, baculovirus, papillomavirus, and papovavirus {e.g., SV40). Examples of expression vectors are pClneo vectors (Promega) for expression in mammalian cells; pLenti4/V5- DEST^{™}, pLenti6/V5-DEST^{™}, and pLenti6.2/V5-GW/lacZ (Invitrogen) for lentivirus-mediated gene transfer and expression in mammalian cells. In particular embodiments, the coding sequences of the chimeric proteins disclosed herein can be ligated into such expression vectors for the expression of the chimeric protein in mammalian cells. The "control elements" or "regulatory sequences" present in an expression vector are those non-translated regions of the vector - origin of replication, selection cassettes, promoters, enhancers, translation initiation signals (Shine Dalgarno sequence or Kozak sequence) introns, a polyadenylation sequence, 5' and 3' untranslated regions - which interact with host cellular proteins to carry out transcription and translation. Such elements may vary in their strength and specificity. Depending on the vector system and host utilized, any number of suitable transcription and translation elements, including ubiquitous promoters and inducible promoters may be used.

### Vectors

In particular embodiments, a cell (e.g., an immune effector cell, such as a T cell) is transfected with a no-viral vector, a retroviral vector, e.g., a lentiviral vector, preferably a non-viral vector encoding a CAR. For example, an NK cell is transfected with a vector encoding a CAR that comprises a antibody or antigen binding fragment that binds a CD3 or TCR alpha/beta polypeptide, with a transmembrane and intracellular signaling domain, such that these transduced cells can elicit a CAR-mediated cytotoxic response.

The term "vector" is used herein to refer to a nucleic acid molecule capable transferring or transporting another nucleic acid molecule. The transferred nucleic acid is generally linked to, e.g., inserted into, the vector nucleic acid molecule. A vector may include sequences that direct autonomous replication in a cell, or may include sequences sufficient to allow integration into host cell DNA. Useful vectors include, for example, plasmids (e.g., DNA plasmids or RNA plasmids), transposons, cosmids, bacterial artificial chromosomes, and viral vectors. Useful viral vectors include, e.g., replication defective retroviruses and lentiviruses. In further embodiments of the invention, a gene editing technique, preferably CRISPR-Cas, mediated integration of the CD19 CAR encoding nucleic acid into a host genome may be employed. Appropriate vectors for CRISPR/Cas and TALEN-mediated insertion are known to a skilled person. In certain embodiments, the gene editing techniques include zinc finger nucleases (ZFNs), integrases, site specific recombinases, meganucleases, homing endonucleases, or TALENs, more preferably a CRISPR-Cas9 derived from *Streptococcus pyogenes.*

As will be evident to one of skill in the art, the term "viral vector" is widely used to refer either to a nucleic acid molecule (e.g., a transfer plasmid) that includes virus-derived nucleic acid elements that typically facilitate transfer of the nucleic acid molecule or integration into the genome of a cell or to a viral particle that mediates nucleic acid transfer. Viral particles will typically include various viral components and sometimes also host cell components in addition to nucleic acid(s).

In a preferred embodiment the invention therefore relates to a method for transfecting cells with an expression vector encoding a CAR. For example, in some embodiments, the vector comprises additional sequences, such as sequences that facilitate expression of the CAR, such a promoter, enhancer, poly-A signal, and/or one or more introns. In preferred embodiments, the CAR-coding sequence is flanked by transposon sequences, such that the presence of a transposase allows the coding sequence to integrate into the genome of the transfected cell.

In some embodiments, the genetically transformed cells are further transfected with a transposase that facilitates integration of a CAR coding sequence into the genome of the transfected cells. In some embodiments the transposase is provided as DNA expression vector. However, in preferred embodiments, the transposase is provided as an expressible RNA or a protein such that long-term expression of the transposase does not occur in the transgenic cells. For example, in some embodiments, the transposase is provided as an mRNA (e.g., an mRNA comprising a cap and poly- A tail). Any transposase system may be used in accordance with the embodiments of the present invention. However, in some embodiments, the transposase is salmonid-type Tel -like transposase (SB). For example, the transposase can be the so called "Sleeping beauty" transposase, see e.g., U.S. Patent 6,489,458, incorporated herein by reference. In some embodiments, the transposase is an engineered enzyme with increased enzymatic activity. Some specific examples of transposases include, without limitation, SB 10, SB 1 1 or SB 100X transposase (see, e.g., Mates et al, 2009, Nat Genet. 41 (6):753-61, or US9228180). For example, a method can involve electroporation of cells with an mRNA encoding an SB 10, SB 1 1 or SB 100X transposase.

### Gene Editing and transfer

A gene editing technique can be used to interfere with the endogenous expression of any expressed structure on a genome. "Endogenous" as used herein is particularly used to define a gene expression that originates from within a system such as a host, organism, tissue, or cell whereas "exogenous" particularly defines a genetic sequence introduced from externally into a cell by one or more genetic, biochemical or other methods.

Gene editing is a type of genetic engineering in which DNA is inserted, deleted or replaced in the genome of a living organism by manipulated nucleases, or "molecular Scissors". These nucleases generate site-specific double-strand breaks (DSBs) at desired sites in the genome. The induced double-strand breaks are repaired by non-homologous end compound (HEJ) or homologous recombination (HR), resulting in targeted mutations ("edits"). Five families of manipulated nucleases can be used for gene editing: (a) meganucleases, (b) zinc finger nuclei (ZFNs), (c) transcription activator-like effector-based nucleases (TALENs), (d) Mega-TALENs, and (e) the CRISPR-Cas system.

In certain embodiments, CRISPR-Cas9 is preferably used for gene editing. A "targeting sequence", "target sequence" or "target site" mainly refers to a chromosomal or extrachromosomal nucleic acid sequence that defines a part of a nucleic acid to which a binding molecule binds and/or cleaves, provided there are sufficient conditions for binding and/or cleavage. In any of these gene editing approaches inducing double-strand breaks, the target sequence comprises an identical sequence to a recognition site of the endogenous DNA sequence into which an integration is intended, preferably selected from the group consisting of a homology arm, a guide RNA target site, a restriction enzyme recognition site, a ZFN recognition site, a ZFN cleaving site, a TALEN DNA-binding site, a recombinase recognition site, an integrase site and/or a homing nuclease recognition site Techniques using each of these artificial nucleases are well known among experts. For example, when cells are edited with a genome editing complex (e.g., a TALEN, CRISPR/Cas9, ZFN, Mega-TALENs, or meganucleases) to introduce a functional gene or to knock out a gene, nucleases (e.g., Fokl or Cas9) induce a double strand break at the site of modification. Upon induction of the double strand break, a protein, such as a DNA repair protein, e.g., phosphorylated (serl778) 53BP1 (p53BPI) or gH2AC can accumulate at the site of the double strand break and is indicative of a DNA damage response.

One or more of these techniques can be used alone or in any combination to disrupt endogenous expression of a TCR-complex component in the method of the invention. When the gene editing technique is used to disrupt the expression of a TCR complex component, the gene-editing technology can particularly target one or more of the following: (i) a CD3 zeta locus, (ii) a safe harbor locus, (iii) a TRAC locus; (ii) a locus controlling TCR-beta expression; (iii) a locus controlling TCR gamma expression; (iv) a locus that controls TCR delta expression; (v) a locus that controls the expression of a CD3 chain. CD3 chains are described above. The CD3 chain can be CD3 zeta, CD3 epsilon, CD3 delta or CD3 gamma. In certain embodiments, genome loci harboring genetic information for expressing at least one ITAM domain are preferably target for gene editing.

In some embodiments, a genetically modified cell or animal comprising an inactivated genomic sequence may be termed a "knock-out" or a "conditional knock-out." Similarly, a genetically modified cell or animal comprising an integrated sequence may be termed a "knock-in" or a "conditional knock-in".

### Promoter

The term "promoter" is used for a DNA sequence that initiates and regulates transcription from the DNA downstream of it through binding of proteins. The transcribed RNA may encode a protein. Promoters are preferably located near the transcription start sites of genes, upstream of the coding DNA (towards the 5' region of the sense strand).

In some embodiments, it may be preferable to use a cell-type, cell lineage or tissue-specific expression control sequence to achieve cell-type, lineage or tissue-specific expression of a desired polynucleotide sequence (e.g. to express a particular nucleic acid encoding a peptide only in a subset of cell types, cell lines or tissues or during certain stages of development).

### Expression termination signal

"Expression termination signal" comprises any structural elements at RNA and/or DNA level that specifies an end of transcription and/or translation, and/or a polyadenylation of the heterologous nucleic acid transcripts. The transcriptional stop is usually defined by a terminator, a nucleic acid sequence that defines the end of a transcription (e.g. of a gene) and initiates the release of the newly synthesized RNA. Terminators are located downstream of the gene to be transcribed and occur directly after 3' regulatory elements, such as the poly-A signal. The term "poly-A site", "poly-A signal" or "poly-A sequence", as used herein, refers to a nucleic acid sequence that signals both, termination and polyadenylation, of the nascent RNA transcript by RNA polymerase II. Polyadenylation sequences can enhance mRNA stability by adding a poly-A tail at the 3' end of the coding sequence, thus contributing to increased translation efficiency. Efficient polyadenylation of the recombinant transcript is preferable because transcripts without a poly-A tail are unstable and degrade rapidly. A "stop codon" is also a sequence of three consecutive nucleotides (termed triplet) within the messenger RNA that signals the termination of a protein translation process at the ribosome. In aspects of the invention, the CAR transgene preferably uses endogenous expression termination signals, including a poly-A signal, a stop codon for RNA translation into protein, and a transcriptional stop signal.

### Integrated sequence

An "integrated sequence" or "integrating sequence" particularly refers to a nucleic acid construct including coding and/or non-coding elements to be inserted into the genome of the transfected cell. In some embodiments, an edited chromosomal sequence may comprise an integrated sequence. The integrated sequence may code for an endogenous protein, an exogenous or heterologous protein, a wild-type protein, a modified protein, a fusion protein or similar. In some embodiments, the integrated sequence is also a transgene. In a specific aspect of the invention, different transgenes are integrated at the different integration sites. An "integration site" or "fusion site" as used herein is the exact nucleic acid position within a genome where a double-strand break is generated and a nucleic acid construct is incorporated into the genome by homology directed repair at the position of the double-strand break. An endogenous sequence can be interrupted by integrating an exogenous sequence, so that the exogenous sequence is under the control of the endogenous promoter. In some embodiments the integrated exogenous sequence and/or the integrated exogenous sequence would be expressed together with an endogenous sequence. An integrated protein coding sequence can also be placed under the control of an endogenous promoter and/or fused in-frame with an endogenous protein coding sequence. In addition, the integrated sequence can also serve as a control element. Accordingly, the integrated sequence may be endogenous or exogenous to the cell. The exogenous sequence may be a homologue of or related to an endogenous sequence. The exogenous sequence can also be a human sequence. An animal or cell with such an integrated sequence can be called a "knock-in". In certain embodiments, a sequence can be integrated to change the expression pattern of a protein of interest. For example, a conditional knock-out system can be created. In aspects of the invention, an integrated sequence can be a nucleic acid sequence encoding a CAR, a CAR fragment, a protein separation site, a CAR regulatory protein and/or an immune stimulatory protein. In some embodiments, the nucleic acid construct integrated into the genome of the transfected cell comprise a CAR fragment and a protein separation site upstream of said CAR fragment.

### Nucleic acids and transfer methods

Methods for introducing exogenous molecules, e.g. nucleic acid sequences, into cells are well known to experts and include lipid-mediated transfer (i.e. liposomes, including neutral and cationic lipids), electroporation, direct injection, cell fusion, particle bombardment, biopolymer nanoparticles, calcium phosphate co-precipitation, DEAE-dextran-mediated transfer and viral vector-mediated transfer. "Gene transfer", "nucleic acid transfer", gene sequence transfer", "transgene transfer" are used interchangeably. In some embodiments, nucleic acid constructs are preferably transferred into cells by electroporation.

A "gene" refers to a DNA region that encodes a gene product including regions regulating the production of the gene product, regardless of whether these sequences are adjacent to coding and/or transcribed sequences. A gene comprises, but is not limited to, promoter sequences, terminators, translational regulatory sequences such as ribosome binding sites and internal ribosome entry sites, enhancers, silencers, insulators, boundary elements, replication origins, matrix attachment sites, and locus control regions.

"Gene expression" is particularly the conversion of the information encoded by a gene into a gene product. A gene product can be the direct transcription product of a gene (e.g., mRNA, tRNA, rRNA, antisense RNA, ribozyme, structural RNA or any other type of RNA) or a protein produced by translation of a mRNA. Gene products also include RNAs modified by processes such as capping, polyadenylation, methylation, and editing, as well as proteins modified by processes such as methylation, acetylation, phosphorylation, ubiquitination, ADP-ribosylation, myristoylation, and glycosylation.

### Upstream/ downstream

"Upstream" and "downstream" as used herein, are employed in the context of the 5'→3' direction of a nucleic acid strand. Upstream refers to nucleotides towards the 5' end and downstream towards the 3' end of any given nucleic acid.

### In-frame, out-of-frame

A fusion product is defined as 'in-frame' when an open reading frame remains intact in the 3'-region downstream of the nucleic acid integration site, regardless of the number of inserted or deleted amino acids at the fusion junction point. A shift in the open reading frame of a protein coding gene sequence refers to "out-of-frame".

### Sequence Variation:

Sequence variants of the claimed nucleic acids, proteins, antibodies, antibody fragments and/or CARs, for example those defined by % sequence identity, that maintain similar binding properties of the invention are also included in the scope of the invention. Such variants, which show alternative sequences, but maintain essentially the same binding properties, such as target specificity, as the specific sequences provided are known as functional analogues, or as functionally analogous.

Sequence identity relates to the percentage of identical nucleotides or amino acids when carrying out a sequence alignment. The recitation "sequence identity" as used herein refers to the extent that sequences are identical on a nucleotide-by-nucleotide basis or an amino acid-by-amino acid basis over a window of comparison. Thus, a "percentage of sequence identity" may be calculated by comparing two optimally aligned sequences over the window of comparison, determining the number of positions at which the identical nucleic acid base (e.g., A, T, C, G, I) or the identical amino acid residue (e.g., Ala, Pro, Ser, Thr, Gly, Val, Leu, Phe, Tyr, Trp, Lys, Arg, His, Asp, Glu, Asn, Gin, Cys and Met) occurs in both sequences to yield the number of matched positions, dividing the number of matched positions by the total number of positions in the window of comparison (i.e., the window size), and multiplying the result by 100 to yield the percentage of sequence identity. Included are nucleotides and polypeptides having at least about 50%, 55%, 60%, 65%, 70%, 75%, 80%, 85%, 90%, 95%, 96%, 97%, 98%, 99% or 100% sequence identity to any of the reference sequences described herein, typically where the polypeptide variant maintains at least one biological activity of the reference polypeptide.

It will be appreciated by those of ordinary skill in the art that, as a result of the degeneracy of the genetic code, there are many nucleotide sequences that encode a polypeptide as described herein. Some of these polynucleotides bear minimal homology or sequence identity to the nucleotide sequence of any native gene. Nonetheless, polynucleotides that vary due to differences in codon usage are specifically contemplated by the present invention. Deletions, substitutions and other changes in sequence that fall under the described sequence identity are also encompassed in the invention.

Protein sequence modifications, which may occur through substitutions, are also included within the scope of the invention. Substitutions as defined herein are modifications made to the amino acid sequence of the protein, whereby one or more amino acids are replaced with the same number of (different) amino acids, producing a protein which contains a different amino acid sequence than the primary protein. Substitutions may be carried out that preferably do not significantly alter the function of the protein. Like additions, substitutions may be natural or artificial. It is well known in the art that amino acid substitutions may be made without significantly altering the protein's function. This is particularly true when the modification relates to a "conservative" amino acid substitution, which is the substitution of one amino acid for another of similar properties. Such "conserved" amino acids can be natural or synthetic amino acids which because of size, charge, polarity and conformation can be substituted without significantly affecting the structure and function of the protein. Frequently, many amino acids may be substituted by conservative amino acids without deleteriously affecting the protein's function.

In general, the non-polar amino acids Gly, Ala, Val, lie and Leu; the non-polar aromatic amino acids Phe, Trp and Tyr; the neutral polar amino acids Ser, Thr, Cys, Gin, Asn and Met; the positively charged amino acids Lys, Arg and His; the negatively charged amino acids Asp and Glu, represent groups of conservative amino acids. This list is not exhaustive. For example, it is well known that Ala, Gly, Ser and sometimes Cys can substitute for each other even though they belong to different groups.

Substitution variants have at least one amino acid residue in the antibody molecule removed and a different residue inserted in its place. The sites of greatest interest for substitutional mutagenesis include the hypervariable regions, but framework alterations are also contemplated. If such substitutions result in a change in biological activity, then more substantial changes, denominated "exemplary substitutions" in the table immediately below, or as further described below in reference to amino acid classes, may be introduced and the products screened.

### Potential Amino Acid Substitutions:

| **Original residue** | **Preferred conservative substitutions** | **Examples of exemplary substitutions** |
|---|---|---|
| Ala (A) | Val | Val; Leu; Ile |
| Asg (R) | Lys | I vs; Gln; Asn |
| Asn (N) | Gln | Gln; His; Asp, Lys; Arg |
| Asp (D) | Glu | Glu; Asn |
| Cvs (C) | Ser | Ser; Ala |
| Gln (Q) | Asn | Asn, Glu |
| Glu (E) | Asp | Asp; Gln |
| Gly (G) | Ala | Ala |
| His (H) | Arg | Asn; Gln; Lys; Arg |
| Ile (I) | Leu | Leu; Val; Met; Ala; Phe; Norleucine |
| Leu (L) | Ile | Norleucine; Ile; Val; Met; Ala; Phe |
| Lys (K) | Arg | Arg; Gln; Asn |
| Met (M) | Leu | Leu; Phe; Ile |
| Phe (F) | Tvr | Leu; Val; Ile; Ala; Tvr |
| Pro (P) | Ala | Ala |
| Ser (S) | Thr | Thr |
| Thr (T) | Ser | Ser |
| Trp (W) | Tvr | Tyr; Phe |
| Tvr (Y) | Phe | Trp; Phe; Thr; Ser |
| Val (V) | Leu | Ile; Leu; Met; Phe; Ala; Norleucine |

Substantial modifications in the biological properties of the antibody are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain.

Conservative amino acid substitutions are not limited to naturally occurring amino acids, but also include synthetic amino acids. Commonly used synthetic amino acids are omega amino acids of various chain lengths and cyclohexyl alanine which are neutral non-polar analogues; citrulline and methionine sulfoxide which are neutral non-polar analogues, phenylglycine which is an aromatic neutral analog; cysteic acid which is a negatively charged analog and ornithine which is a positively charged amino acid analogue. Like the naturally occurring amino acids, this list is not exhaustive, but merely exemplary of the substitutions that are well known in the art.

### Genetically modified cells and Immune cells

The present invention contemplates, in particular embodiments, cells genetically modified to express the CARs contemplated herein, for use in the methods of the invention or for the treatment of medical conditions.

As used herein, the term "genetically engineered" or "genetically modified" refers to the addition of extra genetic material in the form of DNA or RNA into the total genetic material in a cell. Genetic modifications can be carried out selectively or not at a specific location in the genome of a cell. In an embodiment, the genetic change is site-specific. In an embodiment, the genetic modification is not site-specific. The terms, "genetically modified cells"," genetically modified immune cells", "modified cells," and, "redirected cells," are used interchangeably. As used herein, the term "gene therapy" refers to the introduction-permanently or transiently- of extra genetic material in the form of DNA or RNA into the total genetic material in a cell that restores, corrects, or modifies expression of a gene, or for the purpose of expressing a therapeutic polypeptide, e.g., a CAR. In particular embodiments, the CARs contemplated herein are introduced and expressed in immune effector cells so as to redirect their specificity to a target antigen of interest, e.g., a CD19 polypeptide.

An "immune cell" or "immune effector cell" is any cell of the immune system that has one or more effector functions (e.g., cytotoxic cell killing activity, secretion of cytokines, induction of ADCC and/or CDC). An immune effector cell can be also differentiated from iPSCs (induced pluripotent stem cells) or derived from peripheral human blood and/or human cord blood. Immune effector cells of the invention can be autologous/autogeneic ("self") or non-autologous ("non- self," e.g., allogeneic, syngeneic or xenogeneic). "Autologous", as used herein, refers to cells from the same subject, and represent a preferred embodiment of the invention. "Allogeneic," as used herein, refers to cells of the same species that differ genetically to the cell in comparison.

"Syngeneic," as used herein, refers to cells of a different subject that are genetically identical to the cell in comparison. "Xenogeneic," as used herein, refers to cells of a different species to the cell in comparison. In preferred embodiments, the cells of the invention are autologous or allogeneic. Illustrative immune effector cells used with the CARs contemplated herein include T lymphocytes. The terms "T cell" or "T lymphocyte" are art-recognized and are intended to include thymocytes, immature T lymphocytes, mature T lymphocytes, resting T lymphocytes, cytokine-induced killer cells (CIK cells) or activated T lymphocytes. Cytokine-induced killer (CIK) cells are typically CD3-and CD56-positive, non-major histocompatibility complex (MHC)- restricted, natural killer (NK)-like T lymphocytes. A T cell can be a T helper (Th; CD4+ T cell) cell, for example a T helper (Th) cell, such as a TH1, TH2, TH3, TH17, TH9 or TFH cell. The T cell can be a cytotoxic T cell (CTL; CD8+ T cell), CD4+CD8+ T cell, CD4 CD8 T cell, or a regulatory T cell (reg) or any other subset of T cells. The T cell can be a naive, effector, memory, effector storage, central storage, memory stem T cell. The T cell can be an umbilical cord blood cell. The T cell can be a peripheral lymphocyte. The T-cell may be expanded from PBMNCs. The T-cell may be autologous with respect to an individual to whom it is to be administered. The T-cell may be allogeneic with respect to an individual to whom it is to be administered. The T-cell may be partially HLA incompatible with an individual to whom it is to be administered.

"Immortalized", as used herein, refers to immortalized cells as a population of cells that would not normally reproduce for an unlimited number of cell cycles. Due to a mutation, immortal cells escape from a normal cellular senescence and instead keep undergoing the cell proliferation. The mutation can occur spontaneously or induced by UV light, genetic manipulation, or entry by a virus, toxin or bacteria into the cell. The cells can therefore be cultivated in vitro over a longer period of time for experimental, therapeutic or medical purposes. Said immortalized immune cells include K13, αβ T cells, γδ T cells, NK cells, NKT cells, NK-92 and YT cells, stem cells, or stem cell-derived cells including cells of the aforementioned immune system, preferably NKT cells, NK-92 cells, YT cells, and NK cells. An immortalized T cell line may retains its lytic function.

Other illustrative populations of T cells suitable for use in particular embodiments include naive T cells and memory T cells and stem cell-like memory cells TSCM).

For example, when reintroduced back to patients after autologous cell transplantation, the T cells modified with the CAR of the invention as described herein may recognize and kill tumor cells. CIK cells may have enhanced cytotoxic activity compared to other T cells, and therefore represent a preferred embodiment of an immune cell of the present invention.

As would be understood by the skilled person, other cells may also be used as immune effector cells with the CARs as described herein. In particular, immune effector cells also include NK cells, NKT cells, neutrophils, and macrophages. Immune effector cells also include progenitors of effector cells wherein such progenitor cells can be induced to differentiate into an immune effector cells in vivo or in vitro. Progenitors can be iPSCs that become immune effector cells under defined culture conditions. The present invention provides methods for making the immune effector cells which express the CAR contemplated herein. In one embodiment, the method comprises transfecting or transducing immune effector cells isolated from an individual such that the immune effector cells express one or more CAR as described herein. In certain embodiments, the immune effector cells are isolated from an individual and genetically modified without further manipulation in vitro. Such cells can then be directly re-administered into the individual. In further embodiments, the immune effector cells are first activated and stimulated to proliferate in vitro prior to being genetically modified to express a CAR. In this regard, the immune effector cells may be cultured before and/or after being genetically modified (i.e., transduced or transfected to express a CAR contemplated herein).

Natural killer cells, also known as NK cells or large granular lymphocytes (LGL), are a type of cytotoxic lymphocyte critical to the innate immune system that belong to the rapidly expanding family of known innate lymphoid cells (ILC) and represent 5-20% of all circulating lymphocytes in humans. The role of NK cells is analogous to that of cytotoxic T cells in the vertebrate adaptive immune response. NK cells provide rapid responses to virus-infected cell and other intracellular pathogens acting at around 3 days after infection, and respond to tumor formation. Typically, immune cells detect the major histocompatibility complex (MHC) presented on infected cell surfaces, triggering cytokine release, causing the death of the infected cell by lysis or apoptosis. NK cells are unique, however, as they have the ability to recognize and kill stressed cells in the absence of antibodies and MHC, allowing for a much faster immune reaction. NK cells can be identified by the presence of CD56 and the absence of CD3 (CD56+, CD3-).

In particular embodiments, prior to in vitro manipulation or genetic modification of the immune effector cells described herein, the source of cells is obtained from a subject. In particular embodiments, the CAR-modified immune effector cells comprise T cells. T cells can be obtained from a number of sources including, but not limited to, peripheral blood mononuclear cells, bone marrow, lymph nodes tissue, cord blood, thymus issue, tissue from a site of infection, ascites, pleural effusion, spleen tissue, and tumors. In certain embodiments, T cells can be obtained from a unit of blood collected from a subject using any number of techniques known to the skilled person, such as sedimentation, e.g., FICOLL^{™} separation, antibody-conjugated bead-based methods such as MACS^{™} separation (Miltenyi). In one embodiment, cells from the circulating blood of an individual are obtained by apheresis. The apheresis product typically contains lymphocytes, including T cells, monocytes, granulocyte, B cells, other nucleated white blood cells, red blood cells, and platelets. In one embodiment, the cells collected by apheresis may be washed to remove the plasma fraction and to place the cells in an appropriate buffer or media for subsequent processing. The cells can be washed with PBS or with another suitable solution that lacks calcium, magnesium, and most, if not all other, divalent cations. As would be appreciated by those of ordinary skill in the art, a washing step may be accomplished by methods known to those in the art, such as by using a semiautomated flow through centrifuge. For example, the Cobe 2991 cell processor, the Baxter CytoMate, or the like. After washing, the cells may be resuspended in a variety of biocompatible buffers or other saline solution with or without buffer. In certain embodiments, the undesirable components of the apheresis sample may be removed in the cell directly resuspended culture media.

In certain embodiments, T cells are isolated from peripheral blood mononuclear cells (PBMCs) by lysing the red blood cells and depleting the monocytes, for example, by centrifugation through a PERCOLL^{™} gradient. A specific subpopulation of T cells can be further isolated by positive or negative selection techniques. One method for use herein is cell sorting and/or selection via negative magnetic immunoadherence or flow cytometry that uses a cocktail of monoclonal antibodies directed to cell surface markers present on the cells negatively selected.

PBMC may be directly genetically modified to express CARs using methods contemplated herein. In certain embodiments, after isolation of PBMC, T lymphocytes are further isolated and in certain embodiments, both cytotoxic and helper T lymphocytes can be sorted into naive, memory, and effector T cell subpopulations either before or after genetic modification and/or expansion. CD8+ cells can be obtained by using standard methods. In some embodiments, CD8+ cells are further sorted into naive, central memory, and effector cells by identifying cell surface antigens that are associated with each of those types of CD8+ cells.

In some embodiments, the immune cell of the present invention, for example the T cells described herein, can be obtained from inducible pluripotent stem cells (iPSCs) using methods known to a skilled person. Accepted approaches for producing CAR-T cells rely on the genetic modification and expansion of mature circulating T cells. Such processes utilize autologous T cells and reduce risk of graft-versus- host (GvHD) disease from allogeneic T cells through endogenous TCR expression as well as rejection through MHC incompatibility. As an alternative, direct in vitro differentiation of engineered T cells from pluripotent stem cells, such as inducible pluripotent stem cells, provides an essentially unlimited source of cells that can be genetically modified to express the CAR of the present invention. In some embodiments, a so-called master iPSC line can be maintained, which represents a renewable source for consistently and repeatedly manufacturing homogeneous cell products. In some embodiments, the transformation of a master iPSC cell line with the CAR encoding nucleic acid is contemplated, prior to expansion and differentiation to the desired immune cell, preferably T cell or NK cell. T lymphocytes can for example be generated from iPSCs, such that iPSCs could be modified with the CAR or the CD3 zeta-deficient CAR fragment encoding nucleic acids and subsequently expanded and differentiated to T cells for administration to the patient. Differentiation to the appropriate immune cell, such a T cell, could also be conducted from the iPSCs before transformation with the CAR or the CD3 zeta-deficient CAR encoding nucleic acids and expansion prior to administration. All possible combinations of iPSC expansion, genetic modification and expansion to provide suitable numbers of cells for administration are contemplated in the invention.

The immune effector cells, such as T cells, NK cells, CIK cells or Tregs, can be genetically modified following isolation using known methods, or the immune effector cells can be activated and expanded (or differentiated in the case of progenitors) in vitro prior to being genetically modified. In a particular embodiment, the immune effector cells, such as T cells, NK cells, CIK cells or Tregs, are genetically modified with the CD3 zeta-deficient CAR fragment contemplated herein (e.g., transfected with a nucleic acid and pre-complexed Cas9 enzyme or transduced with a viral vector comprising a nucleic acid encoding a CD3 zeta-deficient CAR fragment) and then are activated and expanded in vitro. In various embodiments, T cells can be activated and expanded before or after genetic modification to express a CAR or a CD3 zeta-deficient CAR fragment, using methods as described, for example, in U.S. Patents 6,352,694; 6,534,055; 6,905,680; 6,692,964; 5,858,358; 6,887,466; 6,905,681; 7,144,575; 7,067,318; 7,172,869; 7,232,566; 7,175,843; 5,883,223; 6,905,874; 6,797,514; 6,867,041; and U.S. Patent Application Publication No. 20060121005.

In a further embodiment, a mixture of, e.g., one, two, three, four, five or more, different expression vectors can be used in genetically modifying a donor population of immune effector cells wherein each vector encodes a different chimeric antigen receptor protein as contemplated herein. The resulting modified immune effector cells forms a mixed population of modified cells, with a proportion of the modified cells expressing more than one different CAR protein.

In one embodiment, the invention provides a method of storing genetically modified murine, human or humanized CAR protein expressing immune effector cells which target a CD19 protein, comprising cryopreserving the immune effector cells such that the cells remain viable upon thawing. A fraction of the immune effector cells expressing the CAR proteins can be cryopreserved by methods known in the art to provide a permanent source of such cells for the future treatment of patients afflicted with the B cell, T cell, NK cell, dendritic cell (DC), cytotoxic induced killer cell (CIK) related condition. When needed, the cryopreserved transformed immune effector cells can be thawed, grown and expanded for more such cells.

### Cell culture and bioreactors

As described herein, the invention relates to methods of culturing or co-culturing cells, for example NK and/or T cells. Methods for isolating culturing and co-culturing cells are established and require no undue effort from a skilled person to implement. A review of established processes is provided in Iyer et al (Front Med (Lausanne). 2018; 5: 150).

Traditionally, immune cell expansion in research environments has relied on the use of animal or human sera. Manufacturing of cell therapy products is however now possible with serum-free (SF) or xenogeneic-free (XF) media and reagents. Animal component free (ACF) media that are chemically defined (CD) meet the stricter definition of not containing any human or non-human animal components, and being mixed in defined or known quantities. With the advent of both eukaryotic and prokaryotic recombinant expression systems, the availability of ACF media has become more commonplace in recent times. Another aspect to media manufacturing, particularly for later clinical stage and commercial use, is that the reagents comply with good manufacturing practices (GMP) standards. Adherence to GMP standards ensures a high degree of quality in production as well as control.

Magnetic-Activated Cell Sorting (MACS) using antibodies conjugated to magnetic beads is currently the most common method for isolating a population of cells, as it is quicker and has greater scalability than FACS. Miltenyi Biotec and ThermoFisher have developed closed system magnetic cell selection technologies (CliniMACS Prodigy and CTS Dynamag, respectively) along with corresponding reagents that enable isolation of specific cell populations.

Conventional bioreactor platform technologies developed for large scale mammalian cell expansion are effective in the present invention, and are capable of delivering nutrients and oxygen to an expanding cell population. These systems often utilize mechanisms to enhance oxygen delivery, such as stirring, rocking, or perfusion. For most cell therapies, cell expansion is required to reach the clinical dose required. There are several platforms available that enable expansion of the cells. For immunotherapies, the most commonly used systems are static gas permeable culture bags, G-Rex bioreactors, wave-mixed bioreactors, and the Miltenyi Prodigy system. Several companies offer gas permeable bags (GPB) including VueLife (Saint Gobain), Charter Medical and OriGen. GPB are designed to enable a high rate of gas transfer to the cells while maintaining low water permeability enabling culturing of cells in a closed system, unlikely conventional tissue culture flasks, and several groups have demonstrated growth of T cells in these bags.

G-Rex bioreactors (Wilson Wolf) are tissue culture vessels that have a gas-permeable membrane at the base of the vessel and allow for expansion of cells to a high density due to efficient gas exchange at the cell-liquid interface. The G-Rex bioreactors allow expansion of cells from a low seeding density and don't necessary require a media exchange as the design has a large enough reservoir of media to enable culture for 8-10 days. As they mimic the format and handling of tissue culture flasks, they can represent a simple and cost-effective way to initially transfer a process from a preclinical to early clinical setting.

In some embodiments, closed-system bioreactors are employed. For example, the G-Rex cell culture platform is based on a gas-permeable membrane technology that provides advantages over other systems. Closed-system bioreactors, such as gas-permeable membrane-based bioreactors, provide a physiologic environment and avoid the risk and cost associated with more complex systems. The result is a more robust, interacting cell population established through unlimited oxygen and nutrients that are available on demand. By removing the need to actively deliver oxygen, these bioreactors can hold larger medium volumes (more nutrients) which allows the cells to reach a maximum density without complexity or need for media exchange. This platform approach is scaled to meet the needs of research through commercial production with a direct, linear correlation between small and large devices. In the G-Rex platform, examples of cell expansion (9-14 day duration) include; CAR-T cells, which have atypical harvest density of 20-30 × 106/cm2 (or 2-3 × 10⁹ cells in a 100 cm² device); NK cells, which have a typical harvest density of 20-30 × 10⁶/cm² (or 2-3 × 10⁹ cells in a 100 cm² device) and numerous other cell types that proliferate without the need for intervention or complex processes normally associated with large scale culture.

Wave-mixed bioreactors use the rocking wave motion to enable efficient mixing and oxygen transfer within the reactor in a low shear side-to-side motion and have become a common platform for commercial scale-up of T-cell immunotherapies. There are several wave-mixed bioreactors on the market including the SmartRocker (Finesse), Allegro (Pall), Biostat RM (Sartorius), and Xuri Cell Expansion System (GE Healthcare), among others. Wave-mixed bioreactors are functionally closed-system bioreactors, and reduce the amount of manual labor in the cell expansion phase of the culture due to their built-in automation features.

The CliniMACS Prodigy (Miltenyi Biotec) is an all-in-one solution that not only enables immune cell expansion, but several other steps within the immunotherapy workflow including cell selection through magnetic separation, cell washing, and final product formulation in a closed system. The CliniMACS Prodigy supports a culture volume of up to 400 mL (300 mL working volume). There are also several alternative technologies that can potentially be used for culture expansion of immunotherapies. Stirred tank reactors (STRs) are commonly used in monoclonal antibody production and have a scale ranging from hundreds of milliliters (e.g., spinner flasks) to thousands of liters. Culturing T cells in an STR could be useful in an allogenic setting, where scale-up will be more important (vs. scale out with patient-specific therapies).

### Compositions and Formulations

The compositions contemplated herein may comprise one or more polypeptides, polynucleotides, vectors comprising same, genetically modified immune effector cells, etc., as contemplated herein. Compositions include, but are not limited to, pharmaceutical compositions.

A "pharmaceutical composition" refers to a composition formulated in pharmaceutically-acceptable or physiologically- acceptable solutions for administration to a cell or an animal, either alone, or in combination with one or more other modalities of therapy. It will also be understood that, if desired, the compositions of the invention may be administered in combination with other agents as well, such as, e.g., cytokines, growth factors, hormones, small molecules, chemotherapeutics, prodrugs, drugs, antibodies, or other various pharmaceutically-active agents. There is virtually no limit to other components that may also be included in the compositions, provided that the additional agents do not adversely affect the ability of the composition to deliver the intended therapy.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein "pharmaceutically acceptable carrier, diluent or excipient" includes without limitation any adjuvant, carrier, excipient, glidant, sweetening agent, diluent, preservative, dye/colorant, flavor enhancer, surfactant, wetting agent, dispersing agent, suspending agent, stabilizer, isotonic agent, solvent, surfactant, or emulsifier which has been approved by the United States Food and Drug Administration as being acceptable for use in humans or domestic animals.

Plasmids, nucleic acids, nucleic acids complexed with a Cas enzyme, AAV vectors, lentiviral vectors and/or other compositions, agents, drugs, biologics (proteins) can be incorporated into pharmaceutical compositions, e.g., pharmaceutically acceptable carriers or excipients. Such pharmaceutical compositions are particularly useful for administration and delivery to a subject in vivo or ex vivo.

In particular embodiments, compositions of the present invention comprise an amount of CAR-expressing immune effector cells contemplated herein. As used herein, the term "amount" refers to "an amount effective" or "an effective amount" of a genetically modified therapeutic cell, e.g., T cell, NK cell, CIK cell, or Treg cell, to achieve a beneficial or desired prophylactic or therapeutic result, including clinical results.

A "prophylactically effective amount" refers to an amount of a genetically modified therapeutic cell effective to achieve the desired prophylactic result. Typically, but not necessarily, since a prophylactic dose is used in subjects prior to or at an earlier stage of disease, the prophylactically effective amount is less than the therapeutically effective amount. The term prophylactic does not necessarily refer to a complete prohibition or prevention of a particular medical disorder. The tern prophylactic also refers to the reduction of risk of a certain medical disorder occurring or worsening in its symptoms.

A "therapeutically effective amount" of a genetically modified immune cell may vary according to factors such as the disease state, age, sex, and weight of the individual, and the ability of the stem and progenitor cells to elicit a desired response in the individual. A therapeutically effective amount is also one in which any toxic or detrimental effects of the virus or transduced or transfected therapeutic cells are outweighed by the therapeutically beneficial effects. The term "therapeutically effective amount" includes an amount that is effective to "treat" a subject {e.g., a patient). When a therapeutic amount is indicated, the precise amount of the compositions of the present invention to be administered can be determined by a physician with consideration of individual differences in age, weight, tumor size, extent of infection or metastasis, and condition of the patient (subject). It can generally be stated that a pharmaceutical composition comprising the T cells or CIK cells or NK cells or Treg cells described herein may be administered at a dosage of 10² to 10¹⁰ cells/kg body weight, preferably 10⁵ to 10⁷ cells/kg body weight, including all integer values within those ranges. The number of cells will depend upon the ultimate use for which the composition is intended as will the type of cells included therein. For uses provided herein, the cells are generally in a volume of a liter or less, can be 500 mL or less, even 250 mL or 100 mL or less. Hence the density of the desired cells is typically greater than 10⁶ cells/ml and generally is greater than 10⁷ cells/mL, generally 10⁸ cells/mL or greater. The clinically relevant number of immune cells can be apportioned into multiple infusions that cumulatively equal or exceed 10⁵, 10⁶, 10⁷, 10⁸, 10⁹, 10¹⁰, 10¹¹, or 10¹² cells. In some aspects of the present invention, particularly since all the infused cells will be redirected to a particular target antigen, lower numbers of cells may be administered. CAR expressing cell compositions may be administered multiple times at dosages within these ranges. The cells may be allogeneic, syngeneic, xenogeneic, or autologous to the patient undergoing therapy.

Generally, compositions comprising the cells activated and expanded as described herein may be utilized in the treatment and prevention of diseases that arise in individuals who are immunocompromised. In particular, compositions comprising the CAR-modified T cells, CAR-modified NK cells, CAR-modified CIK cells or CAR-modified Treg cells contemplated herein are used in the treatment of cancer, preferably solid or liquid, more preferably solid malignancies, more preferably rectal cancer, lung cancer, breast cancer, liver cancer, pancreatic cancer, stomach cancer, and ovarian cancer. The CAR-modified T cells, CAR-modified NK cells, CAR-modified CIK cells or CAR-modified Treg cells of the present invention may be administered either alone, or as a pharmaceutical composition in combination with carriers, diluents, excipients, and/or with other components such as interleukins or other immune response stimulating cytokines e.g. IL-15 and/or checkpoint inhibitory molecules, e.g. PD1 polypeptide or a PD1-antibody, or cell populations.

In particular embodiments, pharmaceutical compositions contemplated herein comprise an amount of genetically modified T cells, in combination with one or more pharmaceutically or physiologically acceptable carriers, diluents or excipients. In particular embodiments, pharmaceutical compositions contemplated herein comprise an amount of genetically modified NK cells, genetically modified CIK cells or genetically modified Treg cells in combination with one or more pharmaceutically or physiologically acceptable carriers, diluents or excipients.

Pharmaceutical compositions of the present invention comprising a CAR-expressing immune effector cell population, such as T cells or NK cells or CIK cells or Treg cells, may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. Compositions of the present invention are preferably formulated for parenteral administration, e.g., intravascular (intravenous or intraarterial), intraperitoneal or intramuscular administration.

The liquid pharmaceutical compositions, whether they are solutions, suspensions or other like form, may include one or more of the following: sterile diluents such as water for injection, saline solution, preferably physiological saline, Ringer's solution, isotonic sodium chloride, fixed oils such as synthetic mono or diglycerides which may serve as the solvent or suspending medium, polyethylene glycols, glycerin, propylene glycol or other solvents; antibacterial agents such as benzyl alcohol or methyl paraben; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in ampoules, disposable syringes or multiple dose vials made of glass or plastic. An injectable pharmaceutical composition is preferably sterile.

In a particular embodiment, compositions contemplated herein comprise an effective amount of CAR-expressing immune effector cells, alone or in combination with one or more therapeutic agents. Thus, the CAR-expressing immune effector cell compositions may be administered alone or in combination with other known cancer treatments, such as radiation therapy, chemotherapy, transplantation, immunotherapy, hormone therapy, photodynamic therapy, etc. The compositions may also be administered in combination with antibiotics. Such therapeutic agents may be accepted in the art as a standard treatment for a particular disease state as described herein, such as a particular cancer. Exemplary therapeutic agents contemplated include cytokines, growth factors, steroids, NSAIDs, DMARDs, anti-inflammatories, chemotherapeutics, radiotherapeutics, therapeutic antibodies, or other active and ancillary agents.

A combined immunotherapy encompasses simultaneous treatment, co-treatment or joint treatment, and includes the administration of genetically modified immune cells expressing a nucleic acid construct encoding a CAR or a CD3 zeta-deficient CAR fragment combined with immunotherapies, such as checkpoint inhibitors and/or immune stimulatory cytokines, whereby treatment may occur within minutes of each other, in the same hour, on the same day, in the same week or in the same month as one another. A combination medicament, comprising one or more of said genetically modified immune cells with another immunotherapeutic, may also be used in order to co-administer the various components in a single administration or dosage. A "medicament" particularly refers to a drug including an immune-therapeutic cell or genetically modified cell used to cure, treat, or prevent a disease.

As used herein, the term "tumor microenvironment" relates to the cellular environment in which any given tumor exists, including the tumor stroma, surrounding blood vessels, immune cells, fibroblasts, other cells, signaling molecules, and the ECM.

### Therapeutic Methods

The genetically modified immune effector cells contemplated herein provide improved methods of adoptive immunotherapy for use in the treatment of medical disorders, such as those associated with the presence of pathogenic cells expressing a tumor marker, that include, but are not limited to, immunoregulatory conditions and/or hematological and/or solid malignancies.

"Cancer", as used herein, is a disease characterized by the uncontrolled growth of abnormal cells. Cancer refers to any type of cancerous growth or carcinogenic process, metastatic tissue or malignant transformed cells, tissues or organs, regardless of histopathological type or invasive stage. Cancer cells can spread locally or to other parts of the body via the bloodstream and lymphatic system. Cancer cells spreading to other parts to the body are termed "metastatic cells" or "metastatic tumor cells". The terms "tumor" and "cancer", used herein, are utilized interchangeably, e.g. both terms include solid and liquid, e.g. general or circulating tumors, premalignant and malignant cancers and tumors. Examples of liquid cancers include, but not limited to, acute lymphocytic leukemia (ALL), chronic lymphocytic leukemia (CLL), acute myeloid Leukemia (acute myelogenous leukemia (AML), chronic bone marrow cancer, chronic myelogenous leukemia (CML), Hodgkin lymphoma, non-Hodgkin lymphoma, and myeloma. Examples of solid tumors are liver, lung, breast, lymphatic system, digestive organs (e.g. colon), urogenital organs (e.g. kidney, urothelial cells), prostate and throat, malignant organ systems including tumors such as sarcomas, adenocarcinomas and cancer. Examples for breast cancer that can be treated with the are ductal carcinoma in situ (DCIS), lobular carcinoma in situ (LCIS), invasive ductal carcinoma (IDC), invasive ductal carcinoma including tubular, medullary, mucinous, papillary, and cribriform carcinomas, invasive lobular carcinoma (ILC), inflammatory breast cancer, male breast cancer, Paget's Disease of the nipple, phyllodes tumors of the breast, recurrent and/or metastatic breast cancer. Adenocarcinoma includes most malignant tumors such as colon cancer, rectal cancer, renal cell cancer, liver cancer, non-small cell lung cancer, small intestine cancer and esophageal cancer. In certain forms the cancer is a melanoma, e.g. an advanced stage melanoma. Metastatic lesions of the cancer can also be treated or prevented with the methods and compositions of the invention. Examples of other types of cancer that can be treated are bone cancer, pancreatic cancer, skin cancer, head and neck cancer, skin or intraocular melanoma, uterine cancer, ovarian cancer, rectal cancer, anal cancer, stomach cancer, testicular cancer, faropius duct cancer, Endometrial cancer, cervical cancer, vaginal cancer, vulvar cancer, Hodgkin's disease, non-Hodgkin's lymphoma, esophageal cancer, small intestine cancer, endocrine cancer, thyroid cancer, parathyroid cancer, adrenal cancer, soft tissue sarcoma, urethral cancer, penile cancer, acute myeloid leukemia, chronic myelocytic leukemia, acute lymphoblastic leukemia, chronic or acute leukemia including chronic lymphatic leukemia, solid tumor in childhood, lymphatic lymphoma, bladder cancer, kidney or ureter cancer, renal pelvis cancer, neoplasm of the central nervous system (CNS) primary CNS lymphoma, tumor angiogenesis, spinal axis tumor, brainstem glioma, pituitary adenoma, Kaposi's sarcoma, epidermoid carcinoma, squamous cell carcinoma, asbestos-induced T cell lymphoma Including a combination of environmental cancer and cancer including things Treatment of metastatic cancer, e.g. metastatic cancer that expresses PD-L1 (Iwai et al. (2005) Int. Immunol. 17: 133-144) can be performed with the inhibitory molecules described in this invention.

"Cancer-associated antigen" or "tumor antigen" is expressed interchangeably on the surface of cancer cells, either completely or as a fragment (e.g. MHC / peptide), and the drug targets cancer cells preferentially. Molecules (usually proteins, carbohydrates or lipids) that are useful for. Tumor antigen refers to an antigen that is commonly found in a specific hyperproliferative disease. In one aspect, the antigen of hyperproliferative disease of the present invention is a primary or metastatic melanoma, thymoma, lymphoma, sarcoma, lung cancer, liver cancer, non-Hodgkin's lymphoma, Hodgkin's lymphoma, Leukemia, uterine cancer, cervical cancer, bladder cancer, derived from cancers such as kidney and breast cancer, prostate cancer, ovarian cancer, adenocarcinoma such as pancreatic cancer and the like. In certain embodiments, the tumor antigen is a marker expressed on both normal and cancer cells, such as a marker of cell lineage, like CD19 on B cells.

In certain embodiments, the tumor antigen is overexpressed in cancer cells compared to normal cells, e.g. 1-fold overexpression, 2-fold overexpression, 3-fold overexpression or more overexpression compared to normal cells. Molecules of the cell surface. In certain forms, a tumor antigen is a cell surface molecule that is inadequately synthesised in cancer cells, e.g. a molecule that contains deletions, additions or mutations compared to a molecule expressed in normal cells. In certain embodiments, the tumor antigen is expressed exclusively, completely or as a fragment (e.g. MHC / peptide) on the cell surface of cancer cells and is not synthesised or expressed on the surface of normal cells. In certain embodiments, a CAR of the invention comprises a CAR containing an antigen binding domain (e.g. an antibody or antibody fragment) which binds to an MHC-presenting peptide. Normally, peptides derived from endogenous proteins fill the pockets of major histocompatibility complex (MHC) class I molecules and are recognized by the T cell receptor (TCR) on CD8 + T lymphocytes. Class I MHC complexes are constitutively expressed by all nucleated cells. In cancer, virus- and/or tumor-specific peptides / MHC complexes represent a unique class of cell surface targets for immunotherapy. TCR-like antibodies have been described which target peptides of virus or tumor antigens associated with human leukocyte antigen (HLA) - A1 or HLA-A2 (see e.g. Sastry et al., J Virol. 2011 85 (5) : 1935-1942; Sergeeva et al, Blood, 2011 117 (16): 4262-4272; Verma et al., J Immunol 2010 184 (4): 2156-2165; Willemsen et al., Gene Ther 2001 8 (21 ): 1601-1608; Dao et al., Sci Transl Med 2013 5 (176): 176ra33; Tassev et al., Cancer Gene Ther 2012 19 (2): 84-100). For example, TCR-like antibodies can be identified by searching a library such as a human scFv phage display library.

In particular embodiments, compositions comprising CAR-modified T cells contemplated herein are used in the treatment of cancer, including but not limited to solid or liquid/hematological malignancies, such as, for example, rectal cancer, lung cancer, breast cancer, liver cancer, pancreatic cancer, stomach cancer, ovarian cancer, and metastatic tumor cells positive for CD19, or hematological malignancies such as, leukemia and lymphoma or myeoloproliferative disorders/neoplasms with or without a leukemic tumor cell dissemination, preferably solid tumors and multiple myeloma.

As used herein "treatment" or "treating," includes any beneficial or desirable effect on the symptoms or pathology of a disease or pathological condition, and may include even minimal reductions in one or more measurable markers of the disease or condition being treated. Treatment can involve optionally either the reduction or amelioration of symptoms of the disease or condition, or the delaying of the progression of the disease or condition. "Treatment" does not necessarily indicate complete eradication or cure of the disease or condition, or associated symptoms thereof.

As used herein, "prevent," and similar words such as "prevented," "preventing" or "prophylactic" etc., indicate an approach for preventing, inhibiting, or reducing the likelihood of the occurrence or recurrence of, a disease or condition. It also refers to delaying the onset or recurrence of a disease or condition or delaying the occurrence or recurrence of the symptoms of a disease or condition. As used herein, "prevention" and similar words also includes reducing the intensity, effect, symptoms and/or burden of a disease or condition prior to onset or recurrence of the disease or condition.

In one embodiment, a method of treating a cancer related condition in a subject in need thereof comprises administering an effective amount, e.g., therapeutically effective amount of a composition comprising genetically modified immune effector cells contemplated herein. The quantity and frequency of administration will be determined by such factors as the condition of the patient, and the type and severity of the patient's disease, although appropriate dosages may be determined by clinical trials.

The administration of the compositions contemplated herein may be carried out in any convenient manner, including by aerosol inhalation, injection, ingestion, transfusion, implantation or transplantation. In a preferred embodiment, compositions are administered parenterally. The phrases "parenteral administration" and "administered parenterally" as used herein refers to modes of administration other than enteral and topical administration, usually by injection, and includes, without limitation, intravascular, intravenous, intramuscular, intraarterial, intrathecal, intracapsular, intraorbital, intratumoral, intracardiac, intradermal, intraperitoneal, transtracheal, subcutaneous, subcuticular, intraarticular, subcapsular, subarachnoid, intraspinal and intrasternal injection and infusion. In one embodiment, the compositions contemplated herein are administered to a subject by direct injection into a tumor, lymph node, or site of infection.

### SEQUENCES

### Preferred amino acid sequences of the invention:

| **SEQ ID NO** | **Description** | **Sequence** |
|---|---|---|
| 1 | AAVS1 left Homology Arm (HA) | ACCGTTTTTCTGGACAACCCCAAAGTACCCCGTCTCCCTGGCTTTAG CCACCTCTCCATCCTCTTGCTTTCTTTGCCTGGACACCCCGTTCTCCT GTGGATTCGGGTCACCTCTCACTCCTTTCATTTGGGCAGCTCCCCTA CCCCCCTTACCTCTCTAGTCTGTGCTAGCTCTTCCAGCCCCCTGTCA TGGCATCTTCCAGGGGTCCGAGAGCTCAGCTAGTCTTCTTCCTCCAA CCCGGGCCCCTATGTCCACTTCAGGACAGCATGTTTGCTGCCTCCA GGGATCCTGTGTCCCCGAGCTGGGACCACCTTATATTCCCAGGGCC GGTTAATGTGGCTCTGGTTCTGGGTACTTTTATCTGTCCCCTCCACC CCACAGTGGGGCCACTAGGGACAG |
| 2 | CMV promotor / enhancer | CATTGATTATTGAGTAGTTATTAATAGTAATCAATTACGGggtcattagttcat agcccatatatggagttccgcgttacataacttacggtaaatggcccgcctggctgaccgcccaacga cccccgcccattgacgtcaataatgacgtatgttcccatagtaacgccaatagggactttccattgacgt caatgggtggagtatttacggtaaactgcccacttggcagtacatcaagtgtatcatatgccaagtacg ccccctattgacgtcaatgacggtaaatggcccgcctggcattatgcccagtacatgaccttatgggac tttcctacttggcagtacatctacgtattagtcatcgctattaccatggtgatgcggttttggcagtacatca atgggcgtggatagcggtttgactcacggggatttccaagtctccaccccattgacgtcaatgggagttt gttttggcaccaaaatcaacgggactttccaaaatgtcgtaacaactccgccccattgacgcaaatgg gcggtaggcgtgtacggtgggaggtctatataagcagagctggtttagtgaaccgtcagatccgctag cgctaccggtcgccacc |
| 3 | Membrane Export Signal (CD8a derived) | ATGGCTCTTCCTGTGACTGCCCTTCTGCTGCCCCTGGCTCTCCTGCT CCATGCTGCCCGGCCA |
| 4 | myc Tag | gagcaaaaacttatctctgaagaggacctc |
| 5 | muBMA13 single chain variable fragment | GAAGTACAGCTGCAGCAAAGTGGTCCCGAATTGGTGAAACCCGGTG CATCCGTGAAAATGAGTTGCAAAGCATCTGGCTACAAATTTACCTCCT ATGTCATGCATTGGGTGAAGCAAAAGCCCGGACAAGGCTTGGAGTG GATCGGCTATATCAACCCATATAACGACGTAACGAAATACAATGAAAA ATTTAAAGGCAAGGCGACTCTGACCTCAGACAAATCTAGCTCTACTG CTTACATGGAGCTCTCCAGTCTGACGAGCGAAGACAGCGCCGTTCAT TACTGTGCACGAGGTTCTTATTATGATTACGATGGTTTTGTCTATTGG GGACAAGGAACGCTTGTCACTGTATCTGCTGGAGGTGGTGGATCAG GAGGTGGAGGTTCCGGAGGTGGAGGAAGTGGCGGTGGAGGTAGCC AAATTGTGCTGACACAGAGCCCGGCAATCATGAGCGCATCTCCCGG AGAAAAGGTCACCATGACATGTTCTGCCACCTCTTCTGTCTCTTACAT GCACTGGTATCAGCAGAAATCAGGCACAAGTCCTAAGAGATGGATAT ACGACACAAGCAAACTGGCGAGCGGAGTGCCTGCTCGCTTCTCCGG CAGTGGAAGCGGGACTAGCTATAGCCTTACCATCAGCAGCATGGAG GCCGAAGACGCCGCAACCTACTATTGTCAACAGTGGTCCAGTAACCC TCTGACTTTTGGAGCCGGAACAAAGCTGGAACTCAAG |
| 6 | OKT3 single chain variable fragment | CAAGTGCAGCTCCAACAGTCTGGCGCTGAGCTTGCCAGACCAGGCG CCTCAGTGAAAATGAGTTGCAAGGCCAGCGGTTACACCTTCACCCGC TATACAATGCACTGGGTGAAGCAAAGACCGGGCCAGGGCCTCGAAT GGATCGGCTACATAAATCCATCCAGGGGGTACACCAATTACAACCAG AAATTCAAAGATAAAGCAACCCTCACTACCGACAAGAGTTCAAGCAC GGCTTACATGCAACTTTCCAGTCTGACGTCAGAGGACTCCGCTGTTT ACTACTGCGCACGCTACTATGATGATCATTACTCACTGGATTATTGGG GACAGGGAACTACGCTTACCGTGTCCAGCGGAGGTGGTGGATCAGG AGGTGGAGGTTCCGGAGGTGGAGGAAGTGGCGGTGGAGGTAGCGA CATCGTACTGACTCAATCACCCGCCATTATGAGTGCATCACCAGGCG AGAAAGTTACCATGACTTGTTCAGCCTCATCATCTGTTAGCTACATGA ATTGGTATCAACAGAAGTCTGGGACCTCCCCAAAGAGATGGATATAT GATACCTCCAAACTTGCCTCTGGGGTGCCTGCACACTTTAGAGGAAG CGGCAGCGGAACCAGCTACTCTCTGACCATCAGTGGAATGGAAGCT GAGGACGCAGCCACCTACTACTGCCAGCAGTGGAGCTCTAATCCATT TACATTCGGCTCTGGCACCAAGCTTGAAATAAACCGC |
| 7 | CD28 domain | ATAGAAGTAATGTATCCCCCTCCCTACTTGGACAACGAGAAATCTAAC GGCACAATCATACACGTTAAGGGCAAGCATCTGTGTCCCTCCCCTCT TTTCCCCGGACCGTCTAAGCCATTTTGGGTCCTCGTGGTGGTCGGG GGTGTGCTGGCCTGCTACAGCTTGCTGGTCACAGTGGCCTTCATCAT CTTCTGGGTGCGCTCCAAGAGGAGCCGGCTGCTTCACAGTGATTAC ATGAACATGACCCCCAGGAGGCCAGGACCCACCAGGAAGCACTACC AGCCCTACGCTCCCCCGCGGGACTTTGCTGCTTACCGCAGC |
| 8 | CD8a domain | TTCGTGCCAGTTTTCTTACCCGCGAAGCCGACCACAACACCAGCACC GCGCCCACCAACACCAGCTCCCACAATAGCCTCGCAGCCGCTCTCA CTGCGCCCGGAGGCTTGTAGGCCCGCTGCCGGTGGAGCAGTTCATA CGCGAGGACTTGATTTCGCCTGTGACATATATATTTGGGCACCTCTC GCCGGAACCTGTGGGGTCTTGCTGCTGTCTCTCGTTATAACGCTTTA TTGC |
| 9 | 4-1BB domain | AAACGGGGCAGAAAGAAACTCCTGTATATATTCAAACAACCATTTATG AGACCAGTACAAACTACTCAAGAGGAAGATGGCTGTAGCTGCCGATT TCCAGaagaagaagaaggAGGATGTGAACTG |
| 10 | CD3-zeta domain | AGGGTCAAATTTTCTAGATCTGCAGATGCGCCGGCCTATCAAcaaggcc agaaccagctcTATAACGAGCTCAATCTAGGACGAAGAGAGGAGTACGAT GTTTTGGACAAGAGACGTGGCCGGGACCCTGAGATGGGGGGAAAG CCGAGAAGGAAGAACCCTCAGGAAGGCCTGTACAATGAACTGCAGA AAGATAAGATGGCGGAGGCCTACAGTGAGATTGGGATGAAAGGCGA GCGCCGGAGGGGCAAGGGGCACGATGGCCTTTACCAGGGTCTCAG TACAGCCACCAAGGACACCTACGACGCCCTTCACATGCAGGCCCTG CCCCCTCGCTAA |
| 11 | adapter sequence for cloning | AGATCTCGAGTCTAGctcgagggcgcgccCcGCTGATCAGCCTCGA |
| 12 | polyadenylation | CTGTGCCTTCTAGTTGCCAGCCATCTGTTGTTTGCCCCTCCCCCGTG CCTTCCTTGACCCTGGAAGGTGCCACTCCCACTGTCCTTTCCTAATA |
| | signal (bovine Growth Hormone (bGH) derived) | AAATGAGGAAATTGCATCGCATTGTCTGAGTAGGTGTCATTCTATTCT GGGGGGTGGGGTGGGGCAGGACAGCAAGGGGGAGGATTGGGAAG ACAATAGCAGGCATGCTGGGGATGCGGTGGGCTCTATGG |
| 13 | AAVS1 right Homology Arm (HA) | GATTGGTGACAGAAAAGCCCCATCCTTAGGCCTCCTCCTTCCTAGTC TCCTGATATTGGGTCTAACCCCCACCTCCTGTTAGGCAGATTCCTTAT CTGGTGACACACCCCCATTTCCTGGAGCCATCTCTCTCCTTGCCAGA ACCTCTAAGGTTTGCTTACGATGGAGCCAGAGAGGATCCTGGGAGG GAGAGCTTGGCAgggggtgggagggaagggggggaTGCGTGACCTGCCCGG TTCTCAGTGGCCACCCTGCGCTACCCTCTCCCAGAACCTGAGCTGCT CTGACGCGGCCGTCTGGTGCGTTTCACTGATCCTGGTGCTGCAGCT TCCTTACACTTCCCAAGAGGAGAAgcagtttggaaaaacaaaatcagaataagttgg tcctgagttct |
| 14 | CAR1 full | ACCGTTTTTCTGGACAACCCCAAAGTACCCCGTCTCCCTGGCTTTAG CCACCTCTCCATCCTCTTGCTTTCTTTGCCTGGACACCCCGTTCTCCT GTGGATTCGGGTCACCTCTCACTCCTTTCATTTGGGCAGCTCCCCTA CCCCCCTTACCTCTCTAGTCTGTGCTAGCTCTTCCAGCCCCCTGTCA TGGCATCTTCCAGGGGTCCGAGAGCTCAGCTAGTCTTCTTCCTCCAA CCCGGGCCCCTATGTCCACTTCAGGACAGCATGTTTGCTGCCTCCA GGGATCCTGTGTCCCCGAGCTGGGACCACCTTATATTCCCAGGGCC GGTTAATGTGGCTCTGGTTCTGGGTACTTTTATCTGTCCCCTCCACC CCACAGTGGGGCCACTAGGGACAGCATTGATTATTGAGTAGTTATTA ATAGTAATCAATTACGGggtcattagttcatagcccatatatggagttccgcgttacataactt acggtaaatggcccgcctggctgaccgcccaacgacccccgcccattgacgtcaataatgacgtatg ttcccatagtaacgccaatagggactttccattgacgtcaatgggtggagtatttacggtaaactgccca cttggcagtacatcaagtgtatcatatgccaagtacgccccctattgacgtcaatgacggtaaatggcc cgcctggcattatgcccagtacatgaccttatgggactttcctacttggcagtacatctacgtattagtcat cgctattaccatggtgatgcggttttggcagtacatcaatgggcgtggatagcggtttgactcacgggga tttccaagtctccaccccattgacgtcaatgggagtttgttttggcaccaaaatcaacgggactttccaaa atgtcgtaacaactccgccccattgacgcaaatgggcggtaggcgtgtacggtgggaggtctatataa gcagagctggtttagtgaaccgtcagatccgctagcgctaccggtcgccaccATGGCTCTTCC TGTGACTGCCCTTCTGCTGCCCCTGGCTCTCCTGCTCCATGCTGCCC GGCCAgagcaaaaacttatctctgaagaggacctcGAAGTACAGCTGCAGCAAAG TGGTCCCGAATTGGTGAAACCCGGTGCATCCGTGAAAATGAGTTGCA AAGCATCTGGCTACAAATTTACCTCCTATGTCATGCATTGGGTGAAG CAAAAGCCCGGACAAGGCTTGGAGTGGATCGGCTATATCAACCCATA TAACGACGTAACGAAATACAATGAAAAATTTAAAGGCAAGGCGACTC TGACCTCAGACAAATCTAGCTCTACTGCTTACATGGAGCTCTCCAGT CTGACGAGCGAAGACAGCGCCGTTCATTACTGTGCACGAGGTTCTTA TTATGATTACGATGGTTTTGTCTATTGGGGACAAGGAACGCTTGTCAC TGTATCTGCTGGAGGTGGTGGATCAGGAGGTGGAGGTTCCGGAGGT GGAGGAAGTGGCGGTGGAGGTAGCCAAATTGTGCTGACACAGAGCC CGGCAATCATGAGCGCATCTCCCGGAGAAAAGGTCACCATGACATG TTCTGCCACCTCTTCTGTCTCTTACATGCACTGGTATCAGCAGAAATC AGGCACAAGTCCTAAGAGATGGATATACGACACAAGCAAACTGGCGA GCGGAGTGCCTGCTCGCTTCTCCGGCAGTGGAAGCGGGACTAGCTA TAGCCTTACCATCAGCAGCATGGAGGCCGAAGACGCCGCAACCTAC TATTGTCAACAGTGGTCCAGTAACCCTCTGACTTTTGGAGCCGGAAC AAAGCTGGAACTCAAGATAGAAGTAATGTATCCCCCTCCCTACTTGG ACAACGAGAAATCTAACGGCACAATCATACACGTTAAGGGCAAGCAT CTGTGTCCCTCCCCTCTTTTCCCCGGACCGTCTAAGCCATTTTGGGT CCTCGTGGTGGTCGGGGGTGTGCTGGCCTGCTACAGCTTGCTGGTC ACAGTGGCCTTCATCATCTTCTGGGTGCGCTCCAAGAGGAGCCGGC TGCTTCACAGTGATTACATGAACATGACCCCCAGGAGGCCAGGACC CACCAGGAAGCACTACCAGCCCTACGCTCCCCCGCGGGACTTTGCT GCTTACCGCAGCAGGGTCAAATTTTCTAGATCTGCAGATGCGCCGGC CTATCAAcaaggccagaaccagctcTATAACGAGCTCAATCTAGGACGAAGA GAGGAGTACGATGTTTTGGACAAGAGACGTGGCCGGGACCCTGAGA TGGGGGGAAAGCCGAGAAGGAAGAACCCTCAGGAAGGCCTGTACAA |
| | | TGAACTGCAGAAAGATAAGATGGCGGAGGCCTACAGTGAGATTGGG ATGAAAGGCGAGCGCCGGAGGGGCAAGGGGCACGATGGCCTTTAC CAGGGTCTCAGTACAGCCACCAAGGACACCTACGACGCCCTTCACA TGCAGGCCCTGCCCCCTCGCTAAAGATCTCGAGTCTAGctcgagggcgcg ccCcGCTGATCAGCCTCGACTGTGCCTTCTAGTTGCCAGCCATCTGTT GTTTGCCCCTCCCCCGTGCCTTCCTTGACCCTGGAAGGTGCCACTC CCACTGTCCTTTCCTAATAAAATGAGGAAATTGCATCGCATTGTCTGA GTAGGTGTCATTCTATTCTGGGGGGTGGGGTGGGGCAGGACAGCAA GGGGGAGGATTGGGAAGACAATAGCAGGCATGCTGGGGATGCGGT GGGCTCTATGGGATTGGTGACAGAAAAGCCCCATCCTTAGGCCTCCT CCTTCCTAGTCTCCTGATATTGGGTCTAACCCCCACCTCCTGTTAGG CAGATTCCTTATCTGGTGACACACCCCCATTTCCTGGAGCCATCTCT CTCCTTGCCAGAACCTCTAAGGTTTGCTTACGATGGAGCCAGAGAGG ATCCTGGGAGGGAGAGCTTGGCAgggggtgggagggaagggggggaTGCGT GACCTGCCCGGTTCTCAGTGGCCACCCTGCGCTACCCTCTCCCAGA ACCTGAGCTGCTCTGACGCGGCCGTCTGGTGCGTTTCACTGATCCT GGTGCTGCAGCTTCCTTACACTTCCCAAGAGGAGAAgcagtttggaaaaac aaaatcagaataagttggtcctgagttct |
| 15 | CAR2 full | ACCGTTTTTCTGGACAACCCCAAAGTACCCCGTCTCCCTGGCTTTAG CCACCTCTCCATCCTCTTGCTTTCTTTGCCTGGACACCCCGTTCTCCT GTGGATTCGGGTCACCTCTCACTCCTTTCATTTGGGCAGCTCCCCTA CCCCCCTTACCTCTCTAGTCTGTGCTAGCTCTTCCAGCCCCCTGTCA TGGCATCTTCCAGGGGTCCGAGAGCTCAGCTAGTCTTCTTCCTCCAA CCCGGGCCCCTATGTCCACTTCAGGACAGCATGTTTGCTGCCTCCA GGGATCCTGTGTCCCCGAGCTGGGACCACCTTATATTCCCAGGGCC GGTTAATGTGGCTCTGGTTCTGGGTACTTTTATCTGTCCCCTCCACC CCACAGTGGGGCCACTAGGGACAGCATTGATTATTGAGTAGTTATTA ATAGTAATCAATTACGGggtcattagttcatagcccatatatggagttccgcgttacataactt acggtaaatggcccgcctggctgaccgcccaacgacccccgcccattgacgtcaataatgacgtatg ttcccatagtaacgccaatagggactttccattgacgtcaatgggtggagtatttacggtaaactgccca cttggcagtacatcaagtgtatcatatgccaagtacgccccctattgacgtcaatgacggtaaatggcc cgcctggcattatgcccagtacatgaccttatgggactttcctacttggcagtacatctacgtattagtcat cgctattaccatggtgatgcggttttggcagtacatcaatgggcgtggatagcggtttgactcacgggga tttccaagtctccaccccattgacgtcaatgggagtttgttttggcaccaaaatcaacgggactttccaaa atgtcgtaacaactccgccccattgacgcaaatgggcggtaggcgtgtacggtgggaggtctatataa gcagagctggtttagtgaaccgtcagatccgctagcgctaccggtcgccaccATGGCTCTTCC TGTGACTGCCCTTCTGCTGCCCCTGGCTCTCCTGCTCCATGCTGCCC GGCCAgagcaaaaacttatctctgaagaggacctcCAAGTGCAGCTCCAACAGTCT GGCGCTGAGCTTGCCAGACCAGGCGCCTCAGTGAAAATGAGTTGCA AGGCCAGCGGTTACACCTTCACCCGCTATACAATGCACTGGGTGAA GCAAAGACCGGGCCAGGGCCTCGAATGGATCGGCTACATAAATCCA TCCAGGGGGTACACCAATTACAACCAGAAATTCAAAGATAAAGCAAC CCTCACTACCGACAAGAGTTCAAGCACGGCTTACATGCAACTTTCCA GTCTGACGTCAGAGGACTCCGCTGTTTACTACTGCGCACGCTACTAT GATGATCATTACTCACTGGATTATTGGGGACAGGGAACTACGCTTAC CGTGTCCAGCGGAGGTGGTGGATCAGGAGGTGGAGGTTCCGGAGG TGGAGGAAGTGGCGGTGGAGGTAGCGACATCGTACTGACTCAATCA CCCGCCATTATGAGTGCATCACCAGGCGAGAAAGTTACCATGACTTG TTCAGCCTCATCATCTGTTAGCTACATGAATTGGTATCAACAGAAGTC TGGGACCTCCCCAAAGAGATGGATATATGATACCTCCAAACTTGCCT CTGGGGTGCCTGCACACTTTAGAGGAAGCGGCAGCGGAACCAGCTA CTCTCTGACCATCAGTGGAATGGAAGCTGAGGACGCAGCCACCTAC TACTGCCAGCAGTGGAGCTCTAATCCATTTACATTCGGCTCTGGCAC |
| | | CAAGCTTGAAATAAACCGCATAGAAGTAATGTATCCCCCTCCCTACTT GGACAACGAGAAATCTAACGGCACAATCATACACGTTAAGGGCAAGC ATCTGTGTCCCTCCCCTCTTTTCCCCGGACCGTCTAAGCCATTTTGG GTCCTCGTGGTGGTCGGGGGTGTGCTGGCCTGCTACAGCTTGCTGG TCACAGTGGCCTTCATCATCTTCTGGGTGCGCTCCAAGAGGAGCCG GCTGCTTCACAGTGATTACATGAACATGACCCCCAGGAGGCCAGGA CCCACCAGGAAGCACTACCAGCCCTACGCTCCCCCGCGGGACTTTG CTGCTTACCGCAGCAGGGTCAAATTTTCTAGATCTGCAGATGCGCCG GCCTATCAAcaaggccagaaccagctcTATAACGAGCTCAATCTAGGACGAA GAGAGGAGTACGATGTTTTGGACAAGAGACGTGGCCGGGACCCTGA GATGGGGGGAAAGCCGAGAAGGAAGAACCCTCAGGAAGGCCTGTA CAATGAACTGCAGAAAGATAAGATGGCGGAGGCCTACAGTGAGATT GGGATGAAAGGCGAGCGCCGGAGGGGCAAGGGGCACGATGGCCTT TACCAGGGTCTCAGTACAGCCACCAAGGACACCTACGACGCCCTTC ACATGCAGGCCCTGCCCCCTCGCTAAAGATCTCGAGTCTAGctcgaggg cgcgccCcGCTGATCAGCCTCGACTGTGCCTTCTAGTTGCCAGCCATCT GTTGTTTGCCCCTCCCCCGTGCCTTCCTTGACCCTGGAAGGTGCCAC TCCCACTGTCCTTTCCTAATAAAATGAGGAAATTGCATCGCATTGTCT GAGTAGGTGTCATTCTATTCTGGGGGGTGGGGTGGGGCAGGACAGC AAGGGGGAGGATTGGGAAGACAATAGCAGGCATGCTGGGGATGCG GTGGGCTCTATGGGATTGGTGACAGAAAAGCCCCATCCTTAGGCCT CCTCCTTCCTAGTCTCCTGATATTGGGTCTAACCCCCACCTCCTGTTA GGCAGATTCCTTATCTGGTGACACACCCCCATTTCCTGGAGCCATCT CTCTCCTTGCCAGAACCTCTAAGGTTTGCTTACGATGGAGCCAGAGA GGATCCTGGGAGGGAGAGCTTGGCAgggggtgggagggaagggggggaTGC GTGACCTGCCCGGTTCTCAGTGGCCACCCTGCGCTACCCTCTCCCA GAACCTGAGCTGCTCTGACGCGGCCGTCTGGTGCGTTTCACTGATC CTGGTGCTGCAGCTTCCTTACACTTCCCAAGAGGAGAAgcagtttggaaaa acaaaatcagaataagttggtcctgagttct |
| 16 | CAR3 full | ACCGTTTTTCTGGACAACCCCAAAGTACCCCGTCTCCCTGGCTTTAG CCACCTCTCCATCCTCTTGCTTTCTTTGCCTGGACACCCCGTTCTCCT GTGGATTCGGGTCACCTCTCACTCCTTTCATTTGGGCAGCTCCCCTA CCCCCCTTACCTCTCTAGTCTGTGCTAGCTCTTCCAGCCCCCTGTCA TGGCATCTTCCAGGGGTCCGAGAGCTCAGCTAGTCTTCTTCCTCCAA CCCGGGCCCCTATGTCCACTTCAGGACAGCATGTTTGCTGCCTCCA GGGATCCTGTGTCCCCGAGCTGGGACCACCTTATATTCCCAGGGCC GGTTAATGTGGCTCTGGTTCTGGGTACTTTTATCTGTCCCCTCCACC CCACAGTGGGGCCACTAGGGACAGCATTGATTATTGAGTAGTTATTA ATAGTAATCAATTACGGggtcattagttcatagcccatatatggagttccgcgttacataactt acggtaaatggcccgcctggctgaccgcccaacgacccccgcccattgacgtcaataatgacgtatg ttcccatagtaacgccaatagggactttccattgacgtcaatgggtggagtatttacggtaaactgccca cttggcagtacatcaagtgtatcatatgccaagtacgccccctattgacgtcaatgacggtaaatggcc cgcctggcattatgcccagtacatgaccttatgggactttcctacttggcagtacatctacgtattagtcat cgctattaccatggtgatgcggttttggcagtacatcaatgggcgtggatagcggtttgactcacgggga tttccaagtctccaccccattgacgtcaatgggagtttgttttggcaccaaaatcaacgggactttccaaa atgtcgtaacaactccgccccattgacgcaaatgggcggtaggcgtgtacggtgggaggtctatataa gcagagctggtttagtgaaccgtcagatccgctagcgctaccggtcgccaccATGGCTCTTCC TGTGACTGCCCTTCTGCTGCCCCTGGCTCTCCTGCTCCATGCTGCCC GGCCAgagcaaaaacttatctctgaagaggacctcGAAGTACAGCTGCAGCAAAG TGGTCCCGAATTGGTGAAACCCGGTGCATCCGTGAAAATGAGTTGCA AAGCATCTGGCTACAAATTTACCTCCTATGTCATGCATTGGGTGAAG CAAAAGCCCGGACAAGGCTTGGAGTGGATCGGCTATATCAACCCATA TAACGACGTAACGAAATACAATGAAAAATTTAAAGGCAAGGCGACTC TGACCTCAGACAAATCTAGCTCTACTGCTTACATGGAGCTCTCCAGT CTGACGAGCGAAGACAGCGCCGTTCATTACTGTGCACGAGGTTCTTA TTATGATTACGATGGTTTTGTCTATTGGGGACAAGGAACGCTTGTCAC TGTATCTGCTGGAGGTGGTGGATCAGGAGGTGGAGGTTCCGGAGGT GGAGGAAGTGGCGGTGGAGGTAGCCAAATTGTGCTGACACAGAGCC CGGCAATCATGAGCGCATCTCCCGGAGAAAAGGTCACCATGACATG TTCTGCCACCTCTTCTGTCTCTTACATGCACTGGTATCAGCAGAAATC AGGCACAAGTCCTAAGAGATGGATATACGACACAAGCAAACTGGCGA |
| | | GCGGAGTGCCTGCTCGCTTCTCCGGCAGTGGAAGCGGGACTAGCTA TAGCCTTACCATCAGCAGCATGGAGGCCGAAGACGCCGCAACCTAC TATTGTCAACAGTGGTCCAGTAACCCTCTGACTTTTGGAGCCGGAAC AAAGCTGGAACTCAAGTTCGTGCCAGTTTTCTTACCCGCGAAGCCGA CCACAACACCAGCACCGCGCCCACCAACACCAGCTCCCACAATAGC CTCGCAGCCGCTCTCACTGCGCCCGGAGGCTTGTAGGCCCGCTGCC GGTGGAGCAGTTCATACGCGAGGACTTGATTTCGCCTGTGACATATA TATTTGGGCACCTCTCGCCGGAACCTGTGGGGTCTTGCTGCTGTCTC TCGTTATAACGCTTTATTGCAAACGGGGCAGAAAGAAACTCCTGTATA TATTCAAACAACCATTTATGAGACCAGTACAAACTACTCAAGAGGAAG ATGGCTGTAGCTGCCGATTTCCAGaagaagaagaaggAGGATGTGAACT GAGGGTCAAATTTTCTAGATCTGCAGATGCGCCGGCCTATCAAcaagg ccagaaccagctcTATAACGAGCTCAATCTAGGACGAAGAGAGGAGTACG ATGTTTTGGACAAGAGACGTGGCCGGGACCCTGAGATGGGGGGAAA GCCGAGAAGGAAGAACCCTCAGGAAGGCCTGTACAATGAACTGCAG AAAGATAAGATGGCGGAGGCCTACAGTGAGATTGGGATGAAAGGCG AGCGCCGGAGGGGCAAGGGGCACGATGGCCTTTACCAGGGTCTCA GTACAGCCACCAAGGACACCTACGACGCCCTTCACATGCAGGCCCT GCCCCCTCGCTAAAGATCTCGAGTCTAGctcgagggcgcgccCcGCTGATC AGCCTCGACTGTGCCTTCTAGTTGCCAGCCATCTGTTGTTTGCCCCT CCCCCGTGCCTTCCTTGACCCTGGAAGGTGCCACTCCCACTGTCCTT TCCTAATAAAATGAGGAAATTGCATCGCATTGTCTGAGTAGGTGTCAT TCTATTCTGGGGGGTGGGGTGGGGCAGGACAGCAAGGGGGAGGAT TGGGAAGACAATAGCAGGCATGCTGGGGATGCGGTGGGCTCTATGG GATTGGTGACAGAAAAGCCCCATCCTTAGGCCTCCTCCTTCCTAGTC TCCTGATATTGGGTCTAACCCCCACCTCCTGTTAGGCAGATTCCTTAT CTGGTGACACACCCCCATTTCCTGGAGCCATCTCTCTCCTTGCCAGA ACCTCTAAGGTTTGCTTACGATGGAGCCAGAGAGGATCCTGGGAGG GAGAGCTTGGCAgggggtgggagggaagggggggaTGCGTGACCTGCCCGG TTCTCAGTGGCCACCCTGCGCTACCCTCTCCCAGAACCTGAGCTGCT CTGACGCGGCCGTCTGGTGCGTTTCACTGATCCTGGTGCTGCAGCT TCCTTACACTTCCCAAGAGGAGAAgcagtttggaaaaacaaaatcagaataagttgg tcctgagttct |
| 17 | CAR4 full | ACCGTTTTTCTGGACAACCCCAAAGTACCCCGTCTCCCTGGCTTTAG CCACCTCTCCATCCTCTTGCTTTCTTTGCCTGGACACCCCGTTCTCCT GTGGATTCGGGTCACCTCTCACTCCTTTCATTTGGGCAGCTCCCCTA CCCCCCTTACCTCTCTAGTCTGTGCTAGCTCTTCCAGCCCCCTGTCA TGGCATCTTCCAGGGGTCCGAGAGCTCAGCTAGTCTTCTTCCTCCAA CCCGGGCCCCTATGTCCACTTCAGGACAGCATGTTTGCTGCCTCCA GGGATCCTGTGTCCCCGAGCTGGGACCACCTTATATTCCCAGGGCC GGTTAATGTGGCTCTGGTTCTGGGTACTTTTATCTGTCCCCTCCACC CCACAGTGGGGCCACTAGGGACAGCATTGATTATTGAGTAGTTATTA ATAGTAATCAATTACGGggtcattagttcatagcccatatatggagttccgcgttacataactt acggtaaatggcccgcctggctgaccgcccaacgacccccgcccattgacgtcaataatgacgtatg ttcccatagtaacgccaatagggactttccattgacgtcaatgggtggagtatttacggtaaactgccca cttggcagtacatcaagtgtatcatatgccaagtacgccccctattgacgtcaatgacggtaaatggcc cgcctggcattatgcccagtacatgaccttatgggactttcctacttggcagtacatctacgtattagtcat cgctattaccatggtgatgcggttttggcagtacatcaatgggcgtggatagcggtttgactcacgggga tttccaagtctccaccccattgacgtcaatgggagtttgttttggcaccaaaatcaacgggactttccaaa atgtcgtaacaactccgccccattgacgcaaatgggcggtaggcgtgtacggtgggaggtctatataa gcagagctggtttagtgaaccgtcagatccgctagcgctaccggtcgccaccATGGCTCTTCC TGTGACTGCCCTTCTGCTGCCCCTGGCTCTCCTGCTCCATGCTGCCC GGCCAgagcaaaaacttatctctgaagaggacctcCAAGTGCAGCTCCAACAGTCT GGCGCTGAGCTTGCCAGACCAGGCGCCTCAGTGAAAATGAGTTGCA AGGCCAGCGGTTACACCTTCACCCGCTATACAATGCACTGGGTGAA GCAAAGACCGGGCCAGGGCCTCGAATGGATCGGCTACATAAATCCA TCCAGGGGGTACACCAATTACAACCAGAAATTCAAAGATAAAGCAAC CCTCACTACCGACAAGAGTTCAAGCACGGCTTACATGCAACTTTCCA GTCTGACGTCAGAGGACTCCGCTGTTTACTACTGCGCACGCTACTAT GATGATCATTACTCACTGGATTATTGGGGACAGGGAACTACGCTTAC CGTGTCCAGCGGAGGTGGTGGATCAGGAGGTGGAGGTTCCGGAGG |
| | | TGGAGGAAGTGGCGGTGGAGGTAGCGACATCGTACTGACTCAATCA CCCGCCATTATGAGTGCATCACCAGGCGAGAAAGTTACCATGACTTG TTCAGCCTCATCATCTGTTAGCTACATGAATTGGTATCAACAGAAGTC TGGGACCTCCCCAAAGAGATGGATATATGATACCTCCAAACTTGCCT CTGGGGTGCCTGCACACTTTAGAGGAAGCGGCAGCGGAACCAGCTA CTCTCTGACCATCAGTGGAATGGAAGCTGAGGACGCAGCCACCTAC TACTGCCAGCAGTGGAGCTCTAATCCATTTACATTCGGCTCTGGCAC CAAGCTTGAAATAAACCGCTTCGTGCCAGTTTTCTTACCCGCGAAGC CGACCACAACACCAGCACCGCGCCCACCAACACCAGCTCCCACAAT AGCCTCGCAGCCGCTCTCACTGCGCCCGGAGGCTTGTAGGCCCGCT GCCGGTGGAGCAGTTCATACGCGAGGACTTGATTTCGCCTGTGACA TATATATTTGGGCACCTCTCGCCGGAACCTGTGGGGTCTTGCTGCTG TCTCTCGTTATAACGCTTTATTGCAAACGGGGCAGAAAGAAACTCCT GTATATATTCAAACAACCATTTATGAGACCAGTACAAACTACTCAAGA GGAAGATGGCTGTAGCTGCCGATTTCCAGaagaagaagaaggAGGATGT GAACTGAGGGTCAAATTTTCTAGATCTGCAGATGCGCCGGCCTATCA AcaaggccagaaccagctcTATAACGAGCTCAATCTAGGACGAAGAGAGGA GTACGATGTTTTGGACAAGAGACGTGGCCGGGACCCTGAGATGGGG GGAAAGCCGAGAAGGAAGAACCCTCAGGAAGGCCTGTACAATGAAC TGCAGAAAGATAAGATGGCGGAGGCCTACAGTGAGATTGGGATGAA AGGCGAGCGCCGGAGGGGCAAGGGGCACGATGGCCTTTACCAGGG TCTCAGTACAGCCACCAAGGACACCTACGACGCCCTTCACATGCAG GCCCTGCCCCCTCGCTAAAGATCTCGAGTCTAGctcgagggcgcgccCcG CTGATCAGCCTCGACTGTGCCTTCTAGTTGCCAGCCATCTGTTGTTT GCCCCTCCCCCGTGCCTTCCTTGACCCTGGAAGGTGCCACTCCCAC TGTCCTTTCCTAATAAAATGAGGAAATTGCATCGCATTGTCTGAGTAG GTGTCATTCTATTCTGGGGGGTGGGGTGGGGCAGGACAGCAAGGG GGAGGATTGGGAAGACAATAGCAGGCATGCTGGGGATGCGGTGGG CTCTATGGGATTGGTGACAGAAAAGCCCCATCCTTAGGCCTCCTCCT TCCTAGTCTCCTGATATTGGGTCTAACCCCCACCTCCTGTTAGGCAG ATTCCTTATCTGGTGACACACCCCCATTTCCTGGAGCCATCTCTCTCC TTGCCAGAACCTCTAAGGTTTGCTTACGATGGAGCCAGAGAGGATCC TGGGAGGGAGAGCTTGGCAgggggtgggagggaagggggggaTGCGTGACC TGCCCGGTTCTCAGTGGCCACCCTGCGCTACCCTCTCCCAGAACCT GAGCTGCTCTGACGCGGCCGTCTGGTGCGTTTCACTGATCCTGGTG CTGCAGCTTCCTTACACTTCCCAAGAGGAGAAgcagtttggaaaaacaaaatc agaataagttggtcctgagttct |
| 18 | sgRNA Sequence for cutting into human TRAC locus | GGGGCCACTAGGGACAGGAT |
| | | |
| 19 | Membrane Export Signal (CD8a derived) - amino acid | MALPVTALLLPLALLLHAARP |
| 20 | myc Tag - amino acid | EQKLISEEDL |
| 21 | muBMA13 single chain variable fragment - amino acid | EVQLQQSGPELVKPGASVKMSCKASGYKFTSYVMHWVKQKPGQGLE WIGYINPYNDVTKYNEKFKGKATLTSDKSSSTAYMELSSLTSEDSAVHY CARGSYYDYDGFVYWGQGTLVTVSAGGGGSGGGGSGGGGSGGGGS QIVLTQSPAIMSASPGEKVTMTCSATSSVSYMHWYQQKSGTSPKRWIY DTSKLASGVPARFSGSGSGTSYSLTISSMEAEDAATYYCQQWSSNPLT FGAGTKLELK |
| 22 | OKT3 single chain variable fragment - amino acid | QVQLQQSGAELARPGASVKMSCKASGYTFTRYTMHWVKQRPGQGLE WIGYINPSRGYTNYNQKFKDKATLTTDKSSSTAYMQLSSLTSEDSAVYY CARYYDDHYSLDYWGQGTTLTVSSGGGGSGGGGSGGGGSGGGGSDI VLTQSPAIMSASPGEKVTMTCSASSSVSYMNWYQQKSGTSPKRWIYDT |
| | | SKLASGVPAHFRGSGSGTSYSLTISGMEAEDAATYYCQQWSSNPFTFG SGTKLEINR |
| 23 | CD28 domain - amino acid | IEVMYPPPYLDNEKSNGTIIHVKGKHLCPSPLFPGPSKPFWVLVVVGGVL ACYSLLVTVAFIIFWVRSKRSRLLHSDYMNMTPRRPGPTRKHYQPYAPP RDFAAYRS |
| 24 | CD8a domain - amino acid | FVPVFLPAKPTTTPAPRPPTPAPTIASQPLSLRPEACRPAAGGAVHTRGL DFACDIYIWAPLAGTCGVLLLSLVITLYC |
| 25 | 4-1BB domain - amino acid | KRGRKKLLYIFKQPFMRPVQTTQEEDGCSCRFPEEEEGGCEL |
| 26 | CD3-zeta domain - amino acid | RVKFSRSADAPAYQQGQNQLYNELNLGRREEYDVLDKRRGRDPEMGG KPRRKNPQEGLYNELQKDKMAEAYSEIGMKGERRRGKGHDGLYQGLS TATKDTYDALHMQALPPR |
| 27 | CAR1 full - amino acid | MALPVTALLLPLALLLHAARPEQKLISEEDLEVQLQQSGPELVKPGASVK MSCKASGYKFTSYVMHWVKQKPGQGLEWIGYINPYNDVTKYNEKFKGK ATLTSDKSSSTAYMELSSLTSEDSAVHYCARGSYYDYDGFVYWGQGTL VTVSAGGGGSGGGGSGGGGSGGGGSQIVLTQSPAIMSASPGEKVTMT CSATSSVSYMHWYQQKSGTSPKRWIYDTSKLASGVPARFSGSGSGTS YSLTISSMEAEDAATYYCQQWSSNPLTFGAGTKLELKIEVMYPPPYLDN EKSNGTIIHVKGKHLCPSPLFPGPSKPFWVLVVVGGVLACYSLLVTVAFII FWVRSKRSRLLHSDYMNMTPRRPGPTRKHYQPYAPPRDFAAYRSRVK FSRSADAPAYQQGQNQLYNELNLGRREEYDVLDKRRGRDPEMGGKPR RKNPQEGLYNELQKDKMAEAYSEIGMKGERRRGKGHDGLYQGLSTAT KDTYDALHMQALPPR |
| 28 | CAR2 full - amino acid | MALPVTALLLPLALLLHAARPEQKLISEEDLQVQLQQSGAELARPGASVK MSCKASGYTFTRYTMHWVKQRPGQGLEWIGYINPSRGYTNYNQKFKD KATLTTDKSSSTAYMQLSSLTSEDSAVYYCARYYDDHYSLDYWGQGTT LTVSSGGGGSGGGGSGGGGSGGGGSDIVLTQSPAIMSASPGEKVTMT CSASSSVSYMNWYQQKSGTSPKRWIYDTSKLASGVPAHFRGSGSGTS YSLTISGMEAEDAATYYCQQWSSNPFTFGSGTKLEINRIEVMYPPPYLD NEKSNGTIIHVKGKHLCPSPLFPGPSKPFWVLVVVGGVLACYSLLVTVAF IIFWVRSKRSRLLHSDYMNMTPRRPGPTRKHYQPYAPPRDFAAYRSRV KFSRSADAPAYQQGQNQLYNELNLGRREEYDVLDKRRGRDPEMGGKP RRKNPQEGLYNELQKDKMAEAYSEIGMKGERRRGKGHDGLYQGLSTA TKDTYDALHMQALPPR |
| 29 | CAR3 full - amino acid | MALPVTALLLPLALLLHAARPEQKLISEEDLEVQLQQSGPELVKPGASVK MSCKASGYKFTSYVMHWVKQKPGQGLEWIGYINPYNDVTKYNEKFKGK ATLTSDKSSSTAYMELSSLTSEDSAVHYCARGSYYDYDGFVYWGQGTL VTVSAGGGGSGGGGSGGGGSGGGGSQIVLTQSPAIMSASPGEKVTMT CSATSSVSYMHWYQQKSGTSPKRWIYDTSKLASGVPARFSGSGSGTS YSLTISSMEAEDAATYYCQQWSSNPLTFGAGTKLELKFVPVFLPAKPTTT PAPRPPTPAPTIASQPLSLRPEACRPAAGGAVHTRGLDFACDIYIWAPLA GTCGVLLLSLVITLYCKRGRKKLLYIFKQPFMRPVQTTQEEDGCSCRFPE EEEGGCELRVKFSRSADAPAYQQGQNQLYNELNLGRREEYDVLDKRR GRDPEMGGKPRRKNPQEGLYNELQKDKMAEAYSEIGMKGERRRGKG HDGLYQGLSTATKDTYDALHMQALPPR |
| 30 | CAR4 full - amino acid | MALPVTALLLPLALLLHAARPEQKLISEEDLQVQLQQSGAELARPGASVK MSCKASGYTFTRYTMHWVKQRPGQGLEWIGYINPSRGYTNYNQKFKD KATLTTDKSSSTAYMQLSSLTSEDSAVYYCARYYDDHYSLDYWGQGTT LTVSSGGGGSGGGGSGGGGSGGGGSDIVLTQSPAIMSASPGEKVTMT CSASSSVSYMNWYQQKSGTSPKRWIYDTSKLASGVPAHFRGSGSGTS YSLTISGMEAEDAATYYCQQWSSNPFTFGSGTKLEINRFVPVFLPAKPT TTPAPRPPTPAPTIASQPLSLRPEACRPAAGGAVHTRGLDFACDIYIWAP LAGTCGVLLLSLVITLYCKRGRKKLLYIFKQPFMRPVQTTQEEDGCSCRF PEEEEGGCELRVKFSRSADAPAYQQGQNQLYNELNLGRREEYDVLDK RRGRDPEMGGKPRRKNPQEGLYNELQKDKMAEAYSEIGMKGERRRGK GHDGLYQGLSTATKDTYDALHMQALPPR |
| 31 | muBMA13 VH - amino acid | EVQLQQSGPELVKPGASVKMSCKASGYKFTSYVMHWVKQKPGQGLE WIGYINPYNDVTKYNEKFKGKATLTSDKSSSTAYMELSSLTSEDSAVHY CARGSYYDYDGFVYWGQGTLVTVSAG |
| 32 | muBMA13 VL - amino acid | QIVLTQSPAIMSASPGEKVTMTCSATSSVSYMHWYQQKSGTSPKRWIY DTSKLASGVPARFSGSGSGTSYSLTISSMEAEDAATYYCQQWSSNPLT FGAGTKLELK |
| 33 | muBMA13 H-CDR-1 - amino acid | SYVMH |
| 34 | muBMA13 H-CDR-2 - amino acid | YINPYNDVTKYNEKFKG |
| 35 | muBMA13 H-CDR-3 - amino acid | GSYYDYDGFVY |
| 36 | muBMA13 L-CDR-1 - amino acid | SATSSVSYMH |
| 37 | muBMA13 L-CDR-2 - amino acid | DTSKLAS |
| 38 | muBMA13 L-CDR-3 - amino acid | QQWSSNPLT |
| 39 | OKT3 VH - amino acid | QVQLQQSGAELARPGASVKMSCKASGYTFTRYTMHWVKQRPGQGLE WIGYINPSRGYTNYNQKFKDKATLTTDKSSSTAYMQLSSLTSEDSAVYY CARYYDDHYSLDYWGQGTTLTVSSG |
| 40 | OKT3 VL - amino acid | DIVLTQSPAIMSASPGEKVTMTCSASSSVSYMNWYQQKSGTSPKRWIY DTSKLASGVPAHFRGSGSGTSYSLTISGMEAEDAATYYCQQWSSNPFT FGSGTKLEIN |
| 41 | OKT3 H-CDR-1 - amino acid | RYTMH |
| 42 | OKT3 H-CDR-2 - amino acid | YINPSRGYTNYNQKFKD |
| 43 | OKT3 H-CDR-3 - amino acid | YYDDHYSLDY |
| 44 | OKT3 L-CDR-1 - amino acid | SASSSVSYMN |
| 45 | OKT3 L-CDR-2 - amino acid | DTSKLAS |
| 46 | OKT3 L-CDR-3 - amino acid | QQWSSNPFT |
| 47 | Gly-Ser linker | GGGSGGGGSGGGGSGGGGS |
| 48 | Whitlow linker | GSTSGSGKPGSGEGSTKG |
| 49 | IgG1 spacer | PAEPKSPDKTHTCPPCPAPPVAGPSVFLFPPKPKDTLMIARTPEVTCVV VDVSHEDPEVKFNWYVDGVEVHNAKTKPREEQYNSTYRVVSVLTVLHQ DWLNGKEYKCKVSNKALPAPIEKTISKAKGQPREPQVYTLPPSRDELTK NQVSLTCLVKGFYPSDIAVEWESNGQPENNYKTTPPVLDSDGSFFLYSK LTVDKSRWQQGNVFSCSVMHEALHNHYTQKSLSLSPGKKDPK |
| 50 | IgG1 delta spacer | PAEPKSPDKTHTCPPCPAPPVAGPSVFLFPPKPKDTLMIARTPEVTCVV VDVSHEDPEVKFNWYVDGVEVHNAKTKPREEQYNSTYRVVSVLTVLHQ DWLNGKEYKCKVSNKALPAPIEKTISKAKGQPREPQVYTLPPSRDELTK NQVSLTCLVKGFYPSDIAVEWESNGQPENNYKTTPPVLDSDGSFFLYSK LTVDKSRWQQGNVFSCSVMHEALHNHYTQKSLSSLSPGKK |
| 51 | IgG4(Hi-CH2-CH3) spacer | ESKYGPPCPPCPAPEFEGGPSVFLFPPKPKDTLMISRTPEVTCVVVDVS QEDPEVQFNWYVDGVEVHNAKTKPREEQFNSTYRVVSVLTVLHQDWL NGKEYKCKVSNKGLPSSIEKTISKAKGQPREPQVYTLPPSQEEMTKNQV SLTCLVKGFYPSDIAVEWESNGQPENNYKTTPPVLDSDGSFFLYSRLTV DKSRWQEGNVFSCSVMHEALHNHYTQKSLSLSLGK |
| 52 | IgG4 (Hi-CH3) spacer | ESKYGPPCPPCPGQPREPQVYTLPPSQEEMTKNQVSLTCLVKGFYPSD IAVEWESNGQPENNYKTTPPVLDSDGSFFLYSRLTVDKSRWQEGNVFS CSVMHEALHNHYTQKSLSLSLGK |
| 53 | IgG4 (Hi) spacer | ESKYGPPCPPCP |

### FIGURES

The invention is demonstrated by way of the example through the figures disclosed herein. The figures provided represent particular, non-limiting embodiments and are not intended to limit the scope of the invention.

### Short description of the figures:

**Figure 1****:** Generation of TCR/CD3-specific CAR NK-92 cell lines for removal of residual TCR/CD3-expressing T cells within TCR-modified T cell products.
**Figure 2****:** Selection of the TCR/CD3-CAR NK-92 cell line with highest purification efficacy.
**Figure 3****:** Optimization of TCR/CD3-CAR NK-92 cell-mediated purification and comparison to MACS-mediated CD3-depletion.
**Figure 4****:** Characterization of G-Rex-expanded TCR/CD3-depleted CAR T cell products.
**Figure 5****:** Disappearance of viable gene-edited and wildtype NK-92 cells after gamma-irradiation (30 Gy).
**Figure 6****:** Tyrosine-kinase inhibitor Dasatinib prevents CAR4 NK-92 cell mediated lysis of TCR/CD3+ T cells.
**Figure 7****:** Characterization of TCR/CD3-depleted CAR T cell products.
**Figure 8****:** Additional optimizations of co-culture conditions and extended monitoring of TCR/CD3+ cell fractions during cytokine-driven expansion.
**Figure 9****:** Further functional characterization of CAR T cell products after purification by CAR4 NK-92 cell co-culture or MACS.

### Detailed description of the figures:

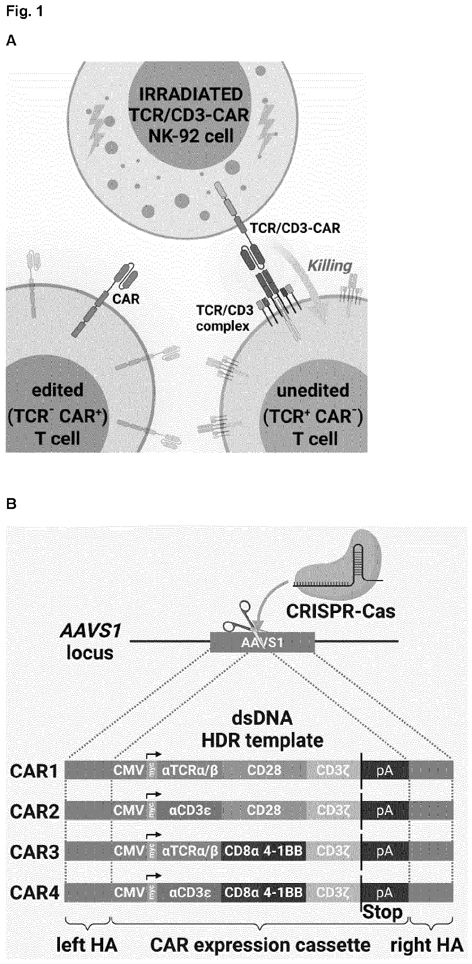

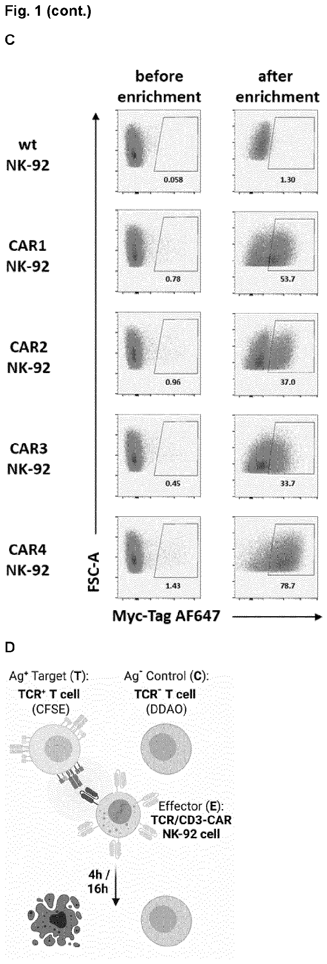

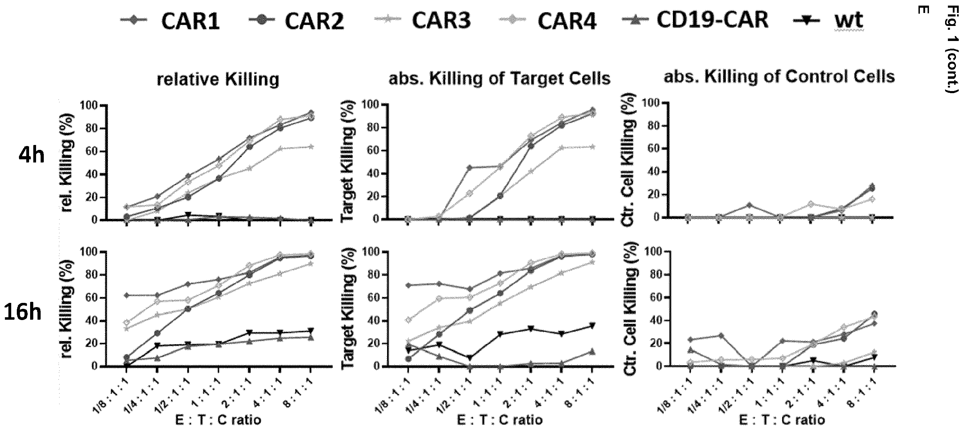

**Figure 1****: Generation of TCR/CD3-specific CAR NK-92 cell lines for removal of residual TCR/CD3-expressing T cells within TCR-modified T cell products. a)** Outline of cell mediated purification strategy. NK-92 cells equipped with a CAR specific for the TCR/CD3 complex are added to expanding TCR-modified (CAR) T cells after gamma-irradiation (30 Gy). The TCR/CD3-CAR-directed cytotoxicity of the NK-92 cells then leads to a reduction/removal of residual TCR-wildtype T cells within the TCR-modified T cell product. **b)** Generation of TCR/CD3-specific CAR NK-92 cell lines by site-specific integration of 4 CARs (termed CAR1-CAR4) with specificity either for the TCRα/β dimer or for CD3ε into the human *AAVS1* safe-harbor locus. **c)** CAR expression after knock-in (left) and after MACS-enrichment. **d,e)** Experimental setup and results of flow cytometric VITAL assays to test the effector function of the four gene-modified TCR/CD3-CAR NK-92 cell lines.

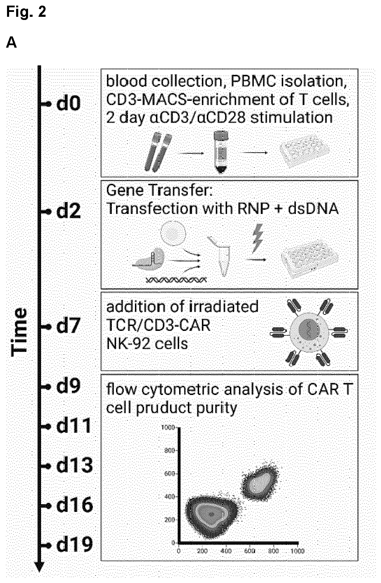

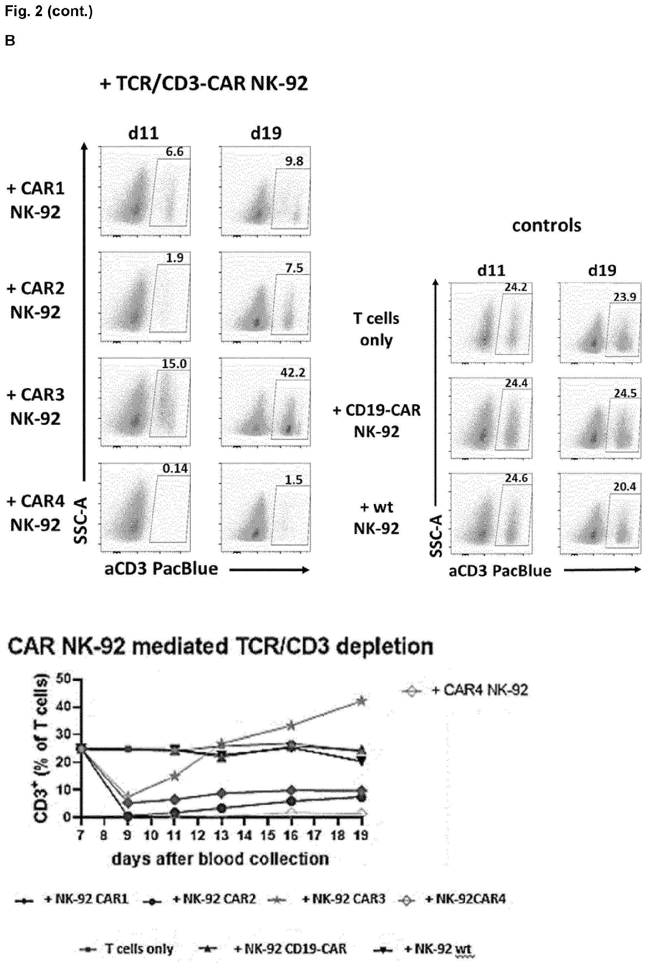

**Figure 2****: Selection of the TCR/CD3-CAR NK-92 cell line with highest purification efficacy, a)** Experimental setup for non-viral TCR-to-CAR replacement (in T cells) and subsequent NK-92 cell-mediated depletion of remaining TCR-unedited cells. **b)** Flow cytometric detection of residual TCR-wildtype T cells after co-culture with TCR/CD3-CAR NK-92 cells (left) or in control conditions (right); and summary of data (bottom right).

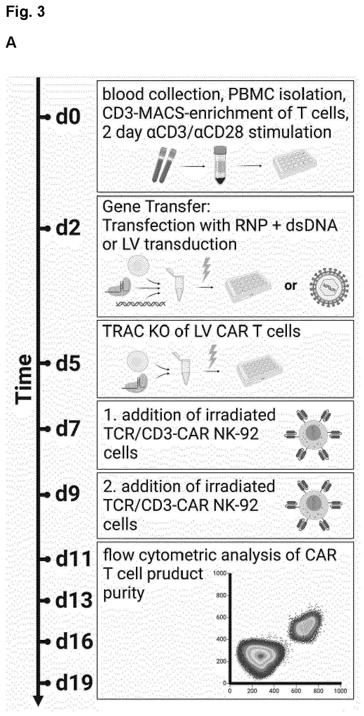

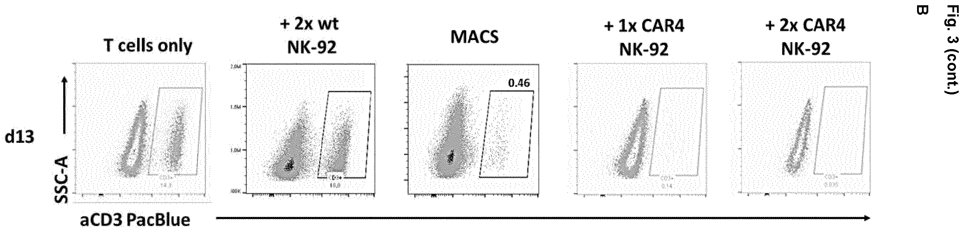

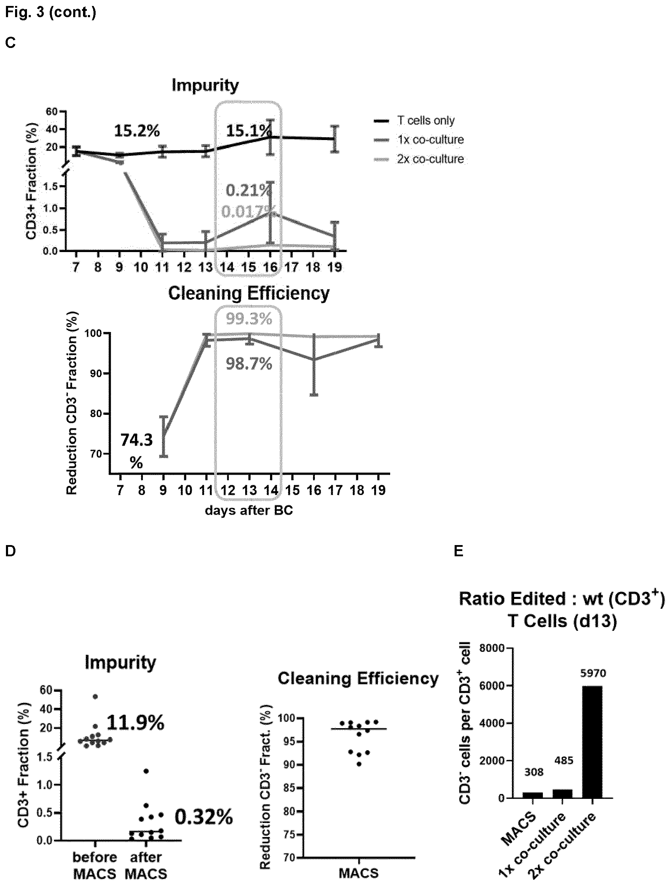

**Figure 3****: Optimization of TCR/CD3-CAR NK-92 cell-mediated purification and comparison to MACS-mediated CD3-depletion. a)** Experimental setup for lentiviral or virus-free TCR-to-CAR replacement and subsequent NK-92 cell-mediated depletion of remaining TCR-unedited cells in one or two co-culture cycles. **b)** Flow cytometric detection of residual TCR-wildtype T cells cultured alone (1^{st} dot plot), after two co-culture cycles with unedited NK-92 cells (2^{nd} dot plot), after MACS-mediated CD3-depletion (3^{rd} dot plot), or after one (4^{th} dot plot) or two (5^{th} dot plot) co-culture cycles with TCR/CD3-specific (CAR4) NK-92 cells. **c)** Summary of impurity (in terms of the remaining TCR/CD3⁺ cellular fraction) and cleaning efficiency (relative reduction of the CD3⁺ fraction) after 0, 1 or 2 co-culture cycles. **d)** Impurity and cleaning efficiency after MACS-mediated CD3-depletion. **e)** Ratio of successfully edited (TCR/CD3⁻) cells per 1 remaining unedited (TCR/CD3⁺) wildtype cell.

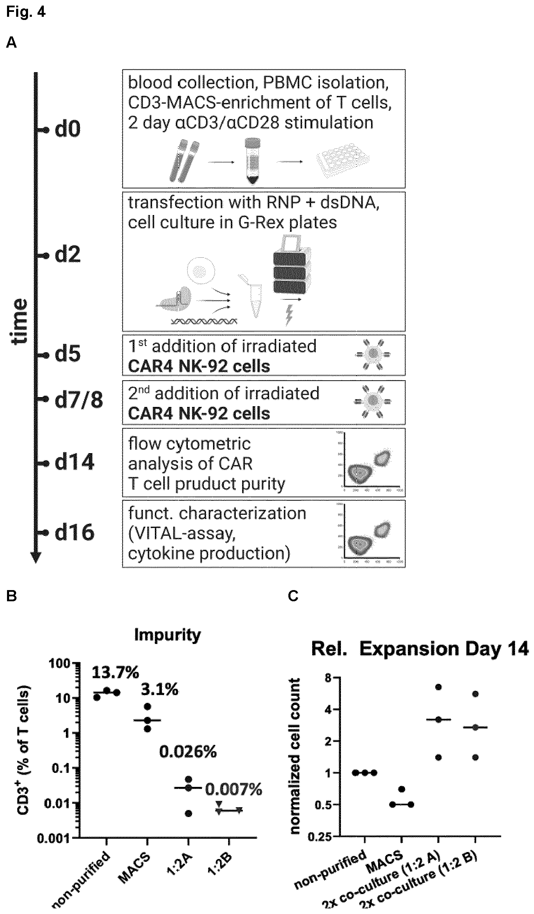

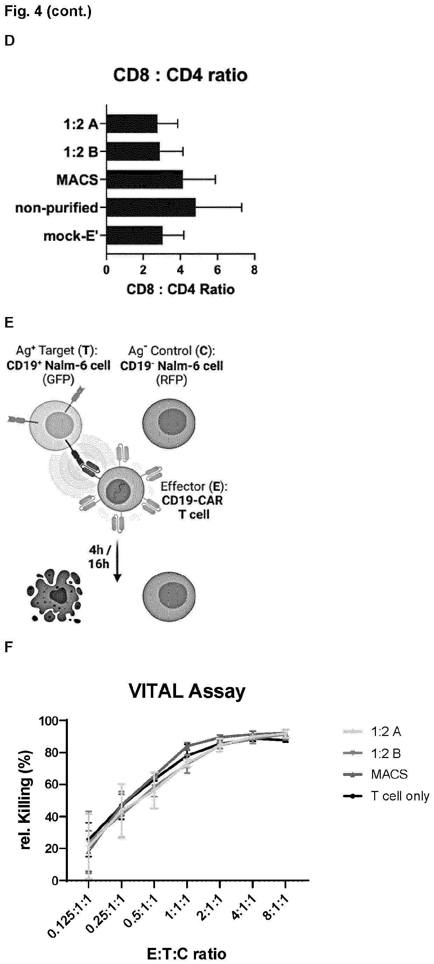

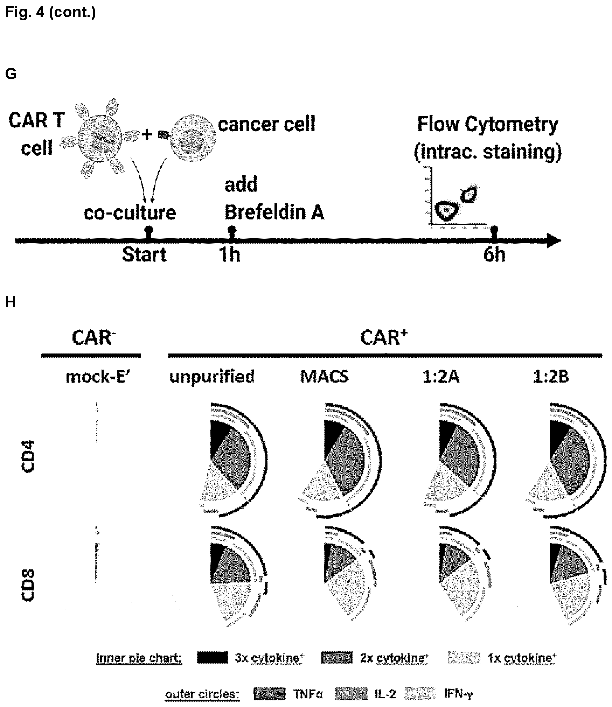

**Figure 4****: Characterization of G-Rex-expanded TCR/CD3-depleted CAR T cell products. a)** Experimental setup for non-viral TRAC-integration of a CD19-CAR in primary human T cells and subsequent CAR4 NK-92 cell-mediated depletion of remaining TCR-unedited cells. **b)** Remaining TCR/CD3⁺ (wildtype) T cell fraction after purification, **c)** Relative cell counts at the end of the CAR T cell expansion phase. **d)** CD8:CD4 ratios of CAR T cells at the end of the expansion phase. **e,f)** Experimental setup (**e**) and results (**f**) of a VITAL Assay to assess target-specific cytotoxicity of the differently purified CD19-CAR T cells. **g,h)** Experimental setup (**g**) and results (**h**) of an intracellular cytokine expression assay to assess effector function of the purified CD19-CAR T cells after CD19⁺ target cell encounter.

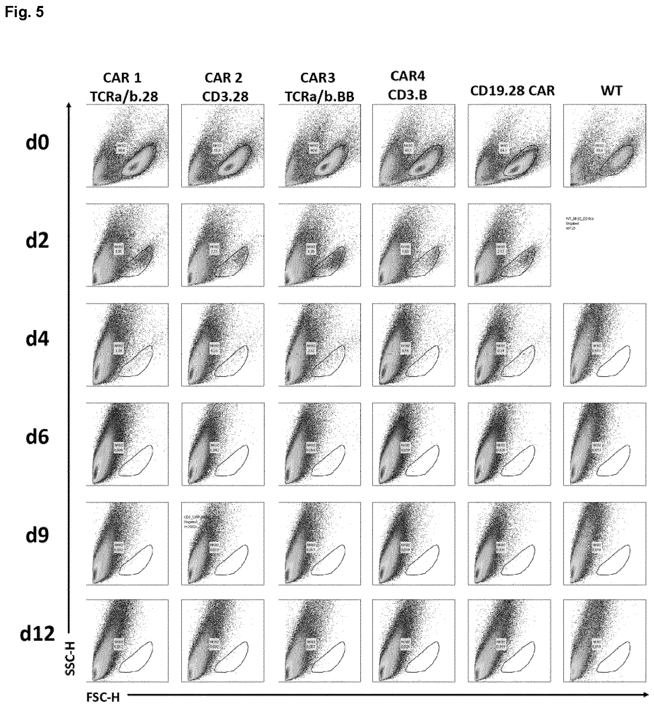

**Figure 5****: Disappearance of viable gene-edited and wildtype NK-92 cells after gamma-irradiation (30 Gy).**

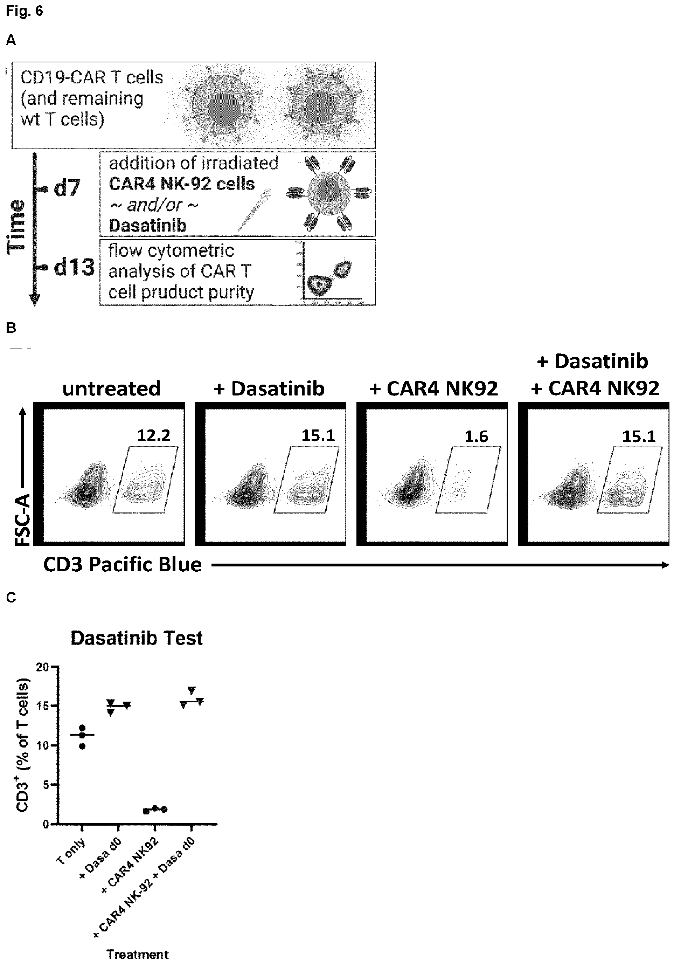

**Figure 6****: Tyrosine-kinase inhibitor Dasatinib prevents CAR4 NK-92 cell mediated lysis of TCR/CD3⁺ T cells. a)** Experimental setup to study the mechanism of CAR4 NK-92 mediated TCR/CD3⁺ T cell depletion. CAR4 NK-92 cells or Dasatinib or the combination of the two were added to expanding *TRAC*-edited CAR T cells. The percentage of remaining TCR/CD3⁺ (wildtype) T cells was assessed by flow cytometry. **b,c)** Representative density plots (**b**) and summary (**c**) of flow cytometric detection of residual TCR/CD3⁺ (wildtype) T cells.

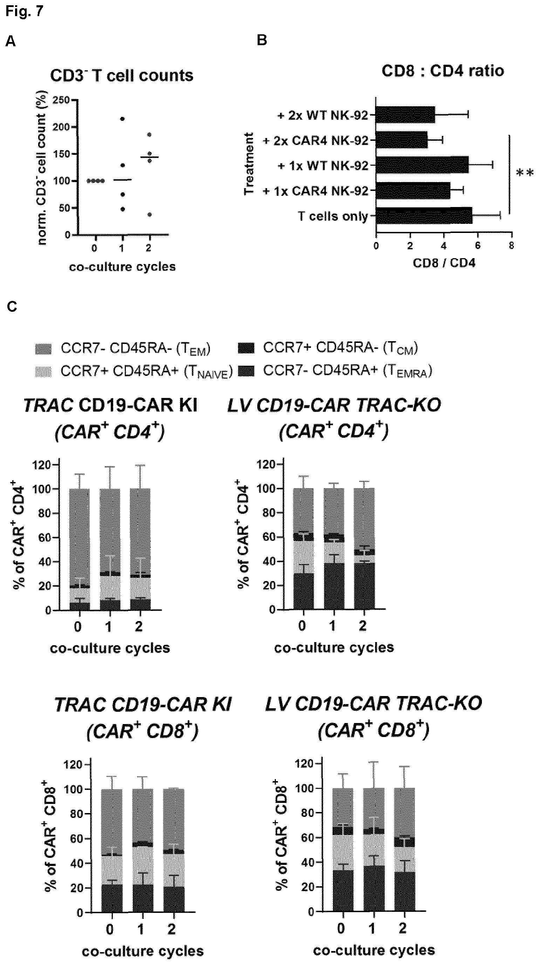

**Figure 7****: Characterization of TCR/CD3-depleted CAR T cell products.** a) relative cell counts at the end of the CAR T cell expansion phase. CAR T cells were either culture alone (black) or co-cultured once (blue) or twice (pink) with TCR/CD3-specific (CAR4) NK-92 cells. b) CD8:CD4 ratios of CAR T cells cultured alone or co-cultured once (blue) or twice (pink) either with wildtype NK-92 cells or gene-modified TCR/CD3-specific (CAR4) NK-92 cells. c) CAR T cell surface expression of CCR7 and CD45RA as markers for memory phenotype.

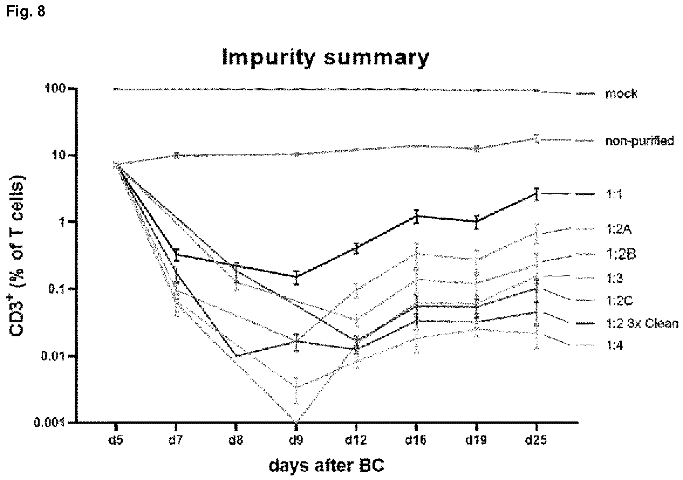

**Figure 8****: Additional optimizations of co-culture conditions and extended monitoring of TCR/CD3+ cell fractions during cytokine-driven expansion.**

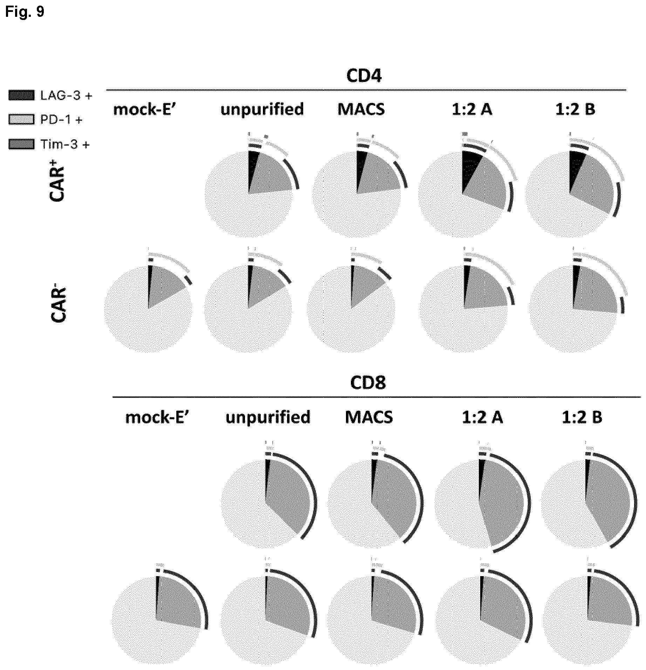

**Figure 9****: Further functional characterization of CAR T cell products after purification by CAR4 NK-92 cell co-culture or MACS.** Expression of inhibitory receptors LAG-3, PD-1, Tim-3. Colors of the outer circle represent individual markers while the number of inhibitory receptors per cell is indicated by different shades of grey within the inner circle.

### EXAMPLES

The invention is demonstrated through the examples disclosed herein. The examples provided represent particular embodiments and are not intended to limit the scope of the invention. The examples are to be considered as providing a non-limiting illustration and technical support for carrying out the invention.

### Example 1: Generation of CAR NK92 cell lines

For the purpose of TCR/CD3⁺ T cell depletion by TCR/CD3-specific CAR NK-92 cells (**Fig. 1a**), we rationally designed a total of four TCR/CD3 complex-targeting, myc-tagged second-generation CARs for expression in NK-92 cells. As antigen recognition domains, we tested one single chain variable fragment (scFv) specific for CD3ε (clone OKT3, patent US5929212A), and one scFv specific for TCRα/β dimers (clone BMA031^{18,19,20}). Each scFv was fused on a CD28.CD3ζ and a CD8α.4-1BB.CD3ζ CAR base thus creating four TCR/CD3-specific CARs termed CAR1 - CAR4 (**Fig. 1b**). CAR-encoding DNA sequences were cloned into plasmid backbones carrying a cytomegalovirus- (CMV) derived promotor and two flanking 400 bp sequences homologous to the human AAVS1 safe-harbor locus. Relevant DNA and protein sequences are shown in the **sequence table above.** These homology-arm flanked CAR expression cassettes were PCR-amplified and delivered into NK-92 cells by electroporation together with AAVS1 locus targeting Cas9-sgRNA ribonucleoprotein for nuclease-assisted transgene integration. Electroporation was performed in a Lonza 4D nucleofector device with 1× 10⁶ NK92-cells suspended in 20 µl SF Cell Line Nucleofector Solution, 1 µg CAR dsDNA donor template, 4 µg Cas9 complexed to 1.5 µg sgRNA pre-mixed with poly-L-glutamic acid²¹ using electroporation program CA-137. Initial CAR⁺ rates assessed by flow cytometry two weeks after electroporation ranged from 3 to 5 % and were increased up to 85% by two rounds of magnetic cell separation (MACS) via the CAR's myc-tag (**Fig. 1c**).

### Example 2: Initial testing of four CAR NK-92 cell lines

The killing capacity of the genetically modified NK-92 cell lines was first assessed in VITAL assays²² which are co-culture assays with three cell types: 1.) effector cells, here the TCR/CD3-specific CAR NK-92 cells; 2.) target cells, here fluorescently labeled (CFSE) wildtype T cells and 3.) control cells, here fluorescently labeled (DDAO) TCR/CD3-KO T cells (**Fig. 1d**). Wildtype NK-92 cells and CD19-specific CAR NK-92 cells (as irrelevant effectors) served as negative controls. For the four TCR/CD3-specific CAR NK-92 cell lines, VITAL assays run over 4 and 16 hours showed high target-specific cytotoxicity, i.e. preferential killing of wildtype T cells relative to TCR/CD3-KO T cells (**Fig. 1e****, left**). Only the CAR3 (TCRα/β.CD8α.4-1BB.CD3ζ CAR) NK-92 cell line showed a somewhat more moderate relative killing capacity, but remained effective. Importantly, while absolute target cell numbers drastically decreased, absolute cell numbers of TCR/CD3-KO control cells were only affected to a moderate extent at high effector to target ratios, indicating minimal off-target cytotoxicity of the redirected NK-92 cell lines (**Fig. 1e****, center and right panels**).

### Example 3: Selection of one CAR NK-92 cell line for depletion of unedited T cells after TCR-KO

We tested the four cell lines' ability to selectively eradicate the remaining fraction of potentially allo-reactive TCR/CD3⁺ (wildtype) T cells within expanding TCR/CD3-KO CAR T cells. Such TCR/CD3-deleted CAR T cells were generated from primary human T cells either via lentiviral (LV) gene transfer and subsequent TRAC-KO, or via site-specific integration of the CAR transgene into the *TRAC* locus as previously reported⁹ (**Fig. 2a**). Following gamma-irradiation (30 Gy), TCR/CD3-specific CAR NK-92 cells were added in a 2:1 cellular ratio to the expanding CAR T cells on day 7 after T cell isolation. Gamma-irradiation of NK-92 derived cell lines is a standard safety measurement taken before their clinical application which prevents their outgrowth without affecting their short-term cytotoxicity ¹⁷. Purity of the expanding CAR T cells was assessed repeatedly over the following 12 days until day 19 after T cell isolation. This single administration of all four CAR NK-92 cell lines led to an immediate reduction of remaining wildtype TCR/CD3⁺ T cells. Inhibition of CAR activation in irradiated NK-92 cells using the tyrosine kinase inhibitor Dasatinib prevented the elimination of TCR⁺ T cells, highlighting that lysis of wildtype T cells is supported by CAR NK-92 activation within the first 24 hours of the co-culture (**Fig. 6**). A re-appearance of wildtype T cells starting 6 days after CAR NK-92 cell administration was seen for all four CAR NK-92 cell lines and was highest for CAR3 (**Fig. 2b**). The lowest percentage of contaminating TCR/CD3⁺ cells at the end of the CAR T cell expansion phase was detected for CAR4, thus the CAR4 NK-92 cell line was selected for all further studies.

### Example 4: A second administration of CAR NK-92 cells to increase CAR T product purity

We compared a single administration versus two consecutive co-culture cycles of CAR4 NK-92 cells (**Fig. 3a**). Six days after the initial co-culture (day 13 after T cell isolation), a time point towards the late phase of a typical CAR T cell product expansion phase, purity of TCR/CD3-deleted CAR T cells was 63 % without, 99.8% with a single, and >99.98% with two rounds of CAR4 NK-92 cell administration (**Fig. 3b****,c**). This translates to a removal of 98.6% of residual wildtype T cells through a single administration and more than 99.9% through 2 co-culture cycles. CD3-MACS-depletion, also performed on day 13 after T cell isolation for comparison in an independent experiment, only increased product purity from 88.1% to 99.67%, which translates to a removal of contaminating TCR/CD3+ T cells of only 97.3% (**Fig. 3d**). Thus, frequencies of potentially allo-reactive wildtype T cells achieved with our method are approx. 19-fold lower than with classic bead-mediated depletion. The degree of product purity can also be visualized by the number of edited T cells per one unedited (wildtype) T cell (edited : wildtype ratio, **Fig. 3e**). The large divergence in this parameter highlights the dramatic difference in efficiency of the two approaches. Importantly, at this small scale we did not observe any significant changes in the total CD3⁻ T cell counts nor in the memory phenotype within the final CAR T cell product (**Fig. 7**). Interestingly, CD4:CD8 ratios were moderately changed towards a more balanced state from 1:5.7 without, 1:4.4 with a single, and 1:3.1 with two treatment cycles. This appears to be a general effect of NK-92 cell co-culture, as a similar shift was observed after co-culture with unmodified NK-92 cells.

### Example 5: Additional optimizations and extended monitoring during cytokine-driven expansion

We evaluated additional parameters to improve the purity of TCR⁻ CAR T cell products, including different T cell to CAR4 NK-92 cell ratios (conditions 1:1 up to 1:4), (2) spacing between the co-cultures (conditions; A: 2 days, B, C: 3 days) and (3) a third co-culture (3x clean-up). To accommodate all the different conditions, the optimizations were performed in 24-well-culture plate format. The CAR T cells were monitored over an extended time frame up to day 25 after blood collection (**Fig. 8**). As expected, higher CAR4 NK-92 amounts during co-culture and additional 3^{rd} clean-up co-culture tended to improve the purity. Similarly, increasing the time between first and second co-culture led to improved purity and more sustained TCR-depletion. However, at the envisioned time point for CAR T cell formulation (days 12-14 after blood collection), conditions with higher T cell: NK92 cell ratios (and additional clean-ups) were also effective. Nevertheless, 1:2 T cell to CAR4 NK-92 cell ratio and two NK cell treatments (clean-ups) are considered as the most economical (efficient) approach for scaling attempts, although other ratios and numbers of treatments also are effective.

### Example 6: CAR NK-92 cell mediated depletion can be scaled within the G-rex bioreactor system

Allogeneic CAR T cell manufacturing requires large volumes, and the process may optimally be performed in a closed or semi-closed system. Therefore, we tested whether cell-mediated clean-up would also be feasible in the G-Rex cell culture device. Herein, we assessed the two clean-up co-cultures spaced by 2 (condition 1:2A) or 3 days (conditions 1:2B). In regimen 1:2A, both co-cultures were performed at a 1:2 NK-92 to T cell ratio, while for regimen 1:2B, the second co-culture was performed at a 1:1 ratio. For comparison, *TRAC*-replaced CAR T cells were also expanded in separate G-Rex culture wells and CD3-MACS depleted on day 13 (**Figure 4a**)**.** With average TCR⁺ T cells of 0.026% (1:2A) and 0.007% (1:2B), both CAR4 NK-92 approaches yielded higher purity than MACS (mean: 3.1% TCR+ T cells) (**Figure 4b**). Intriguingly, we observed improved expansion of the CAR T cells when CAR4 NK-92 cells were added in G-Rex leading to 3-fold and 6-fold higher TCR⁻ T cell yield compared to unpurified and MACS-purified CAR T cells, respectively (**Figure 4c**). We observed an increased proportion of CD4 T cells after expansion in the conditions treated with irradiated CAR4 NK-92 cells compared to non-purified or MACS-depleted CAR T cells (**Figure 4d**). Phenotypical analysis of CAR T cells demonstrated no significant impact on the expression of inhibitory receptors, Lag-3, Tim-3 and PD-1, which are usually associated with T cell exhaustion (**Fig. 9**).

### Example 7: Characterization of TCR/CD3-depleted CAR T cell products

In addition to the degree of TCR/CD3-wildtype T cell depletion, we assessed CD19-CAR-dependent cytotoxicity and cytokine production as parameters of CAR T cell product functionality (**Fig. 4e****-h**). In a VITAL assay, CD19-CAR T cells induced dose-dependent lysis of CD19⁺ Nalm-6 cells regardless of the chosen purification method (**Figure 4f**). Similarly, the rates of intracellularly detected cytokines after tumor cell challenge were comparable among all groups (**Figure 4h**).

### Discussion of the Examples:

A novel strategy to purify TCR-disrupted T cells via co-culture with gamma-irradiated TCR/CD3-specific NK-92 cells during *ex vivo* expansion is described in the examples herein. Production of multiple efficient CD3-specific CAR constructs in NK-92 cells enabled a process with purification rates exceeding 99.9% TCR- T cells displaying a >10-fold improvement over magnetic cell separation in our hands or reported in studies preparing or performing clinical trials on TCR-edited CAR T cells¹⁰⁻¹². Co-cultures with irradiated TCR/CD3-specific CAR NK-92 cells appear to also support CAR T cell expansion and do not negatively impact *in vitro* CAR T cell function. We expect positive effects to the CD4:CD8 ratio and anti-tumor performance *in vivo,* experiments are ongoing.

Previous trials have established CAR T cell dose-response relationships with higher CAR T cell counts associating with better treatment response¹⁵. Therefore, optimizing manufacturing to achieve high yields and enable higher dosages is paramount to ensure optimal cost-effectiveness despite additional release test requirements of allogeneic cell products¹. Like Jullierat et al¹⁶, the use of TCR/CD3-specific CAR NK-92 cells replaces bead-mediated depletion procedures with an uncomplicated co-culture. It simplifies cell handling and avoids cell loss associated with magnetic bead separation prior to CAR T cell product formulation.

Compared to Jullierat et al¹⁶ we achieve more efficient and cost-effective manufacture of CAR NK cells using gene editing strategies, as disclosed by way of example in the examples. There is also a reduced risk of malignant transformation compared to viral vector modified cells due to a reduced risk of insertional mutagenesis and lower transgene copy numbers.

Additional advantages are evident over the prior art approaches. Jullierat et al¹⁶ shows about 0.5-1.0% residual CD3+/TCRαβ+ T cells in the final mixture after CAR NK cell treatment; therefore their method seems to have less effective and less reproducible reduction of unwanted T cells below 0.5% CD3+ T cells. A further improvement in the present method over Jullierat et al¹⁶ is that they observed skewing toward CD8+ T cells. This is suboptimal and in contrast to the present method, which actually increases the amount of CD4+ cells relative to the control (untreated) conditions. An additional risk to the approach of Jullierat et al¹⁶ is that the CAR could trigger additional differentiation of the CAR T cells, introducing a potentially disruptive feature to the cells of the prior art. Thus, the approach employed herein using gene editing techniques to generate anti-TCR CAR NK cells is associated with multiple unexpected advantages over he prior art.

Autologous or allogeneic feeder cells are used in many protocols for CAR T cell generation in preclinical and also clinical settings²³⁻²⁵. Further, in G-Rex devices, the CAR NK-92 cells supported the expansion of TCR-edited CAR T cells demonstrating a "feeder cell effect" (**Fig. 4c**). By enhancing both the product purity and optimizing the overall cell yield, we increase the safety of high dosages of allogeneic CAR T cells without exceeding the TCR⁺ T cell per kg bodyweight thresholds associated with GvHD.

Prior to clinical use, a CAR4 NK-92 cell line would be established as a master cell bank under good manufacturing practice (GMP) conditions¹. Building on GMP-compatible protocols^{9,29,30}, large scale production of CAR T cells may be performed by combining non-viral gene editing and co-culture with CAR NK-92 in the semi-closed G-rex culture devices²⁴. Our approach is considered compatible with other bioreactor systems or automated cell manufacturing devices³⁶. Due to the previous clinical application of gamma-irradiated NK-92 cells^{17,37}, adding the CAR NK-92 cells only adds minor risks to the process development. To mediate any potential risk, release testing of CAR T cell products can include an assay for the detection of residual alive CAR4 NK-92 cells, due to their potential to deplete the patient's T cells *in vivo.*

The presented strategy may be used to improve CAR T cell yields for many allogeneic CAR T cell product candidates which have been previously described, including donor-derived CAR T cells for leukemia after allogeneic hematopoietic stem cell transplantation²⁶, universal off-the-shelf CAR T cells for T cell malignancies^{12,27,28}, multiple myeloma²⁹ or even CAR T cells for solid tumors³⁰. Hypo-immunogenic cell products with B2M-KO overexpressing NK cell inhibitory receptors, such as HLA-E^{32,33} or CD47^{34,35}, may be combined with our NK-92 cell based approach in future studies. Further, the cell-based depletion strategy can be adopted to deplete TCRαβ T cells in other cell expansion systems, such as TCRγδ or NK cell preparations. To this end, optimizing the CAR construct with other binders, such as the TCRαβ-specific clone BMA03, or identifying other binders which enable targeting of specific T cell subsets, is underway.

### Materials and Methods:

*Cell sources:* Frozen culture aliquots of Natural Killer cell line NK-92 derived from natural killer lymphoma and NALM-6 B cell precursor leukemia cell line were purchased from the Leibniz Institute, DSMZ-German Collection of Microorganisms and Cell Cultures GmbH. Primary T cells were obtained from healthy human adults' peripheral blood. Informed consent was collected following the declaration of Helsinki (Charité ethics committee approval EA4/091/19).

*PBMC isolation and T cell enrichment:* Density-gradient isolation of peripheral blood mononuclear cells (PBMC) was executed on sterile PBS (Gibco) 1:1 diluted whole blood (heparin tube collection). Subsequently the solution was stratified onto PANcoll separation medium (PAN Biotech, Germany) in 50 ml tubes (Falcon) and centrifuged at 800 g or 20 minutes at room temperature without applied brake. The newly formed mononuclear cell layer was harvested and diluted in sterile PBS. Pellet obtained after 450 g for 10 minutes centrifugation, was once again resuspended in sterile PBS and centrifuged at 300 g for 10 minutes. PBMCs pellets thus obtained were resuspended in 20 ml PBS and counted using a Neubauer hemocytometer. Positive selection for T cells via magnetic cell separation (MACS) was performed with CD3 microbeads (Miltenyi Biotec, 130-050-101) and LS columns, (Miltenyi Biotec, 130-042-401) following the manufacturer's recommendations.

*Cell thawing:* Primary wildtype T cells, CAR T cells and NK-92 derived cells lines were thawed in pre-warmed RPMI 1640 (PAN Biotech), 20% heat-inactivated fetal calf serum (FCS) (Biochrom) and 10 µg/mL DNase I (Roche) (thawing medium). Cryotubes were quickly transferred from liquid nitrogen to a 37°C pre-warmed water bath until the frozen cells' solution becomes liquid again. Cells are diluted 10 times in pre-warmed thawing media in 50 ml tubes (Falcon) and carefully inverted to resuspend. After centrifugation at 280 g for 10 minutes the cell pellet was once again resuspended in thawing medium and centrifuged. Cells were then resuspended in desired medium at the required cell concentration for downstream experiments.

*Cell culture:* NK-92 cell line grows clumps-like in suspension and was cultured in 25 cm² culture flasks (Gereiner) with RPMI 1640 (PAN Biotech), 10% heat-inactivated fetal calf serum (FCS) (Biochrom), 1% penicillin-streptomycin (Gibco) and recombinant human IL-2 (500 IU/ml, Miltenyi). MACS-enriched T cells were cultured in T cell medium [RPMI 1640 (PAN Biotech), 10% heat-inactivated fetal calf serum (FCS) (Biochrom) and recombinant human IL-2 (200 IU/ml), IL-7 (10 ng/ml) and IL-15 (5 ng/ml)]. Stimulation of enriched T cells was performed for 48 hours via culture in anti-CD3/28 antibody-coated 24-well polystyrene tissue culture plates (Corning Inc.). Overnight incubation of culture plates with 500 µl/well of sterile ddH2O supplemented with 1 µg/ml anti-CD3 mAb (clone OKT3, Invitrogen) and 1 µg/ml anti-CD28 mAb (clone CD28.2, Biolegend) provided the necessary antibodies coating. After removal of non-bonded antibodies through sterile PBS rinsing, 1-1.5× 10⁶ per 24-well T cells were seeded. GFP- or RFP-expressing NALM6 cell lines were cultured in RPMI 1640 (PAN Biotech), 10% heat-inactivated fetal calf serum (FCS) (Biochrom). All cell culture was performed at 37°C and 5% CO₂.

*Generation of dsDNA donor template for homology-directed CAR Transgene insertion into the human AAVS1 safe harbor locus:* All DNA sequences are shown in Suppl. Table 1. Design of dsDNA donor templates for HDR-mediated CAR transgene integration was performed as recently described⁹. Molecular assembly was performed starting with an AAVS1 donor template plasmid provided by Dr. Anne Floriane Hennig and Prof. Dr. Uwe Kornak (University of Göttingen). In brief, such dsDNA donor templates consist of a CAR expression cassette (comprising a CMV promoter, the actual CAR transgene and a bovine growth hormone (bGH) derived poly-adenylation sequence) which is flanked by 400 bp long sequences identical to the genomic DNA of the AAVS1 locus at the site of the nuclease-induced DNA double strand break (homology arms). The CAR transgene itself encodes an myc-tagged antibody-derived single chain variable fragment (scFv) as its antigen recognition domain fused either to a CD28.CD3-zeta or a CD8a.4-1BB.CD3-zeta second generation CAR stalk as done by Alabanza et *al*.³⁹ Newly designed DNA sequences were synthesized as 'gBlocks' by Integrated DNA Technologies (IDT). Molecular cloning of plasmids carrying the donor templates was planned with SnapGene software (from Insightful Science; snapgene.com) and performed as multiple-fragment In-Fusion cloning according to the manufacturer's protocol (Clontech, Takara) with the exception that all reaction volumes were reduced by 50% thus preserving the recommended volume ratios. Plasmid purification was performed using the ZymoPURE Plasmid Mini Prep Kit (Zymo Research). Sanger Sequencing (performed at LGC Genomics, Berlin) was used for sequence validation of HDR-templates. PCR with KAPA HiFi HotStart 2x Readymix (Roche) was used to amplify HDR-templates in reaction volumes < 500 µl. Paramagnetic beads (Ampure XP, Beckman Coulter) were used to purify and concentrate PCR products. PCR-amplified HDR-template concentrations were measured on a Qubit 4 Fluorometer (ThermoFisher) using the Qubit dsDNA BR Assay and afterwards adjusted 1000 ng/µl.

*Non-viral CAR transfer to primary human T cells:* Non-viral CAR transfer to primary human T cells was performed as previously described.⁹ph In short, T cells activated over 48 hours on αCD3/αCD28 antibody coated well plates were harvested, washed twice in PBS at 100x g for 10 min using 50 ml Falcon tubes in the first, and 1.5 ml microcentrifuge tubes in the second centrifugation step. Meanwhile, *TRAC*-targeting synthetic modified sgRNA (100 µM in TE buffer, IDT) was mixed with a 100 µg/µL aqueous solution of 15-50 kDa poly(L-glutamic acid) (PGA, Sigma-Aldrich, P4761-500MG)²¹ and recombinant SpCas9 protein (61 µM, IDT, 10000735) in a 0.96:1:0.8 volume ratio to form precomplexed ribonucleoproteins (RNP). T cells were resuspended in TheraPEAK^{®} P3 Primary Cell Nucleofector^{®} Solution (Lonza, G4LP3-22500) at a concentration of 5-10 × 10⁷ cells / ml and mixed with RNP (10.35 µl / 100 µl cell suspension) and dsDNA template (2.5 µl / 100 µl cell suspension) encoding an intermediate-spacer CD19.CD28.CD3ζ-CAR. T cells were then electroporated (either in 20 µl or in 100 µl electroporation vessels) in a Lonza 4D nucleofector device using program EH-115. Immediately after electroporation, pre-warmed (antibiotic-free) T cell medium was added to the electroporated cell suspension in a 5:1 volume ratio.

*Lentiviral CAR transfer to primary human T cells:* Primary human T cells were cultured in T cell medium on anti-CD3/CD28-coated tissue culture plates for a total of two days. 1 day after the start of the stimulation, transduction was performed on these plates by centrifugation at 800*g* for 30 min at 32°C with lentiviral supernatant (MOI of 1) supplemented with 1 mg/mL protamine sulfate (APP Pharmaceuticals, Barceloneta, Puerto Rico). Transduced cells were taken off the stimulation plate the following day.

*Generation of TCR*/*CD3-CAR NK-92 cell lines:* TCR/CD3-CAR transfer to NK-92 cells was performed by electroporation in a Lonza 4D nucleofector device with 1× 10⁶ NK92-cells suspended in 20 µl SF Cell Line Nucleofector Solution (Lonza), 1 µg CAR dsDNA donor template, 4 µg Cas9 complexed to 1.5 µg sgRNA pre-mixed with poly-L-glutamic acid²¹ using electroporation program CA-137. CAR+ cells were enriched twice by magnetic cell separation (MACS) via the CAR's myc-tag using myc-tag-specific Phycoerythrin (PE)-conjugated antibody (clone 9B11, Cell Signaling Technology Inc., 3739S) and anti-PE microbeads (Miltenyi, 130-048-801).

*Gamma-irradiation of NK-92 derived cell lines:* Wildtype and Gene-modified NK-92 cells were harvested, counted and transported to the irradiation facility at room temperature where they were gamma-irradiated at 30 Gy with a GSR-D1 radiation machine (Gamma-Service Medical GmbH). Before use in further assays, cells were centrifuged at 100g for 10 min and resuspended in T cell medium. All cell handling was performed at room temperature.

*VITAL assay*²² *to assess cytotoxicity of CAR NK-92 cell lines:* CFSE-labeled wildtype T cells (Target cells) and DDAO-labeled TCR/CD3-KO T cells (Control cells) were mixed, washed and resuspended in RPMI medium supplemented with 10 % FCS in a 1:1 cellular ratio at a concentration of 2.5 × 10⁵ cells of each kind /ml. The Target:Control cell suspension mixture was seeded in a round-bottom 96-well cell culture plate (100 ul = 25.000 cells of each kind /well). Unlabeled Effector cells (the four TCR/CD3-CAR NK-92 cell lines, wildtype NK-92 cells and a CD19-CAR NK-92 cell line) were added to separate wells of the Target:Control mix at different ratios. After mixing, the plates were centrifuged at 100x g for 3 min and incubated at 37°C and 5% CO₂. At two timepoints (4 and 16 hours) cells were resuspended and mixed with DAPI-containing PBS for live-dead discrimination in a 1:4 volume ratio. Ratios and absolute counts of Target and Control cells was assessed by flow cytometry on a Cytoflex LX device (Beckman Coutler). Relative cytotoxicity was calculated via comparison of Target:Control ratios of each well with effector cells to Target:Control ratios of wells without any effector cells.

*VITAL assay*²² *to assess cytotoxicity of purified CD19-CAR T cells:* The assay was performed in the same general way as the formerly described VITAL assay with GFP-tagged Nalm6 (Target) cells and RFP-tagged CD19-knockout Nalm6 (Control) cells. CD19-CAR T cells with / without prior purification steps (as well as wildtype T cells as non-specific effectors) were washed in PBS and cultured in cytokine-free RPMI medium supplemented with 10 % FCS one day before to the assay. After assay setup, the plates were incubated for 6 hours prior to flow cytometric analysis. Relative cytotoxicity was calculated via comparison of Target:Control ratios in well with CAR T effector cells to Target:Control ratios in wells with wildtype T cells serving as unspecific effector cells.

*CAR NK-92 and T cell co-culture assays:* Co-culture assays were performed either in 24-well polystyrene tissue culture plates (Corning Inc.,) for optimization studies or in G-Rex 6 Well Cell Culture Plates (Wilson Wolf Corporation, KDW0026) for feasibility studies of upscaling the process for clinical purposes. In 24 well plates, the initial co-culture was performed with 0.5 × 10⁶ *TRAC-*edited CD19-CAR T cells and 1 × 10⁶ gamma-irradiated (30 Gy) TCR/CD3-CAR NK-92 cells per well. In G-Rex 6 Well Plates, the cell numbers for the initial co-culture were upscaled to 10 × 10⁶ TRAC-edited CD19-CAR T cells and 20 × 10⁶ gamma-irradiated TCR/CD3-CAR NK-92 cells per well. For consecutive co-cultures, TCR/CD3-CAR NK-92 cell numbers were adjusted to the actual T cell counts in the wells. The frequencies of remaining TCR/CD3⁺ T cells was measured every 2-3 days by flow cytometry.

*CD3-depletion of CAR T cell products by magnetic bead separation:* Remaining TCR/CD3⁺ cells were removed from the final (immunotherapeutic) CAR T cell product by negative selection using the monoclonal antibody-conjugated magnetic beads cell separation technology. Cells were labeled with CD3 microbeads and separated through a depletion column (LD column, Miltenyi, 130-042-901) in which conjugated cells are selectively retained. The fraction of eluted cells (negative fraction) was further analyzed by flow cytometry to assess efficiency of the procedure and purity of the final cell product.

*Intracellular cytokine analysis by flow cytometry:* CD19-CAR T cells purified by different methods (CD3-MACS depletion, TCR/CD3-CAR NK-92 co-culture) and cultured in T cell medium until day 14 after peripheral blood collection were rested in cytokine-free medium for 48 hours prior to the assay. Jurkat cells, which are CD19-negative, served as negative control for antigen-specific CAR stimulation. They were labeled with 2.5 µM carboxyfluorescein diacetate succinimidyl ester (CFDA-SE, Thermo Fisher Scientific, V12883) prior to the assay. The differently treated effector T cells (CD19-CAR T cells and wildtype T cells) were split in 4 conditions on a round bottom 96-well plate: 1) no stimulation, 2) co-culture with CD19-expressing, GFP-tagged Nalm-6 cells, 3) co-culture with CD19-negative CFDA-labeled Jurkat cells, and 4) culture in medium containing 10 ng/mL phorbol 12-myristate 13-acetate (PMA) (Sigma-Aldrich, P8139-1MG) and 2.5 µg/mL ionomycin (Sigma Aldrich, I3909) as control for polyclonal stimulation independent of TCR or CAR expression. The plates were centrifuged for 1 min at 100x g, at room temperature and then incubated at 37°C / 5% CO₂. Brefeldin A (Sigma Aldrich, B5936) was added to the wells at a concentration of 10 µg/mL after 30 min. After incubation for 16 hours, cells were washed with PBS and stained on the 96-well plate. A first staining was performed with anti-Fc AF647 (Alexa Flour 647 anti-human IgG (Fcγ fragment specific), Jackson ImmunoResearch, 109-605-098) and fixable blue dead cell stain dye. Cells were washed in PBS once, then fixed and permeabilized using the Intracellular Fixation & Permeabilization Buffer Set (Thermo Fisher Scientific, 88-8824). Then, a first intracellular staining with anti-FC AF647 was performed prior to washing twice with 1× permeabilization buffer. A second intracellular staining was performed with all other antibodies as stated in supplementary table 2.

*Data analysis, statistics and presentation:* FlowJo Software (BD) was used to analyze flow cytometry data. Sorting of such results and data from different assays were collected in Excel (Microsoft). Graph creation and statistics performed with Prism 9 (GraphPad). Illustrations were created on BioRender.com.

### References

1. Abou-EI-Enein, M. et al. Scalable Manufacturing of CAR T cells for Cancer Immunotherapy. Blood Cancer Discov 2, 408-422 (2021).
2. Wagner, D. L. et al. Immunogenicity of CAR T cells in cancer therapy. Nature Reviews Clinical Oncology 1-15 (2021) doi:10.1038/s41571-021-00476-2.
3. Kluesner, M. G. et al. CRISPR-Cas9 cytidine and adenosine base editing of splice-sites mediates highly-efficient disruption of proteins in primary and immortalized cells. Nat Commun 12, 2437 (2021).
4. Torikai, H. et al. A foundation for universal T-cell based immunotherapy: T cells engineered to express a CD19-specific chimeric-antigen-receptor and eliminate expression of endogenous TCR. Blood 119, 5697-5705 (2012).
5. Osborn, M. J. et al. Evaluation of TCR Gene Editing Achieved by TALENs, CRISPR/Cas9, and megaTAL Nucleases. Mol Ther 24, 570-581 (2016).
6. Eyquem, J. et al. Targeting a CAR to the TRAC locus with CRISPR/Cas9 enhances tumour rejection. Nature 543, 113-117 (2017).
7. MacLeod, D. T. et al. Integration of a CD19 CAR into the TCR Alpha Chain Locus Streamlines Production of Allogeneic Gene-Edited CAR T Cells. Molecular Therapy 25, 949-961 (2017).
8. Jing, R. et al. Cas9-Cleavage Sequences in Size-Reduced Plasmids Enhance Nonviral Genome Targeting of CARs in Primary Human T Cells. Small Methods 5, 2100071 (2021).
9. Kath, J. et al. Pharmacological interventions enhance virus-free generation of TRAC-replaced CAR T cells. Molecular Therapy - Methods & Clinical Development 25, 311-330 (2022).
10. Qasim, W. et al. Molecular remission of infant B-ALL after infusion of universal TALEN gene-edited CAR T cells. Sci. Transl. Med. 9, eaaj2013 (2017).
11. Benjamin, R. et al. Genome-edited, donor-derived allogeneic anti-CD19 chimeric antigen receptor T cells in paediatric and adult B-cell acute lymphoblastic leukaemia: results of two phase 1 studies. The Lancet 396, 1885-1894 (2020).
12. Diorio, C., Murray, R., Naniong, M. & et al. Cytosine Base Editing Enables Quadruple-Edited Allogeneic CAR-T Cells for T-ALL. Blood (2022).
13. Bertaina, A. et al. HLA-haploidentical stem cell transplantation after removal of αβ+ T and B cells in children with nonmalignant disorders. Blood 124, 822-826 (2014).
14. Shah, R. M. et al. T-cell receptor αβ+ and CD19+ cell-depleted haploidentical and mismatched hematopoietic stem cell transplantation in primary immune deficiency. J Allergy Clin Immunol 141, 1417-1426.e1 (2018).
15. Gauthier, J. et al. Factors associated with outcomes after a second CD19-targeted CAR T-cell infusion for refractory B-cell malignancies. Blood 137, 323-335 (2021).
16. Juillerat, A. et al. Straightforward Generation of Ultrapure Off-the-Shelf Allogeneic CAR-T Cells. Frontiers in Bioengineering and Biotechnology 8, (2020).
17. Tonn, T. et al. Treatment of patients with advanced cancer with the natural killer cell line NK-92. Cytotherapy 15, 1563-1570 (2013).
18. Kurrle, R., Lang, W., Kanzy, E. J. & Seiler, F. R. Distinction of two CD3-monoclonal antibodies in respect to the effectivity to modulate the induction of IgM- and gamma-interferon synthesis. Behring Inst Mitt 48-58 (1986).
19. Borst, J. et al. BMA031, a monoclonal antibody suited to identify the T-cell receptor alpha beta/CD3 complex on viable human T lymphocytes in normal and disease states. Hum Immunol 29, 175-188 (1990).
20. Shearman, C. W. et al. Construction, expression and characterization of humanized antibodies directed against the human alpha/beta T cell receptor. J Immunol 147, 4366-4373 (1991).
21. Nguyen, D. N. et al. Polymer-stabilized Cas9 nanoparticles and modified repair templates increase genome editing efficiency. Nat Biotechnol 38, 44-49 (2020).
22. Hermans, I. F. et al. The VITAL assay: a versatile fluorometric technique for assessing CTL- and NKT-mediated cytotoxicity against multiple targets in vitro and in vivo. Journal of Immunological Methods 285, 25-40 (2004).
23. Oelke, M. et al. Ex vivo induction and expansion of antigen-specific cytotoxic T cells by HLA-Ig-coated artificial antigen-presenting cells. Nat Med 9, 619-624 (2003).
24. Vera, J. F. et al. Accelerated production of antigen-specific T-cells for pre-clinical and clinical applications using Gas-permeable Rapid Expansion cultureware (G-Rex). J Immunother 33, 305-315 (2010).
25. Nakamura, K. et al. Autologous antigen-presenting cells efficiently expand piggyBac transposon CAR-T cells with predominant memory phenotype. Mol Ther Methods Clin Dev 21, 315-324 (2021).
26. Wiebking, V. et al. Genome editing of donor-derived T-cells to generate allogenic chimeric antigen receptor-modified T cells: Optimizing αβ T cell-depleted haploidentical hematopoietic stem cell transplantation. Haematologica haematol.2019.233882 (2020) doi:10.3324/haematol.2019.233882.
27. Georgiadis, C. et al. Base-edited CAR T cells for combinational therapy against T cell malignancies. Leukemia 35, 3466-3481 (2021).
28. Rasaiyaah, J., Georgiadis, C., Preece, R., Mock, U. & Qasim, W. TCRaβ/CD3 disruption enables CD3-specific antileukemic T cell immunotherapy. JCI Insight 3, (2018).
29. Shy, B. R. et al. High-yield genome engineering in primary cells using a hybrid ssDNA repair template and small-molecule cocktails. Nat Biotechnol (2022) doi:10.1038/s41587-022-01418-8.
30. Mueller, K. P. et al. Production and characterization of virus-free, CRISPR-CAR T cells capable of inducing solid tumor regression. J Immunother Cancer 10, e004446 (2022).
31. Komatsu, F. & Kajiwara, M. Relation of natural killer cell line NK-92-mediated cytolysis (NK-92-lysis) with the surface markers of major histocompatibility complex class I antigens, adhesion molecules, and Fas of target cells. Oncol Res 10, 483-489 (1998).
32. Gornalusse, G. G. et al. HLA-E-expressing pluripotent stem cells escape allogeneic responses and lysis by NK cells. Nat Biotechnol 35, 765-772 (2017).
33. Jo, S. et al. Endowing universal CAR T-cell with immune-evasive properties using TALEN-gene editing. Nat Commun 13, 3453 (2022).
34. Deuse, T. et al. Hypoimmunogenic derivatives of induced pluripotent stem cells evade immune rejection in fully immunocompetent allogeneic recipients. Nat. Biotechnol. 37, 252-258 (2019).
35. Hu, X. et al. Abstract 5598: Engineered hypoimmune allogeneic CAR T cells as potential off-the-shelf CAR T cell immunotherapies. Cancer Research 82, 5598 (2022).
36. Alzubi, J. et al. Automated generation of gene-edited CAR T cells at clinical scale. Molecular Therapy - Methods & Clinical Development 20, 379-388 (2021).
37. Zhang, C. et al. Chimeric Antigen Receptor-Engineered NK-92 Cells: An Off-the-Shelf Cellular Therapeutic for Targeted Elimination of Cancer Cells and Induction of Protective Antitumor Immunity. Frontiers in Immunology 8, (2017).
38. Voynova, E. et al. Increased Activity of a NK-Specific CAR-NK Framework Targeting CD3 and CD5 for T-Cell Leukemias. Cancers (Basel) 14, 524 (2022).
39. Alabanza, L. et al. Function of Novel Anti-CD19 Chimeric Antigen Receptors with Human Variable Regions Is Affected by Hinge and Transmembrane Domains. Mol Ther 25, 2452-2465 (2017).

## Claims

1. A genetically modified natural killer (NK) cell, comprising a nucleic acid sequence encoding a chimeric antigen receptor (CAR), wherein said CAR comprises an extracellular antigen-binding domain that specifically binds a component of a T cell receptor complex, and wherein said nucleic acid sequence is integrated in the NK cell genome without a viral vector.

2. The genetically modified NK cell according to claim 1, wherein the component of the T cell receptor complex bound by the CAR is CD3, preferably CD3 epsilon, or a TCR alpha, beta, gamma and/or delta, preferably a TCR alpha/beta dimer.

3. The genetically modified NK cell according to any one of the preceding claims, wherein the NK cell is an NK-92 cell in which the nucleic acid sequence encoding a chimeric antigen receptor (CAR) is integrated.

4. The genetically modified NK cell according to any one of the preceding claims, wherein the CAR that specifically binds a component of a T cell receptor complex comprises:
a. an extracellular antigen-binding domain that binds CD3 epsilon or a TCR alpha/beta dimer, preferably comprising an antibody or antigen binding fragment thereof,
b. a CD8α or a CD28 transmembrane domain, and
c. an intracellular domain, comprising a 4-1BB and/or a CD28 co-stimulatory domain, optionally comprising additionally a CD3zeta (4-1BB or CD28) signaling domain.

5. The genetically modified NK cell according to any one of the preceding claims, wherein the extracellular antigen-binding domain comprises a single chain variable fragment (scFv) selected from SEQ ID NO 21 that binds a TCR alpha/beta dimer or SEQ ID NO 22 that binds CD3 epsilon.

6. The genetically modified NK cell according to any one of the preceding claims, wherein said NK cell is present in an *in vitro* mixture with:
a. a first genetically modified T cell, preferably a CAR T cell, comprising one or more disruptive modifications to endogenous TCR components, for example a mutated, knocked-out or RNAi-inhibited TCR alpha constant (TRAC) gene and/or CD3 gene,and
b. optionally a second T cell comprising an endogenous TCR expressed and/or presented on the cell surface.

7. A method for destroying a T cell comprising an endogenous TCR expressed and/or presented on the cell surface, the method comprising contacting said T cell with a genetically modified natural killer (NK) cell according to any one of claims 1 to 6.

8. The method according to claim 7, wherein the T cell comprising an endogenous TCR expressed and/or presented on the cell surface is in a mixture with a genetically modified T cell, preferably a CAR T cell, comprising one or more disruptive modifications to endogenous TCR components.

9. The method according to claim 8, comprising purifying and/or enriching a genetically modified T cell, preferably a CAR T cell, comprising one or more disruptive modifications to endogenous TCR components.

10. The method according to any one of claims 7 to 9, wherein the NK cell is gamma irradiated prior to contacting with the T cell(s).

11. The method according to any one of claims 8 to 10, wherein the T cell(s) is administered two or more treatments of the NK cells.

12. The method according to any one of claims 8 to 11, wherein the mixture of T cells after NK cell treatment comprises at least 99.5% genetically modified T cells, preferably CAR T cells, comprising one or more disruptive genetic modifications to TCR component-encoding genes (by cell number), preferably at least 99.9%.

13. A method for manufacturing a therapeutic genetically modified T cell, preferably a CAR T cell, comprising one or more disruptive modifications to endogenous TCR components, the method comprising purifying and/or enriching said cells using the method of any one of claims 7 to 12.

14. An *in vitro* cell population obtained by the method of claim 13, comprising a mixture of:
a. first genetically modified T cells, preferably CAR T cells, comprising one or more disruptive modifications to endogenous TCR components, and
b. second T cells, comprising an endogenous TCR expressed and/or presented on the cell surface,
wherein the cell population comprises at least 99.5% (by cell number) of said first genetically modified T cells, preferably at least 99.9%.

15. The cell population according to claim 14 for use in the treatment and/or prevention of a medical condition susceptible to T cell therapy in a subject.

16. The cell population for use according to claim 15, wherein the cell population is allogeneic to said subject.
